(19) Europäisches Patentamt
European Patent Office
Office européen des brevets

(11) EP 4 194 019 B1

(12) **EUROPEAN PATENT SPECIFICATION**

(45) Date of publication and mention of the grant of the patent:
**08.07.2026 Bulletin 2026/28**

(21) Application number: **21853077.2**

(22) Date of filing: **27.07.2021**

(51) International Patent Classification (IPC):
***A61L 27/36*** (2006.01) ***A61L 27/38*** (2006.01)
***A61L 27/54*** (2006.01) ***A61F 2/02*** (2006.01)

(52) Cooperative Patent Classification (CPC):
**A61F 2/02; A61L 27/34; A61L 27/3683; A61L 27/3834; A61L 27/54;** A61F 2/04; A61F 2240/001; A61L 2300/258; A61L 2430/28; A61L 2430/40

(86) International application number:
**PCT/KR2021/009732**

(87) International publication number:
**WO 2022/030856 (10.02.2022 Gazette 2022/06)**

(54) **METHOD FOR PRODUCING HIGHLY FUNCTIONAL ARTIFICIAL ORGANS USING APTAMERS**

VERFAHREN ZUR HERSTELLUNG HOCHFUNKTIONELLER KÜNSTLICHER ORGANE MIT APTAMEREN

PROCÉDÉ DE PRODUCTION D'ORGANES ARTIFICIELS HAUTEMENT FONCTIONNELS À L'AIDE D'APTAMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.08.2020 KR 20200098314**
**12.11.2020 KR 20200150913**

(43) Date of publication of application:
**14.06.2023 Bulletin 2023/24**

(73) Proprietor: **Kangstem Biotech Co., Ltd.**
**Seoul 06179 (KR)**

(72) Inventors:
- **KANG, Kyung-Sun**
**Seoul 06338 (KR)**
- **KIM, Da-Hyun**
**Hanam-si, Gyeonggi-do 12911 (KR)**

(74) Representative: **Dehns**
**10 Old Bailey**
**London EC4M 7NG (GB)**

(56) References cited:
**WO-A1-2014/128289** **WO-A1-2018/107148**
**CA-A1- 3 068 989** **CN-A- 110 180 026**
**KR-A- 20190 137 010** **KR-B1- 101 825 012**
**KR-B1- 101 829 407**

- **SCHLEICHER MARTINA, WENDEL HANS PETER, FRITZE OLAF, STOCK ULRICH A: "In vivo tissue engineering of heart valves: evolution of a novel concept", REGENERATIVE MEDICINE, FUTURE MEDICINE LTD., GB, vol. 4, no. 4, 1 July 2009 (2009-07-01), GB , pages 613 - 619, XP055894742, ISSN: 1746-0751, DOI: 10.2217/rme.09.22**
- **JAN HOFFMANN, ANGELA PAUL, MARC HARWARDT, JÜRGEN GROLL, THOMAS REESWINKEL, DORIS KLEE, MARTIN MOELLER, HEIKE FISCHER, TOBIAS WALKE: "Immobilized DNA aptamers used as potent attractors for porcine endothelial precursor cells", JOURNAL OF BIOMEDICAL MATERIALS RESEARCH PART A, JOHN WILEY & SONS, vol. 84A, no. 3, 1 March 2008 (2008-03-01), pages 614 - 621, XP055075888, ISSN: 15493296, DOI: 10.1002/jbm.a.31309**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 194 019 B1

Remarks:

The complete document including Reference Table(s) and the Sequence Listing(s) can be downloaded from the EPO website

## Description

[Technical Field]

The present invention relates to a method for producing highly functional artificial organs using aptamers.
The present application claims priority to and the benefit of Korean Patent Application Nos. 10-2020-0098314 and 10-2020-0150913 filed in the Korean Intellectual Property Office on August 6, 2020 and November 12, 2020, respectively.

[Background Art]

Artificial organ biotechnology is technology for producing devices which replace organs constituting the body by various methods, including a technique for culturing stem cells and cell growth factors (cell fluid nutrients, and the like) on a three-dimensional bio-artificial scaffold and creating an artificial organ using this formwork. Although donor organ transplantation is performed for almost all organs due to the development of modern medicine, many patients cannot benefit due to the lack of donors, and in the case of artificial organs, side effects occur due to the lack of biocompatibility.
Meanwhile, chronic liver diseases such as cirrhosis are diseases that kill about 2 million patients each year. In recent decades, liver transplantation has been conducted in many cases of end-stage liver disease due to breakthrough developments in immunology and organ transplantation, and liver transplantation has been currently recognized as the only treatment for congenital or acquired liver disease, but the number of donors is insufficient. In this respect, the production of an artificial liver through tissue engineering has been attracting attention as a substitute for donor organs. Since Korea has a very high mortality rate due to liver-related diseases compared to other diseases, has a considerable number of people waiting for transplantation due to the shortage of organs for transplantation, and is one of the countries with the highest number of liver transplants, there is a need for developing a technique capable of ameliorating these situations.
Although various methods such as tissue engineering, application of 3D printer technology, and organ reconstruction (organoid) using stem cells have been studied in order to produce a liver scaffold, technical limitations such as the inability to mimic organ fine structures and size limitations have not been overcome. As a human organ-mimicking scaffold with safety and functionality while overcoming the above problems, a decellularized scaffold in which all cells have been removed from animal organs is being evaluated as a useful substrate. Cellular components capable of inducing immune responses are removed, and simultaneously, organ-specific microenvironments such as microstructures and biochemical signals are created in scaffolds, so that there is an advantage in that the three-dimensional organization of cells in the scaffold is facilitated when human cells are injected. Therefore, decellularized scaffolds may be used as suitable substrates for producing artificial organs, and research has been conducted to reconstruct various organs using decellularized scaffolds.
Since the liver has a histologically very complex vascular structure and is an organ through which 25% or more of the cardiac output of blood passes, the vascularization of the organ can be the key to successful reconstruction of an artificial liver. After transplantation, artificial organs in which vascular structures have not been formed are connected to the recipient's bloodstream, and then, intravascular thrombi are formed due to acute immune responses, and there is a high possibility of subsequent graft failure. Therefore, various coating agents for efficiently reconstructing vascular structures have been studied.
Aptamers are single-stranded nucleic acids consisting of short sequences, and are applied as substitutes for antibodies because there are advantages of their high affinity to specific proteins, low immunogenicity, and ability to be mass-produced. Aptamers are usually used for medical diagnosis, and are also attracting attention as therapeutic agents that target cancer or viral diseases. However, the usefulness of aptamers as coating agents in terms of tissue engineering has not been clarified.
Further, a human leukocyte antigen (HLA) is one of the human histocompatibility antigens, and immune rejection may occur in the case where homologous cells or artificial organs do not match the human leukocyte antigen type when transplanted into a patient. Recently, many studies have been conducted on universal cells established by removing such human leukocyte antigen-A, B, and C types through gene editing. However, there is a limitation that cells from which human leukocyte antigens-A, B, and C have been removed still show sensitivity to NK cells during transplantation. Therefore, cells capable of ultimately evading immune responses can be produced by overexpressing the "Don't eat me" signal, which suppresses NK cell responses. In particular, cells differentiated into various lineages from off-the-shelf universal stem cells (USCs) having such characteristics may be applied as cell compositions to minimize the occurrence of immune rejection during artificial organ production.
Therefore, in this technique, the efficiency of vasculature reconstruction was maximized using a nucleic acid aptamer as a coating agent, and a technique capable of minimizing thrombus formation after artificial organ transplantation has been established for the first time. In addition, through this technique, a vascularized artificial liver that can be

transplanted in vivo using universal stem cell (USC)-derived liver-constituting cells, and the like and has enhanced functionality was produced.

[Related Art Documents]

K.H. Hussein, K.M. Park, K.S. Kang, H.M. Woo, Heparin-gelatin mixture improves vascular reconstruction efficiency and hepatic function in bioengineered livers, Acta Biomater 38(2016) 82-93

KR 101 829 407 describes the vascular regeneration of degenerated hepatic tissue by decellularizing the hepatic tissue, coating the inner wall of the blood vessels of the depleted tissue with a mixture of a biodegradable support and a bioactive factor to increase adherence of endothelial cells, thereby forming a scaffold. Cells are inoculated into the scaffold and cultured, thereby vascularizing the scaffold.

[Disclosure]

[Technical Problem]

Thus, the present inventors maximized the efficiency of vasculature reconstruction using a nucleic acid aptamer as a coating agent, and have established a technique capable of minimizing thrombus formation after artificial organ transplantation for the first time. In addition, the present inventors confirmed for the first time that an artificial organ that can be transplanted in vivo using a nucleic acid aptamer and has enhanced functionality can be produced, thereby completing the present invention.

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

[Technical Solution]

Embodiments of the present invention are described in the claims.

In one aspect, the present invention provides an *ex vivo* use of a composition comprising anti-CD31 aptamers for coating blood vessels of a decellularized liver, wherein the anti-CD31 aptamers comprise a base sequence represented by SEQ ID NO: 1.

In another aspect, the present invention provides an *ex vivo* method for coating blood vessels of a decellularized liver, the method comprising a step of treating with a composition comprising anti-CD31 aptamers, wherein the anti-CD31 aptamers comprise a base sequence represented by SEQ ID NO: 1.

In the present invention, the anti-CD31 aptamers comprise a base sequence represented by SEQ ID NO: 1.

In yet another exemplary embodiment of the present invention, the anti-CD31 aptamer may be characterized by being specific for vascular endothelial cells, but is not limited thereto.

In yet another exemplary embodiment of the present invention, the anti-CD31 aptamer may increase the expression of one or more selected from the group consisting of integrin beta 3 and phosphorylated Akt, but is not limited thereto.

In yet another exemplary embodiment of the present invention, the anti-CD31 aptamer may decrease the expression of cleaved caspase-3, but is not limited thereto.

In yet another exemplary embodiment of the present invention, the composition may further comprise one or more cells selected from the group consisting of parenchymal cells and non-parenchymal cells, but is not limited thereto.

In yet another exemplary embodiment of the present invention, the cells may be stem cell-derived cells, but are not limited thereto.

In yet another exemplary embodiment of the present invention, the cells may be characterized by comprising stem cell-derived differentiated cells, but are not limited thereto.

In yet another exemplary embodiment of the present invention, the composition may further comprise stem cell-derived parenchymal cells, but is not limited thereto.

In yet another exemplary embodiment of the present invention, the composition may further comprise stem cell-derived non-parenchymal cells, but is not limited thereto.

In yet another exemplary embodiment of the present invention, the stem cells may be one or more selected from the group consisting of induced pluripotent stem cells (iPSCs), embryonic stem cells, mesenchymal stromal cells (MSCs), off-the-shelf universal stem cells (USCs), marrow-derived stem cells, adipose tissue-derived stem cells and placenta-derived stem cells, but are not limited thereto.

In yet another exemplary embodiment of the present invention, the off-the-shelf universal stem cells may have one or more of the following characteristics, but are not limited thereto.

the HLA gene has been removed; and
"Don't eat me" signals are overexpressed.

In yet another exemplary embodiment of the present invention, the *ex vivo* method for coating blood vessels of a decellularized liver may comprise the following steps, but is not limited thereto.

a) providing a liver from a tissue source;
b) decellularizing the provided liver; and
c) treating the decellularized liver with anti-CD31 aptamers.

In yet another exemplary embodiment of the present invention, the *ex vivo* method may further comprise one or more steps selected from the group consisting of the following steps, but is not limited thereto.

d-1) recellularizing a decellularized liver into vascular endothelial cells;
d-2) recellularizing a decellularized liver into parenchymal cells; and
d-3) recellularizing a decellularized liver into non-parenchymal cells.

[Advantageous Effects]

When the aptamer of use in accordance with the present invention is used as a coating agent of a decellularized scaffold, it is possible to more efficiently reconstruct vasculature than existing antibodies by enhancing blood vessel adhesion ability, viability, angiogenesis potential, and the like. Therefore, a vascularized artificial liver produced using the aptamers exhibits the effects of reducing thrombogenesis in vivo as well as enhancing liver function, and thus is expected to be applied as a method for treating diseases by using artificial organs produced using the aptamers according to the present invention.

[Description of Drawings]

FIGS. 1A to 1G illustrate the results of analyzing the characteristics of anti-CD31 aptamers: FIG. 1A illustrates the functional structure of an anti-CD31 aptamer that forms a CD31-aptamer complex; FIGS. 1B to 1D illustrate the results of confirming the dose-dependent binding of a cy5-labeled anti-CD31 aptamer to HUVECs, HepG2 cells and MSCs, FIG. 1B illustrates the dose-dependent binding efficiency of an anti-CD31 aptamer with a FACS profile of HUVECs, HepG2 cells and MSCs, FIG. 1C illustrates the results of quantifying the binding rate, and FIG. 1D illustrates the results of quantifying the mean fluorescence intensity (MFI) of anti-CD31 aptamers bound to HUVECs, HepG2 cells and MSCs; FIG. 1E illustrates immunocytochemical images of HUVECs stained with a cy5-labeled anti-CD31 aptamer (top, red) and an anti-CD31 antibody (bottom, red); and FIGS. 1F and 1G illustrate immunocytochemical images of HepG2 cells and MSCs stained with an anti-CD31 aptamer (top, red) and an anti-CD31 antibody (bottom, red).

FIGS. 2A to 2J show that an anti-CD31 aptamer enables integrin-mediated adhesion of endothelial cells (ECs) to an extracellular matrix (ECM) under shear stress: FIG. 2A illustrates ECM components; FIG. 2B illustrates representative phase-contrast images of sheared HUVECs; FIG. 2C illustrates the attached rate; FIG. 2D illustrates the results of qRT-PCR analysis of coating a device with PBS (vehicle, uncoated), an anti-CD31 aptamer (APT-coated) and an anti-CD31 antibody (Ab-coated), respectively; FIGS. 2E to 2G illustrate western blot analysis results of integrin beta 3, total Akt and phosphorylated Akt in static HUVECs and exposed HUVECs after shear stress exposure in microfluidic devices coated with the respective coating agents; FIGS. 2H to 2J illustrate western blot analysis results of cleaved caspase-3 expression in HUVECs exposed to shear stress and static HUVECs in groups coated with PBS (vehicle, uncoated), an anti-CD31 aptamer (APT-coated) and an anti-CD31 antibody (Ab-coated), respectively; and FIG. 2J illustrates the qRT-PCR results of nitric oxide synthase 3 (NOS3) expression in sheared HUVECs of each group normalized to static HUVECs.

FIGS. 3A to 3L illustrate the results of efficient re-endothelialization using HUVECs of decellularized liver scaffolds: FIG. 3A illustrates a method for re-endothelialization of decellularized rat liver scaffolds using HUVECs; FIG. 3B illustrates representative immunofluorescence images of endothelial vessels with CFDA-labeled HUVECs on scaffolds coated or uncoated with an anti-CD31 aptamer (APT-coated) or an anti-CD31 antibody (Ab-coated); FIG. 3C illustrates the endothelial coverage of the re-endothelialized vessels of each scaffold; FIG. 3D quantifies the average number of re-endothelialized blood vessels of each scaffold; FIG. 3E illustrates the results of quantifying intravascular dextran discharged from the inferior vena cava in each construct after perfusion of dextran through the portal vein; FIG. 3F illustrates enlarged confocal images of APT-coated constructs stained with zonula occludens-1

(ZO-1); FIG. 3G illustrates the qRT-PCR detection results of VE-cadherin and Claudin 5 (CLDN5) in static HUVECs, uncoated, APT-coated and Ab-coated re-endothelialized constructs; FIG. 3H illustrates the results of confirming the viability of each construct using PrestoBlue reagent on days 3, 5, and 7 by performing a resazurin reduction perfusion assay; FIG. 3I illustrates representative confocal images of re-endothelialized constructs of each group stained with cleaved caspase-3 (red) and DAPI (blue).; FIG. 3J illustrates the results of quantifying cells expressing cleaved caspase-3 in each group; FIG. 3K illustrates the amount of NO produced as quantified by ELISA in re-endothelialized constructs according to each group; and FIG. 3L illustrates the results of ELISA quantification of human VEGF secreted from re-endothelialized constructs in each group on day 7.

FIGS. 4A to 4G are results showing that re-endothelialized constructs form low thrombi after human blood perfusion: FIG. 4A illustrates the method used to transfer human blood to re-endothelialized structures in order to evaluate thrombus formation; FIG. 4B illustrates the macroscopic image of each scaffold after human blood perfusion, in which non-endothelialized DLM was used as a negative control; FIG. 4C illustrates representative immunohistochemical images of harvested constructs from each group stained with integrin αIIb (green); FIG. 4D illustrates the quantification of the fluorescence intensity of integrin αIIb expression; FIG. 4E illustrates the results of performing platelet quantification using a hematology analyzer after the blood perfusate in the DLM group was harvested at certain time points; FIG. 4F illustrates the results of performing platelet quantification using a hematology analyzer after blood perfusate was harvested from re-endothelialized constructs of each group at certain time points; and FIG. 4G illustrates the results of RT-PCR analysis of CD63, PLSCR1, TBXAS1 and THBS1, which are genes involved in platelet aggregation in blood perfusion constructs of each group.

FIGS. 5A to 5M illustrate the results of confirming VBHL structures with enhanced functional maturation: FIG. 5A is a schematic view describing the recellularization process of decellularized rat liver scaffolds using HepG2 cells, LX2 cells, HUVECs and MSCs; FIG. 5B is a set of representative immunofluorescence images of an uncoated VBHL construct (CTL-VBHL) or VBHL construct coated with an anti-CD31 aptamer (APT-VBHL) and an anti-CD31 antibody (Ab-VBHL) on day 21; FIG. 5C illustrates the endothelial coverage of blood vessels of each group; FIG. 5D illustrates the results of quantifying the number of average endothelialized vessels per field; FIG. 5E is a set of representative immunohistochemical images of CTL-VBHL, APT-VBHL and Ab-VBHL constructs stained with α-SMA (red); FIG. 5F illustrates the dextran perfusion analysis results; FIGS. 5G to 5H illustrate the results of ELISA quantification of VEGF and NO secretion from VBHL constructs in each group at the indicated time points; FIGS. 5I and 5J illustrate the results of ELISA quantification of the amounts of albumin and urea secreted from VBHL constructs in each group at the indicated time points; FIG. 5K shows the results illustrating improved viability in APT-VBHL constructs on days 17 and 20; FIG. 5L illustrates representative confocal images of TUNEL assay; and FIG. 5M illustrates the results of quantifying TdT-positive cells in a randomized field.

FIGS. 6A to 6E illustrate successful in vivo reperfusion results of anti-CD31 aptamer-coated VBHL constructs: FIG. 6A illustrates auxiliary grafting photographs of the VBHL construct, in which the renal vein (yellow arrow) was connected to the inferior vena cava of the VBHL construct, the renal artery (white arrow) was connected to the portal vein of the construct, and the VBHL construct was reperfused with the in vivo renal circulation system after removal of the vascular clamp (blue arrow); FIG. 6B illustrates representative images of the construct harvested after transplantation; FIG. 6C is a set of representative confocal images of the harvested constructs of each group stained with integrin αIIb (green); FIG. 6D illustrates the results of quantifying the fluorescence intensity of integrin αIIb expression; and FIG. 6E illustrates the results of RT-PCR analysis of Cd63, Plscr1 and Thbs1 in each VBHL construct after transplantation.

FIGS. 7A to 7F illustrate the results of transplanting VBHL constructs in thioacetamide (TAA)-induced liver fibrosis: FIG. 7Ais a schematic view of the method of transplanting VBHL constructs into TAA-induced liver cirrhosis rats; FIG. 7B illustrates representative H&E staining images of host liver tissue sections and representative picrosirius red staining results of liver sections harvested on week 4 after transplantation of each construct; FIG. 7C illustrates the results of quantifying the degree of fibrosis in host livers of each group; FIG. 7D illustrates the qRT-PCR analysis results of fibrosis-related genes, αSma, Vimentin, Tgf-beta1 and Timp1, represented in host livers of each group; and FIGS. 7E and 7F illustrate the ELISA measurement results of ALT and AST levels in rat serum, in which the red line means the normal range.

[Modes of the Invention]

Hereinafter, the present invention will be described in detail.

The present invention provides an *ex vivo* use of a composition comprising anti-CD31 aptamers for coating blood vessels of a decellularized liver, wherein the anti-CD31 aptamers comprise a base sequence represented by SEQ ID NO: 1.

Tissue engineering is a method of anchoring cells on a certain construct (template) using a combination of cells and various materials, and such a construct (template) is called a scaffold, and the purpose of making this is to create a

construct capable of replacing a damaged tissue or organ, that is, an organ. Although there are various types, a construct (template) that leaves only outlines of the microstructure and the organ after cells are removed by decellularizing a biological construct (that is, organ) called a bioscaffold.

An artificial organ may be characterized in that a bioscaffold is treated with aptamers to reconstruct a target organ structure, such as a vascular structure, but is not limited thereto. Specifically, the composition comprising anti-CD31 aptamers used in the present invention may reconstruct or produce a vascular structure by coating the vascular wall of a decellularized liver to specifically bind to vascular endothelial cells.

The present inventors confirmed that when the vascular wall of a decellularized scaffold was coated with an anti-CD31 aptamer such that vascular endothelial cells could adhere to the wall of the decellularized scaffold, a highly functional vasculature, in which the function of the vascular barrier was maintained, was reconstructed (see the Examples of the present invention).

In the present invention, the blood vessel is a blood vessel of a decellularized liver.

In the present invention, the blood vessel may be a hepatic vessel, for example, the hepatic portal vein, hepatic sinusoid, a hepatic vein or the hepatic artery, but is not limited thereto.

An artificial organ may be biocompatible. The term biocompatibility is used interchangeably with "transplant compatibility" and refers to a property of not causing immune rejection upon cell transplantation. A cell, tissue, or organ may be rendered transplant compatible by reducing the expression of genes responsible for immune rejection, and as a non-limiting example thereof, reducing the expression level of the HLA gene may render a cell, tissue or organ transplant compatible. Transplant compatible cells comprise cells in which immunocompatible antigens are homozygous or null, but are not limited thereto.

In the present invention, an aptamer refers to a single-stranded nucleic acid (DNA, RNA, or modified nucleic acid) having a stable tertiary structure by itself as a material capable of specifically binding to a target material, and can bind to a target protein (material). The aptamer may be prepared by determining a sequence of an oligonucleotide which is selective and has binding ability to a target material to be confirmed and synthesizing the sequence according to a general method of preparing an aptamer, and then modifying the 5' end or 3' end of the oligonucleotide with -SH, - COOH, -OH or $NH_2$, such that the end can bind to a functional group of an aptamer chip, but is not limited thereto.

In the present invention, the aptamer is an anti-CD31 aptamer.

In the present invention, the anti-CD31 aptamer comprises a base sequence represented by SEQ ID NO: 1, or may consist of the base sequence represented by SEQ ID NO: 1.

In the present invention, the anti-CD31 aptamer may be characterized by being specific for vascular endothelial cells, but is not limited thereto.

In the present invention, the aptamer may increase the expression of one or more selected from the group consisting of integrin beta 3 and phosphorylated Akt, but is not limited thereto.

In the present invention, the aptamer may decrease the expression of cleaved caspase-3, but is not limited thereto.

In the present invention, the composition may typically be delivered to a tissue or organ matrix by a cell-miscible solution (for example, a physiological composition) under physiological conditions (for example, 37°C). The composition may include a buffer, a nutrient (for example, a sugar and carbohydrate), an enzyme, a proliferation and/or differentiation medium, a cytokine, an antibody, an inhibitory factor, a growth factor, a salt solution, or a serum-derived protein, and the like, but is not limited thereto.

In the present invention, the composition may further comprise parenchymal cells, but is not limited thereto. As used herein, the term "parenchymal cells" refers to cells that form the main part that performs functions inherent to cells. In the present invention, the parenchymal cells may specifically be hepatic parenchymal cells, renal parenchymal cells, corneal parenchymal cells, vascular endothelial cells, and the like, preferably hepatic parenchymal cells, that is, hepatocytes, but are limited thereto. In the present invention, hepatic parenchymal (parenchymal hepatocyte) cells make up a total of 80% of the liver under non-pathological conditions.

In the present invention, the parenchymal cells may be derived from the same species as a recipient, but are not limited thereto.

In the present invention, the composition may further comprise stem cell-derived parenchymal cells, but is not limited thereto. The parenchymal cells may be parenchymal cells differentiated from human off-the-shelf universal stem cells from which human leukocyte antigens have been removed and/or which overexpress "Don't eat me" signals, but are not limited thereto.

In the present invention, the composition may further comprise non-parenchymal cells, but is not limited thereto. The non-parenchymal cells may be one or more selected from the group consisting of hepatic endothelial cells, Kupffer cells, hepatic stellate cells, Ito cells, lipocytes, fat-storing cells, cholangiocytes, pit cells, vascular epithelial cells and fibroblasts, but are not limited thereto.

In the present invention, the composition may further comprise stem cell-derived non-parenchymal cells, but is not limited thereto. The stem cell-derived non-parenchymal cells may be non-parenchymal cells differentiated from human

off-the-shelf universal stem cells from which human leukocyte antigens have been removed and/or which overexpress "Don't eat me" signals, but are not limited thereto.

In the present invention, the cells may be characterized by comprising stem cell-derived differentiated cells, but are not limited thereto.

As used herein, the term "stem cells' refers to cells which have the property capable of continuously producing the same cells as themselves for a certain period of time in a differentiated state and the property of differentiating into specific cells under suitable conditions.

In the present invention, the stem cells may be one or more selected from the group consisting of induced pluripotent stem cells (iPSCs), embryonic stem cells, mesenchymal stromal cells (MSCs), off-the-shelf universal stem cells (USCs), marrow-derived stem cells, adipose tissue-derived stem cells and placenta-derived stem cells, but are not limited thereto.

In the present invention, the off-the-shelf universal stem cells may be characterized in that HLAs have been removed, or "Don't eat me" signals are overexpressed, but are not limited thereto. The *ex vivo* use and *ex vivo* method of the present invention is expected to be clinically applicable by constructing a universal artificial liver comprising stem cell-derived constitutive cells from which human leukocyte antigens have been removed and/or which overexpress "Don't eat me" signals to minimize immune rejection during transplantation.

In the present invention, the off-the-shelf universal stem cells may be HLA class I knockout iPSC cells or HLA class I knockout MSC cells, but are not limited thereto.

In the present invention, the "Don't eat me" signal may be CD47 or CD24, but is not limited thereto.

Further, the present invention provides an *ex vivo* method for coating blood vessels of a decellularized liver, the method comprising a step of treating with a composition comprising anti-CD31 aptamers, wherein the anti-CD31 aptamers comprise a base sequence represented by SEQ ID NO: 1.

In the present invention, the *ex vivo* method may comprise the following steps, but is not limited thereto:

a) providing a liver from a tissue source;
b) decellularizing the provided liver; and
c) treating the decellularized liver with anti-CD31 aptamers.

In the present invention, the method may further comprise one or more steps selected from the group consisting of d-1) recellularizing the decellularized liver into vascular endothelial cells;

d-2) recellularizing the decellularized liver into parenchymal cells; and
d-3) recellularizing the decellularized liver into non-parenchymal cells, but is not limited thereto.

In the present invention, the tissue source may be selected from various mammalian groups such as humans, monkeys, mice, rats, pigs, cows and rabbits.

In the present invention, the decellularization may be performed by methods known in the art, but in an exemplary embodiment of the present invention, the hepatic portal vein obtained from the tissue source is washed with deionized water and PBS, then washed with deoxyribonuclease, and then sterilized with peroxyacetic acid. For example, the following references describe the perfusion-based decellularization of the lungs, liver, kidneys, brain and limbs: Van Putte et al., 2002, Ann. Thorac. Surg., 74(3):893-8; den Butter et al., 1995, Transpl. Int., 8:466-71; Firth et al., 1989, Clin. Sci. (Lond.), 77(6):657-61; Mazzetti et al., 2004, Brain Res., 999(1):81-90; Wagner et al., 2003, J. Artif. Organs, 6(3):183-91. As an alternative to perfusion-based decellularization, biological tissues and organs may be decellularized by immersion in a decellularization solution that removes cells. See, for example, US Pat. Nos. 6,376,244 and 6,753,181.

In the present invention, physiological buffers suitable for perfusion comprise nutrient supplies that may be used for storage and/or organ perfusion, comprising transplantation, and examples comprise buffers with glucose, EGM-2, EGM-2MV, DMEM, Promocell endothelial cell medium, Medium 200, and DMEMF/12, but are not limited thereto. Further, the buffer comprises a culture medium solution suitable for endothelial cell culture or phosphate buffered saline (PBS), but is not limited thereto.

In the present invention, alternating the direction of perfusion (for example, anterograde and retrograde) during decellularization may help effectively remove cells from the whole liver. The decellularization as described in the present specification may cause little damage to the ECM by essentially removing cells from within the liver with little damage to the ECM, but is not limited thereto. Livers may be decellularized at a suitable temperature between 4 to 40°C. Depending on the size and weight of the liver, the specific detergent(s) in a cell disintegration medium, and the concentration of the detergent(s), the liver is generally perfused with the cell disintegration medium for about 2 to about 12 hours per gram of solid liver. Livers comprising lavage fluid may be perfused for about 1 to about 12 hours per gram of tissue. Perfusion is generally regulated by physiological conditions comprising pulsatile blood flow, flow velocity and pressure.

For recellularization to generate an artificial liver, the number of regenerative cells introduced into and onto a

decellularized liver may vary depending on the developmental stage of the liver (for example, the size and weight of the liver) and regenerative cell. Different types of cells may have different tendencies with respect to the population density that these cells reach. For example, a decellularized liver may be "inoculated" with at least 1,000, 10,000, 100,000, 1,000,000, 10,000,000 or 100,000,000 regenerative cells; or may have about 1,000 cells/tissue mg (wet weight, that is, weight before decellularization) to about 100,000,000 cells/tissue mg (wet weight) after recellularization, but is not limited thereto.

In the present invention, the *ex vivo* method may further comprise culturing a decellularized liver treated with an anti-CD31 aptamer which comprises a base sequence represented by SEQ ID NO:1 in a bioreactor for 1 to 100 days, 1 to 90 days, 1 to 80 days, 1 to 70 days, 1 to 60 days, 1 to 50 days, 1 to 40 days, 1 to 1 month, 1 to 3 weeks, 1 to 15 days, 1 to 2 weeks, 5 to 15 days, 5 to 2 weeks, 1 week to 3 weeks or about 2 weeks, but is not limited thereto.

Throughout the specification of the present application, when one part comprises one constituent element, unless otherwise specifically described, this does not mean that another constituent element is excluded, but means that another constituent element may be further comprised. Throughout the specification of the present application, a term of a degree, such as about or substantially, is used in a corresponding numerical value or used as a meaning close to the numerical value when natural manufacturing and material tolerance errors are presented in a described meaning, and is used to prevent an unscrupulous infringer from illegally using disclosed contents comprising a numerical value illustrated as being accurate or absolute in order to help understanding of the present invention. Throughout the specification of the present application, a term such as a step ... or a step of ..., does not mean a step for ....

Throughout the specification of the present application, the term combination(s) thereof comprised in the Markush type expression means a mixture or combination of at least one selected from the group consisting of constituent elements described in the Markush type expression, and means comprising at least one selected from the group consisting of the constituent elements.

Throughout the specification of the present application, the description A and/or B means A or B, or A and B.

Specific steps may be performed out of the order described in cases where certain embodiments can be implemented differently. For example, two steps described in succession may be performed substantially concurrently, or may be performed in reverse order to that described.

Hereinafter, preferred examples for helping with understanding of the present invention will be suggested. However, the following examples are provided only so that the present invention may be more easily understood, and the content of the present invention is not limited by the following examples.

## Experimental methods and materials

### 1. Aptamer preparation and analysis

An anti-CD31 single-stranded DNA aptamer (76 mer, 21966-18-01) having a core sequence of SEQ ID NO: 1 was synthesized and characterized by Aptamer Sciences Inc. (Korea). After the anti-CD31 aptamer was dissolved in sterile Ultrapure DEPC-treated water (Invitrogen, USA), the resulting solution was stored at -20°C. Before experiments were performed, the aptamer was heated at 95°C for 10 minutes and allowed to cool at room temperature to induce proper folding. To confirm that the anti-CD31 aptamer specifically targets CD31+ ECs, immunofluorescent staining and flow cytometry were performed using an aptamer labeled with cy5 at the 5-end (5'-cy5-aptamer-3')

<Anti-CD31 aptamer>

5'-TCA GCC GCC AGC CAG TTC(primer) - G6A GAG GAG G6A CG6 AA6 G6C 6GG G6A 6AC CCC GA6 AA6 6(SEQ ID NO: 1; core sequence) - GAC CAG AGC ACC ACA GAG(primer)-3'

6 = NapdU [5-(N-Napthylcarboxyamide)-2'-deoxyuridine]

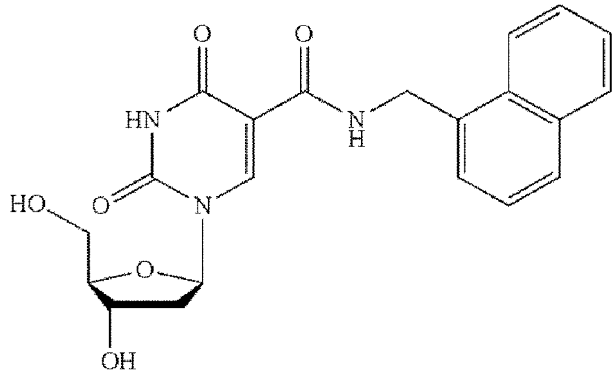

## 2. Cell Culture

Human umbilical vein endothelial cells (HUVECs) and human umbilical cord blood-derived mesenchymal stem cells (MSCs) purchased from ATCC (USA) were maintained in an endothelial growth medium-2 (EGM-2, Lonza, Switzerland). Under the approval of the Seoul National University Institutional Review Board (IRB No. 1608/001-021), MSCs were established as described in Donor-dependent variation of human umbilical cord blood mesenchymal stem cells in response to hypoxic preconditioning and amelioration of limb ischemia, Exp Mol Med 50(4)(2018) 35. Hepatic carcinoma cells (HepG2 cells) purchased from ATCC and LX-2 human stellate cells purchased from Millipore (USA) were cultured in high-glucose-containing Dulbecco's Modified Eagle Medium (DMEM, Hyclone, USA). All media were supplemented with 10% fetal bovine serum (FBS, Gibco), antibiotics (100 U/ml penicillin and 100 μg/ml streptomycin (1% P/S, Gibco)) and 20 μg/ml Primocin (InvivoGen, USA). The culture medium was replaced every 48 hours. Cells were maintained at 37°C in a 5% $CO_2$ humidified incubator.

## 3. Microfluidic device design and cell detachment analysis

A cell detachment rate according to a coating agent was evaluated using a 3D microfluidic system. A device having three microchannels was manufactured by soft lithography and replication molding using polydimethylsiloxane (PDMS, Sylgard 184; Dow Corning, USA). A PDMS prepolymer consisting of a mixture of a PDMS base and a curing agent (10:1) was poured onto a silicon wafer and cured. Subsequently, the PDMS block was fully solidified, and then separated from the wafer.

After an inlet and an outlet were punched using a biopsy punch, the PDMS block was attached to a vacuum adhesive polycarbonate film. Before additional experiments were performed, the device was kept overnight in a drying oven to restore hydrophobicity and sterilized by UV irradiation. The microfluidic device was coated with 100 μg/ml of a mixture of type 1 collagen (rat tail, #354236, Corning, USA), vitronectin (#A31804, Gibco) and fibronectin (#356008, Corning).

Thereafter, the device was coated with each of the following coating agents; PBS (negative control), 600 nM anti-CD31 aptamer and 50 μg/mL anti-CD31 antibody. $5x10^5$ HUVECs were seeded into each channel and incubated for 2 hours. Subsequently, fluid shear stress was applied to a HUVEC monolayer using an infusion syringe pump (PHD 2000 Infusion, Harvard device, USA). Shear stress was calculated based on the modified Poiseuille equation (see Equation 1).

[Equation 1]

$$Tw = 6\ \mu Q/\ wH^2$$

(Tw: shear stress (dynes/cm2), μ: viscosity (Poise) at 37°C, Q: flow rate (mL/s), w: channel width (cm) and H: channel height (cm).)

EGM-2 supplemented with 1% FBS was allowed to pass through the channel at an indicated flow rate for 10 minutes. For each flow, after flow, images of cells adhered to the surface were captured using an optical microscope (IX70, Olympus, Japan) and counted using Image J software. To analyze changes in HUVECs in response to shear stress, RNA and protein were extracted from cells after exposure to a shear stress of 10 dynes/$cm^2$ for 30 minutes.

## 4. Production of decellularized rat liver scaffolds

Natural livers were obtained from 8-week female Sprague-Dawley rats (250 to 300 g) after systemic heparinization. A hepatic portal vein was cannulated with a 24G catheter and perfused with deionized aqueous phase 0.1% SDS (Sigma Aldrich, USA) for 6 hours and then washed with PBS for 10 hours. Subsequently, the scaffold was perfused with 0.1 mg/ml deoxyribonuclease 1 (DNase 1; Sigma Aldrich) for 1 hour and washed with PBS for 2 hours. To remove the bioburden from the scaffold, the scaffold was sterilized with 0.1% peracetic acid (Sigma Aldrich) and stored at 4°C in PBS containing antibiotics. All rat experiments were approved by the Seoul National University Animal Care Committee (SNU-170113-2).

## 5. Re-endothelialization of rat liver scaffold

After EGM-2 was perfused for 1 hour to stabilize the sterile decellularized liver matrix scaffolds, the scaffolds were coated at 4°C for 1 hour by injecting the following respective coating agents via the hepatic portal vein: PBS, 600 nM anti-CD31 aptamer and 50 μg/mL anti-CD31 antibody (14-0319-80, eBioscience, USA). A total of $1X10^7$ HUVECs were labeled with carboxyfluorescin diacetate succinimidyl ester (CFDA) using Vybrant CFDA SE cell tracker kit (Invitrogen).

CFDA-labeled HUVECs and antibiotics suspended in EGM-2 supplemented with 10% FBS were delivered through the portal vein at a rate of 0.5 ml/min. After static culture was maintained for 2 hours, the re-endothelialized construct was perfused with the medium at a rate of 1.5 ml/min using a peristaltic infusion pump in a bioreactor. The culture medium was replaced every 48 hours and samples were harvested on day 7 for additional analysis. The endothelial coverage and the number of re-endothelialized vessels were quantified by Image J software.

**6. Delivery of hepatic parenchymal cells and non-parenchymal cells to decellularized rat livers**

When a decellularized liver scaffold was produced, the bile duct was cannulated with a 26G catheter and the portal vein was cannulated with a 24G catheter. After decellularization, a total of $4\times10^7$ HepG2 cells were delivered through the bile duct to the parenchyma of the scaffold in half and maintained in the bioreactor for 2 days. Two days later, $4\times10^7$ HepG2 cells were substantially delivered again and the construct was cultured for up to 14 days.

On day 14, a mixture of $6x10^6$ CFDA-labeled HUVECs and $3x10^6$ MSCs was delivered through the portal vein to the vessel lumen and simultaneously, $1x10^7$ LX2 cells were injected through the bile duct. All cells were delivered at a rate of 0.5 ml/min. After the static culture was maintained for 2 hours, the construct was perfused at a rate of 1.5 ml/min using a peristaltic infusion pump. Up to 14 days, the VBHL construct was maintained in a DMEM high glucose medium comprising 10% FBS and antibiotics. From day 14 to day 21, the medium was replaced with EGM-2 comprising 10% FBS and antibiotics. Additional analysis was performed on the VBHL construct obtained on day 21.

**7. Statistical analysis**

Statistical analysis was performed using GraphPad Prism version 5.0. All values were reported using mean ± standard deviation. A value of $p < 0.05$ was considered significant. Statistical analysis between both groups was performed with an unpaired, two-tailed Student's t-test. For multiple group comparisons, a two-way ANOVA was performed with the Bonferroni post hoc test. The presented data represents the results of at least three independent experiments.

**8. Immunofluorescence staining**

Cells or tissue sections were fixed with 4% formaldehyde for 10 minutes. Subsequently, samples were permeabilized with 0.2% Triton X-100 (Sigma Aldrich) for 10 minutes and blocked with 5% chlorine serum (Vector Laboratories, Switzerland) for 1 hour. Subsequently, samples were probed with the following primary antibodies at 4°C overnight: anti-human CD31 (14-0319-80, eBioscience, USA), anti-Albumin (GTX102419, GeneTex, USA), anti-Vimentin (ab45939, Abcam, UK), anti-Galactosidase alpha (GTX101178, GeneTex), anti-ZO-1 (#40-2200, Invitrogen), anti-cleaved Caspase-3 (#9664, Cell Signaling Technology, USA), anti-phospho-Akt (#9271, Cell Signaling Technology), anti-Integrin beta 3 (#13166, Cell Signaling Technology), anti-Integrin αIIb (sc-365938, Santa Cruz biotechnology, USA) and anti-α-Smooth muscle actin (ab7814, Abcam). Fluorescent-dye conjugated secondary antibodies (Alexa Fluor 488-labeled and 549-labeled; Invitrogen) were applied for 1 hour. Nuclei were stained with DAPI (sc3598, Santa Cruz Biotechnology) for 10 minutes and cells were mounted on a fluorescent mounting medium (S302380, DAKO, Denmark). Stained signals were visualized by an Eclipse TE 2000 confocal laser scanning microscope (Nikon, Japan). Similarly, paraffin-embedded tissue sections were deparaffinized, hydrated, fixed with 4% formaldehyde, and then stained as described above.

**9. Flow cytometry**

To estimate the binding kinetics of anti-CD31 aptamers in a concentration-dependent manner, HUVECs were cultured with different concentrations of a cy5-labeled anti-CD31 aptamer in a reaction buffer consisting of 1 mM $MgCl_2$ and 2 mg/ml bovine serum albumin at 4°C for 20 minutes. HepG2 cells and MSCs were compared in order to test the specificity of the anti-CD31 aptamer. Cells were washed with 1 ml of reaction buffer, suspended in PBS and analyzed by FACS Calibur (BD Bioscience, USA). A PE-conjugated anti-CD31 antibody (BD555446, BD Biosciences) was used as a positive control. Data was analyzed using FlowJo software (USA).

**10. Cell adhesion assay**

Rat abdominal aortas were harvested and immersed in stirred 0.05% SDS for decellularization. An internal structure was revealed by cutting the decellularized arterial scaffold longitudinally, and sterilized with 0.1% peroxyacetic acid. Subsequently, the scaffold was immersed in the coating agent for 2 hours; PBS (negative control), 600 nM anti-CD31 aptamer and 50 μg/mL anti-human CD31 antibody (14-0319-80, eBioscience, positive control). Subsequently, the coated scaffold was transferred to a 24-well plate and $1x10^6$ HUVECs were seeded onto the inner surface of the arterial scaffold.

After static culture was maintained for the indicated time points (2 and 4 hours), the cell-dispensed scaffold was washed with PBS and transferred to a new plate. The scaffold was subjected to a tetrazolium-based MTT assay to quantify the number of viable cells. For the MTT assay, samples were kept in a medium containing a 10% MTT stock solution (Sigma Aldrich) at 37°C for 4 hours. Subsequently, the supernatant was removed and dimethyl sulfoxide was added to each well for solubilization of purple formazan. Absorbance at a wavelength of 540 nm was measured using a microplate reader (Infinite M200 pro, Tecan, Switzerland).

**11. qRT-PCR**

Total RNA was extracted from cells or engineered constructs using NucleoZOL (Macherey-Nagel, Germany). Thereafter, complementary DNA was synthesized from the extracted RNA using the Superscript III First-Strand Synthesis System (Invitrogen). qRT-PCR was performed with SYBR Green PCR Master Mix (Applied Biosystems, USA) using the ABI 7300 Real time PCR system (Applied Biosystems). The relative quantification of target mRNA expression levels was determined by the 2($\Delta\Delta^{\text{threshold cycle}}$)($2^{-\Delta\Delta CT}$) method. Expression levels for each gene were normalized to housekeeping gene expression. The primer sequences used are shown in Table 1.

[Table 1]

| Species | Genes | 5-Forward sequence-3 | 5-Reverse sequence-3 |
|---|---|---|---|
| human | *GAPDH* | TGATGACATCAAGAAGGTGGTG | ACCCTGTTGCTGTAGCCAAAT |
| human | *ITGB1* | CGTAGCAAAGGAACAGCAGAG | GGTAGTAGAGGTCAATGGGATAGTC |
| human | *ITGB3* | CCTCATCACCATCCACGACC | GTTGTTGGCTGTGTCCCATTT |
| human | *VE-cadherin* | CTTCACCCAGACCAAGTACACA | AATGGTGAAAGCGTCCTGGT |
| human | *CLDN5* | AAGTGTACGACTCGGTGCTG | AAACAGGTAGAGCACGCCTC |
| human | *NOS3* | AAAGACAAGGCAGCAGTGGAAAT | TCCACGATGGTGACTTTGGCTA |
| human | *CD63* | CAGCTAGAGAGCCCCGGA | TTCATTCCTCCTTCCACCGC |
| human | *PLSCR1* | GGCAGCCAGAGAACTGTTTTAATC | GGCAAGTTTGTTTCCGGGTG |
| human | *TBXAS1* | TGCAGAGCACGGTTCCC | GTGGAGTACCATTTCAGGAGGG |
| human | *THBS1* | AGTCGTCTCTGCAACAACCC | ACAGGCATCCATCAATTGGACA |
| human | *ALB* | CGCTATTAGTTCGTTACACCA | TTTACAACATTTGCTGCCCA |
| human | *CYP1A2* | CGGACAGCACTTCCCTGAGA | AGGCAGGTAGCGAAGGATGG |
| rat | *Gapdh* | ACCACAGTCCATGCCATCAC | TCCACCACCCTGTTGCTGTA |
| rat | *Cd63* | CATCAAGAAGCGTCGGGGA | ACCTGAACTGCTACGCCAAT |

(continued)

| Species | Genes | 5-Forward sequence-3 | 5-Reverse sequence-3 |
|---|---|---|---|
| rat | *Plscr1* | CTGCGAGGCTTTAGGGAGAG | CTCTGAGGTCTGCAAGGTGG |
| rat | *Thbs1* | TAGCTGGAAATGTGGTGCGT | AGCAAGCATCAGGCACTTCT |
| rat | *α-Sma* | CATCACCAACTGGGACGACA | TCCGTTAGCAAGGTCGGATG |
| rat | *Vimentin* | CACTCACCTGCGAAGTGGAT | TGGTATTCACGAAGGTGGCG |
| rat | *Tgf-beta1* | CTGCTGACCCCCACTGATAC | AGCCCTGTATTCCGTCTCCT |
| rat | *Timp1* | TGCTCAAAGGATTCGACGCT | AGCAGGGCTCAGATTATGCC |

## 12. Western blot

Proteins were extracted using PRO-PREP (iNtRon Biotechnology, Korea) and whole cell lysates were sonicated. To extract proteins from tissue samples, the tissue was homogenized with steel beads and PRO-PREP, and then sonicated. After centrifugation, the supernatant was transferred to a new tube and analyzed. Protein concentration was quantified using a DC analysis kit (Bio-Rad, USA). Equal amounts of protein (10 μg) were loaded on an 8 to 15% acrylamide Tris-glycine gel. A target protein was examined with the following primary antibodies; anti-GAPDH (AB2302, Millipore), anti-Integrin beta 3 (#13166, Cell Signaling Technology), anti-Akt (#4691, Cell Signaling Technology), anti-phospho-Akt (#9271, Cell Signaling Technology), anti-cleaved Caspase-3 (#9664, Cell Signaling Technology), anti-Galactosidase alpha (GTX101178, GeneTex), anti-Fibronectin (ab2413, Abcam), anti-Collagen IV (ab19808, Abcam), anti-Laminin (ab11575, Abcam), anti-CK18 (MAB3234, Millipore), anti-CYP2E1 (CSB-PA006425EA01HU, CUSABIO, China) and anti-AFP (A0008, DAKO). After incubation with a primary antibody at 4°C overnight, the HRP-conjugated secondary antibody was applied. After probing with an Amersham Enhanced chemiluminescence detection kit (GE Healthcare, USA), specific protein bands were derived by FluorChem HD2 (Alpha Innotech., USA).

## 13. Tissue examination

Samples were washed three times with PBS and fixed in 4% paraformaldehyde at 4°C overnight. Tissues were embedded in paraffin and serially cut into a thickness of 10 μm. After deparaffinization of tissue sections, a series of descending concentration of ethanol ranging from 100% to 70% were used for hydration. Subsequently, tissue slices were stained with H&E and a picrosirius red solution (0.1% Direct Red 80 and 0.1% Fast Green FCF). All reagents were purchased from Sigma Aldrich. PAS staining was performed using a PAS staining kit (ab150680, Abcam). After staining, sections were rinsed with tap water, dehydrated using a series of ascending concentrations of ethanol, and mounted. Visualization was performed using Nikon NIS-Elements software and an optical microscope (Nikon), and quantification of fibrosis was performed using Image J software.

## 14. Resazurin reduction perfusion assay

PrestoBlue Cell Viability Reagent (A13261, Invitrogen), which is a non-toxic resazurin-based solution, was used according to the manufacturer's instructions. To plot a standard curve for HUVECs, 0.02, 0.05, 0.1, 0.2, 0.5, 1 and 2 million cells were seeded in 6 well plates. After culture for 6 hours, cells were washed and medium was replaced with 2 ml of PrestoBlue reagent mixed with complete EGM-2 (ratio of 1:20). After incubation at 37°C for 1 hour, the medium was collected, and then the absorbance at a wavelength of 570 nm was measured using a microplate reader. According to the standard curve, 30 ml of a PrestoBlue-intermediate mixture was perfused into the engineered construct at 37°C for 1 hour. Harvested media were analyzed as described above.

## 15. FITC-conjugated dextran perfusion assay

To evaluate the permeability of re-endothelialized vessels in engineered tissues, perfusion of 500 kDa FITC-dextran (FD500S, Sigma Aldrich) through the portal vein was used. 25 mL of FITC-dextran (0.2 mg/mL) was administered at 20 mmHg. Dextran in a fluid discharged from the inferior vena cava was considered intravascular dextran, whereas dextran in the peripheral blood was considered extravascular dextran. The amount of total dextran was determined by fluorescence intensity (absorbance at 490 nm) multiplied by the amount of fluid discharged.

## 16. TUNEL assay

To detect apoptotic cells from engineered constructs, the ApopTag Red In situ Apoptosis Detection Kit (Millipore) was used. Tissue sections were deparaffinized and digested with proteinase K. After an equilibration buffer was applied to the sections, the sections were probed with TdT enzyme in an incubator at 37°C for 1 hour. After washing with stop/wash buffer, anti-digoxigenin (rhodamine-conjugate) was applied to the sections. Subsequently, these sections were washed with PBS, and then the nuclei were stained with DAPI. TUNEL-positive cells were detected under a confocal microscope.

## 17. Quantitation of secreted NO and VEGF

The concentration of NO secreted from the bioengineered constructs was measured using the NO Plus detection kit (iNtRON). Conditioned media were harvested at the indicated time points and cell debris was removed through centrifugation. Thereafter, NO was measured from the supernatant of the conditioned media according to the manufacturer's instructions. Absorbance at a wavelength of 540 nm was measured using a microplate reader. Constructs were cultured in endothelial basal medium-2 (EBM-2) without growth factors and exposed to the human VEGF Quantikine ELISA kit (DVE00, R & D Systems, USA). Twelve hours later, the supernatant was obtained for VEGF quantification of the conditioned media from each engineered construct. Absorbance at a wavelength of 450 nm was measured using a microplate reader.

## 18. *Ex vivo* human blood perfusion assay

Scaffolds repopulated with 1 x $10^7$ HUVECs were maintained in EGM-2 supplemented with 10% FBS and antibiotics for 7 days before perfusion with human blood. Heparinized human blood obtained from the Korean Red Cross Central Blood Center was mixed with culture medium (1 : 1) and perfused through the portal vein of each scaffold. At the indicated time points, blood tube effluent was collected and the number of platelets in the blood perfusate was counted using an Advia 2120i hematology system (Siemens Healthineers, Germany). 24 hours after perfusion, samples were washed with PBS and subjected to gene expression analysis and immunostaining to evaluate the degree of thrombus development within the scaffolds. cDNA was synthesized from the extracted RNA and then amplified by PCR using primers targeting the thrombosis gene. Further, paraffin-embedded tissue blocks were sectioned and stained with integrin αIIb in accordance with the procedure described in the immunofluorescent staining method.

## 19. Albumin/urea ELISA analysis

The amount of albumin protein secreted from the constructs in the conditioned media was determined using a human albumin ELISA kit (ab108788, Abcam). Conditioned media from VBHL constructs were harvested at the indicated time points. After centrifugation, the concentration of secreted albumin in the supernatant was quantified using ELISA according to the manufacturer's recommendations. Absorbance at a wavelength of 450 nm was measured using a microplate reader. In addition, the amount of urea secreted from the supernatant collected in the conditioned media was measured. The concentration of urea was quantified by measuring the absorbance at a wavelength of 520 nm using the QuantiChrom Urea Assay Kit (BioAssay Systems, USA).

## 20. In vivo reperfusion of vascularized liver constructs

First, decellularized liver constructs were prepared by catheterization as follows: 22G catheter-portal vein, 26G catheter-bile duct and 20G catheter-inferior vena cava. VBHL constructs were produced as described in Experimental Methods Part 6. The VBHL constructs were maintained in a bioreactor for 21 days and then directly connected to a host renal circulation system by catheters with various sizes. For the above procedure, the left kidney was exposed by anesthetizing a 1-year-old healthy rat. After fixation of the renal artery and vein, each 24G catheter was catheterized. Catheters disposed in the portal vein and inferior vena cava of the construct were connected to catheters disposed in the renal artery and vein, respectively. Each connection part was fixed with Vetbond glue (3M, USA). After a vascular clamp

was removed, the blood was perfused *in vivo* for 2 hours. Thereafter, an additional analysis of the harvested liver constructs was performed.

### 21. TAA-induced chronic liver injury rat model and transplantation of VBHL constructs

To evaluate the *in vivo* function of VBHL constructs, chronic liver injury was induced in 4-week-old female rats. After 0.3 g/L TAA (Sigma Aldrich) containing drinking water was continuously administered to rats for 12 weeks, induced liver fibrosis was analyzed. ALT and AST levels in rat serum were measured using the ALT Activity Colorimetric assay kit (K752-100, Biovision, China) and AST Activity Colorimetric assay kit (K753-100, Biovision). After liver cirrhosis was induced in rats for 8 weeks, the liver was exposed by performing a rat laparotomy. Subsequently, fibrous capsules were removed from the VBHL constructs of each group and then transplanted and fixed between the medial and right lateral lobes of the host liver. VBHL constructs were produced as described in Experimental Methods Part 6, using decellularized rat livers. After 4 weeks, the livers of hosts transplanted with liver constructs were harvested again and subjected to additional analyses. Meanwhile, serum samples were obtained before and after surgery.

### Example 1. Characterization of anti-CD31 aptamer

As illustrated in FIG. 1A, an anti-CD31 aptamer capable of specifically binding to the CD31 protein was produced, and then the binding strength between CD31-expressing cells and the aptamer was verified using a flow cytometer.

As a result, as illustrated in FIGS. 1B and 1C, 600 nM and 800 nM aptamers were able to bind to about 99% of vascular endothelial cells (HUVECs), and HepG2 and MSCs that do not express CD31 were bound to about 2% of the cells. In addition, when the binding strength with vascular endothelial cells according to the aptamer concentration was quantified, the binding strength was saturated at a 600 nM aptamer, so that the aptamer concentration condition was optimized at 600 nM.

Furthermore, as illustrated in FIGS. 1E to 1G, it was confirmed through a cell immunostaining method that the 600 nM aptamer binds to HUVECs, but not to HepG2 and MSCs. Based on the above results, it was verified that the anti-CD31 aptamer specifically binds to CD31.

### Example 2. Evaluation of adhesion ability of vascular endothelial cells during aptamer treatment and confirmation of effects on vascular endothelial cells

As illustrated in FIG. 2A, the inside of the microfluidic device was coated with extracellular matrix (ECM) components, and then coated with an anti-CD31 aptamer (APT-coated) and an anti-CD31 antibody (Ab-coated, positive control), respectively, and HUVECs were injected, and then cell adhesion ability was evaluated while flowing the liquid at a constant speed.

As a result, as illustrated in FIGS. 2B and 2C, more than 80% of the cells in the APT-coated group adhered strongly when a shear stress of 10 dynes/$cm^2$ was applied compared to the uncoated group, and showed a high adhesion ability of 61.5% even when a strong shear stress of 20 dynes/$cm^2$ was applied. The Ab-coated group, which is a positive control, showed an adhesion ability of 51% when treated with a shear stress of 20 dynes/$cm^2$. Through this, it is shown that the anti-CD31 aptamer enhances the adhesion ability of vascular endothelial cells with higher efficiency than the anti-CD31 antibody.

Furthermore, to investigate a mechanism that mediates the difference in adhesion ability of HUVECs by each coating agent, the mRNA expression pattern was analyzed by extracting RNA from cells in the microfluidic device.

It is known that integrin proteins consist of an extracellular domain, a transmembrane domain, and an intracellular domain, and when the extracellular domain binds to the extracellular matrix, signals are transmitted into the cell to activate various signaling systems. Thus, it was confirmed whether or not the integrin protein can be activated by shear stress.

As a result, as illustrated in FIGS. 2E to 2G, it was confirmed that not only integrins but also Akt signaling, which is a lower signaling system, were activated in HUVECs subjected to a shear stress of 10 dynes/$cm^2$ in a microfluidic device actually coated with an aptamer. Akt signaling has been reported to be associated with cell survival and angiogenesis potential in vascular endothelial cells. Accordingly, as illustrated in FIG. 2I, it was also confirmed that the expression of cleaved caspase-3, which is a cell apoptosis marker, was reduced in the APT-coated group.

### Example 3. Verification of efficacy of aptamers during reconstruction of vascular structures by re-cellularization of vascular endothelial cells in decellularized scaffolds

As illustrated in FIG. 3A, a decellularized scaffold was produced from the rat liver, and then the inner wall of the blood vessel was coated with a coating agent. HUVECs labeled with CFDA (green) were recellularized, and then cultured for 7 days and each reconstructed vessel structure was confirmed.

As a result, as illustrated in FIG. 3B, it was confirmed that vascular endothelialization was well performed within the scaffold treated with the anti-CD31 aptamer as a coating agent.

Furthermore, as illustrated in FIGS. 3C and 3D, it was confirmed that about 80% endothelialization per blood vessel was achieved in the APT-coated group, and about 80% vascular endothelialization was achieved within the scaffold. Through this, it was confirmed that endothelialization efficiency was maximized in the aptamer-treated group compared to the uncoated group in which coating was not performed.

Further, as illustrated in FIG. 3E, it was confirmed that when dextran was injected through a portal vein catheter to evaluate the barrier function of reconstructed blood vessels, the amount of intravascular dextran that did not leak out of the blood vessel was significantly increased in the APT-coated group. Therefore, it was verified that when the scaffold was treated with the anti-CD31 aptamer as a coating agent, a highly functional vascular structure in which the barrier function was well maintained was efficiently reconstructed in the decellularized scaffold.

In addition, since the viability of cells cultured during in vitro culture can significantly affect the functionality of reconstructed artificial organs, the degree of apoptosis of cultured cells was analyzed through an immunostaining method.

As a result, as illustrated in FIGS. 3I and 3J, cell apoptosis was reduced in the APT-coated group compared to the uncoated group or the anti-CD31 antibody-coated group.

Thereafter, enzyme-linked immunosorbent assay (ELISA) was performed to verify the blood vessel-specific functionality of a reconstructed vascularized organ.

As a result, as illustrated in FIGS. 3K and 3L, it was confirmed that when the amounts of nitric oxide (NO) and VEGF secreted from the HUVEC-recellularized scaffolds were analyzed, the amounts of NO and VEGF secreted in the APT-coated group was significantly increased compared to the other groups.

Overall, it was verified that when the anti-CD31 aptamer was used as a coating agent, not only revascularization efficiency and barrier function, but also vascular viability and angiogenesis potential were enhanced.

## Example 4. Evaluation of thrombus formation in scaffold by *ex vivo* human blood perfusion

As illustrated in FIG. 4A, in order to closely verify the degree of vascular reconstruction, the degree of thrombus formation was evaluated ex vivo by perfusing human blood into a scaffold in which HUVECs were recellularized and cultured for 7 days. By the analysis method, the degree of thrombus formation when artificial organs were implanted in actual humans could be evaluated.

As a result, as illustrated in FIG. 4B, thrombus formation was visually reduced in the APT-coated group. In addition, as illustrated in FIGS. 4C and 4D, it was confirmed through immunostaining that the expression of integrin $\alpha$IIb, which is a thrombus marker, was decreased in the APT-coated group.

Further, as illustrated in FIGS. 4E and 4F, it was confirmed that when the number of platelets was quantified in the perfusate after blood perfusion, the number of platelets remaining in the perfusate of the decellularized liver matrix (DLM) group, which was not re-endothelialized, was decreased to less than 20% after 4 hours due to platelet aggregation, whereas about 60% of platelets remained in the APT-coated group even 24 hours after perfusion.

In addition, as illustrated in FIG. 4G, the expression of markers (CD63, PLSCR1, TBXAS1 and THBS1) associated with platelet aggregation was decreased in the APT-coated group.

Based on the above results, it was verified that aptamer coating enabled the formation of blood vessels capable of perfusion through more efficient vascular endothelialization, and minimized thrombus formation after blood perfusion.

## Example 5. Production of vascularized artificial liver using aptamer and functionality evaluation

In order to reconstruct a vascularized bioengineered human liver (VBHL) using aptamers, hepatic parenchymal cells (HepG2) and interstitial cells (hepatic stellate cells, mesenchymal stem cells), vascular endothelial cells (HUVEC), and the like were cultured by the method illustrated in FIG. 5A.

As a result, as illustrated in FIGS. 5B to 5D, it was confirmed by an albumin immunostaining method that reconstruction of the parenchymal part of the liver as well as more than 90% of vascular endothelialization was achieved in an artificial liver tissue (APT-VBHL) in which vascular endothelialization was performed after coating with an aptamer.

Furthermore, as illustrated in FIG. 5E, it was confirmed through an aSMA immunostaining method that mesenchymal stem cells were well engrafted in the perivascular region during aptamer treatment. In contrast, it was observed that the perivascular region was not developed in the uncoated or antibody-treated groups.

Further, as illustrated in FIG. 5F, it was confirmed that when dextran was flowed through the hepatic portal vein and intravascular dextran was quantified, dextran was significantly increased in the APT-VBHL group. Through this, it was confirmed that the vascular barrier function of liver tissue was maintained.

Therefore, not only vascular endothelialization but also reconstruction of the perivascular region that constitutes the vascular wall during aptamer treatment could result in obtaining highly functional blood vessels which maintain their barrier function well.

In addition, the degree of cell apoptosis in the artificial liver tissue was quantified through TUNEL assay.

As a result, as illustrated in FIG. 5L, the APT-VBHL group exhibited the least cell apoptosis, which is thus linked to the functionality of the artificial liver.

In addition, the NO production amount and the amount of VEGF secreted from the artificial liver were measured in order to confirm blood vessel-specific functionality.

As a result, as illustrated in FIGS. 5G and 5H, the APT-VBHL group showed excellent functionality compared to the other groups.

Furthermore, ELISA, which quantifies albumin and urea components secreted from the artificial liver, was performed in order to confirm liver-specific functionality.

As a result, as shown in FIGS. 5I and 5J, it was confirmed that the amounts of albumin and urea secreted on day 21 of culture were also the highest in the APT-VBHL group. In particular, since liver functionality showed a difference after blood vessels were reconstructed, it can be seen that highly functional revascularization through aptamer coating plays an important role in maintaining the overall functionality of the artificial liver tissue.

## Example 6. Evaluation of in vivo thrombus formation of aptamer-treated vascularized artificial liver

As illustrated in FIG. 6A, the degree of thrombus formation in the vascularized artificial liver *in vivo* was evaluated by connecting the hepatic portal vein and inferior vena cava of the artificial liver to the renal artery and renal vein of rats, respectively, to perfuse blood *in vivo.* Specifically, the renal vein (yellow arrow) was connected to the inferior vena cava of the VBHL construct and the renal artery (white arrow) was connected to the portal vein of the construct. After the vascular clamp (blue arrow) was removed, the VBHL construct was reperfused with a renal circulation system *in vivo.*

As a result, as illustrated in FIG. 6B, thrombus formation was not visually observed in the aptamer-treated artificial liver.

Further, as illustrated in FIGS. 6C and 6D, it was confirmed that the expression of integrin $\alpha$IIb, which is a thrombus marker, was also reduced.

In addition, as illustrated in FIG. 6E, when the expression of blood coagulation-related mRNA markers (Cd63, Plscr1, and Thbs1) was analyzed, the expression of blood coagulation-related markers was most decreased in the APT-VBHL group.

Therefore, based on the above results, it was verified that an artificial liver culture capable of minimizing thrombus formation *in vivo* was successfully achieved through aptamer treatment.

Therefore, it is expected that when the aptamer coating agent is used, the success rate of artificial liver transplantation can be increased by minimizing the most serious side effects that may occur after artificial liver transplantation.

## Example 7. Evaluation of in vivo liver functionality of aptamer-treated vascularized artificial liver

As illustrated in FIG. 7A, the liver-specific functionality *in vivo* was evaluated by transplanting a vascularized artificial liver into a rat model in which hepatic fibrosis was induced using thioacetamide (TAA). Eight weeks after TAA induction, rats received the xenograft transplantation of decellularized liver matrix (DLM transplant) or VBHL constructs (CTL-VBHL; CTL transplant, APT-VBHL; APT transplant, Ab-VBHL; Ab transplant).

Specific experimental groups are as follows:

1) Sham
2) DLM implanted; implantation of cell-free decellularized scaffold alone
3) CTL implanted; implantation of artificial liver reconstructed without coating
4) APT implanted; implantation of artificial liver treated with anti-CD31 aptamer as coating agent
5) Ab implanted; implantation of artificial liver treated with anti-CD31 antibody as coating agent

Four weeks later, the livers of the recipient rats were harvested and subjected to H&E tissue staining and Picrosirius red tissue staining (red: collagen, green: cell cytoplasm).

As a result, as illustrated in FIGS. 7B and 7C, when the decellularized scaffold and the artificial liver were transplanted, the eosinophilic change and degree of fibrosis in the liver were decreased compared to the sham group. Among them, it was confirmed that the degree of liver fibrosis was reduced most when an artificial liver treated with the aptamer was transplanted.

In addition, as illustrated in FIG. 7D, it was confirmed that when the mRNA expressed in the liver of the recipient was analyzed, the expression of fibrosis-related markers ($\alpha$-smooth muscle actin(Sma), Vimentin, Tgf-beta1, and Timp1) was clearly decreased in the APT implanted group.

Furthermore, as illustrated in FIGS. 7E and 7F, it was confirmed that when the ALT and AST levels, which are

indicators of liver injury, were confirmed in the serum levels of rats. The serum ALT and AST levels after surgery in the APT implanted group were maintained at normal levels (between the red lines).

Therefore, the aptamer-treated artificial liver demonstrates that enhanced liver-specific functionality is maintained in vivo, which assists the recipient's impaired liver function.

As described above, the present inventors confirmed for the first time that aptamers can be applied to artificial organs.

Based on the above results, when an aptamer is used as a coating agent for decellularized scaffolds, vasculature can be reconstructed more efficiently than anti-CD31 antibodies used in existing studies by enhancing blood vessel adhesion ability, viability, angiogenesis potential, and the like through the integrin-Akt signaling system. Therefore, a vascularized artificial liver produced using the present invention not only reduces thrombus formation in vivo, but also exhibits the effect of enhancing liver functionality. Further, this is a result that suggests the applicability of the artificial liver as a therapeutic agent for liver disease in terms of tissue engineering.

It should be understood that the above-described embodiments are only exemplary in all aspects and are not restrictive.

[Industrial Applicability]

When the aptamer of use in accordance with the present invention is used as a coating agent of a decellularized scaffold, it is possible to more efficiently reconstruct vasculature than existing antibodies by enhancing blood vessel adhesion ability, viability, angiogenesis potential, and the like. Therefore, a vascularized artificial liver produced using the aptamers exhibits the effects of reducing thrombogenesis in vivo as well as enhancing liver function, and thus is expected to be useful for treating diseases by using artificial organs produced using the aptamers as described herein, so that there is industrial applicability.

## Claims

1. An *ex vivo* use of a composition comprising anti-CD31 aptamers for coating blood vessels of a decellularized liver, wherein the anti-CD31 aptamers comprise a base sequence represented by SEQ ID NO: 1.

2. The use of claim 1, wherein the composition further comprises one or more cells selected from the group consisting of parenchymal cells and non-parenchymal cells.

3. The use of claim 2, wherein the cells comprise stem cell-derived differentiated cells.

4. The use of claim 3, wherein the stem cells are one or more selected from the group consisting of induced pluripotent stem cells (iPSCs), embryonic stem cells, mesenchymal stromal cells (MSCs), off-the-shelf universal stem cells (USCs), marrow-derived stem cells, adipose tissue-derived stem cells and placenta-derived stem cells.

5. The use of claim 4, wherein the off-the-shelf universal stem cells have one or more of the following characteristics:

   human leukocyte antigen (HLA) gene has been removed; and
   "Don't eat me" signals are overexpressed.

6. An *ex vivo* method for coating blood vessels of a decellularized liver, the method comprising a step of treating with a composition comprising anti-CD31 aptamers, wherein the anti-CD31 aptamers comprise a base sequence represented by SEQ ID NO: 1.

7. The method of claim 6, wherein the method further comprises the following steps:

   a) providing a liver from a tissue source;
   b) decellularizing the provided liver; and
   c) treating the decellularized liver with the anti-CD31 aptamers.

8. The method of claim 7, wherein the method further comprises one or more steps selected from the group consisting of the following steps:

   d-1) recellularizing a decellularized liver into vascular endothelial cells;

d-2) recellularizing a decellularized liver into parenchymal cells; and
d-3) recellularizing a decellularized liver into non-parenchymal cells.

## Patentansprüche

1. *Ex-vivo*-Verwendung einer Zusammensetzung, die Anti-CD31-Aptamere zur Beschichtung von Blutgefäßen einer dezellularisierten Leber umfasst, wobei die Anti-CD31-Aptamere eine Basissequenz umfassen, die durch SEQ ID NO: 1 dargestellt wird.

2. Verwendung nach Anspruch 1, wobei die Zusammensetzung weiter eine oder mehrere Zellen umfasst, die aus der Gruppe ausgewählt sind, die aus Parenchymzellen und Nicht-Parenchymzellen besteht.

3. Verwendung nach Anspruch 2, wobei die Zellen aus Stammzellen gewonnene differenzierte Zellen umfassen.

4. Verwendung nach Anspruch 3, wobei die Stammzellen eine oder mehrere sind, die aus der Gruppe ausgewählt sind, die aus induzierten pluripotenten Stammzellen (iPSCs), embryonalen Stammzellen, mesenchymalen Stromazellen (MSCs), handelsüblichen universellen Stammzellen (USCs), aus dem Knochenmark gewonnenen Stammzellen, aus dem Fettgewebe gewonnenen Stammzellen und aus der Plazenta gewonnenen Stammzellen besteht.

5. Verwendung nach Anspruch 4, wobei die handelsüblichen universellen Stammzellen eines oder mehrere der folgenden Merkmale aufweisen:

   das humane Leukozytenantigen- (HLA) -Gen wurde entfernt; und
   "Don't eat me"-Signale werden übermäßig stark exprimiert.

6. Ex-vivo-Verfahren zum Beschichten von Blutgefäßen einer dezellularisierten Leber, wobei das Verfahren einen Schritt der Behandlung mit einer Zusammensetzung umfasst, die Anti-CD31-Aptamere umfasst, wobei die Anti-CD31-Aptamere eine Basissequenz umfassen, die durch die SEQ ID NO: 1 dargestellt wird.

7. Verfahren nach Anspruch 6, wobei das Verfahren weiter die folgenden Schritte umfasst:

   a) Bereitstellen einer Leber aus einer Gewebequelle;
   b) Dezellularisieren der bereitgestellten Leber; und
   c) Behandeln der dezellularisierten Leber mit den Anti-CD31-Aptameren.

8. Verfahren nach Anspruch 7, wobei das Verfahren weiter einen oder mehrere Schritte umfasst, die aus der Gruppe ausgewählt sind, die aus den folgenden Schritten besteht:

   d-1) Rezellularisieren einer dezellularisierten Leber mit vaskulären Endothelzellen;
   d-2) Rezellularisieren einer dezellularisierten Leber mit Parenchymzellen; und
   d-3) Rezellularisieren einer dezellularisierten Leber mit Nicht-Parenchymzellen.

## Revendications

1. Utilisation *ex vivo* d'une composition comprenant des aptamères anti-CD31 pour recouvrir des vaisseaux sanguins d'un foie décellularisé, dans laquelle les aptamères anti-CD31 comprennent une séquence de base représentée par la SEQ ID NO : 1.

2. Utilisation selon la revendication 1, dans laquelle la composition comprend en outre une ou plusieurs cellules sélectionnées dans le groupe consistant en des cellules parenchymateuses et des cellules non parenchymateuses.

3. Utilisation selon la revendication 2, dans laquelle les cellules comprennent des cellules différenciées dérivées de cellules souches.

4. Utilisation selon la revendication 3, dans laquelle les cellules souches sont un ou plusieurs éléments sélectionnés dans le groupe consistant en des cellules souches pluripotentes induites (iPSC), des cellules souches embryonnaires, des cellules stromales mésenchymateuses (MSC), des cellules souches universelles (USC) prêtes à l'emploi, des cellules souches dérivées de moelle osseuse, des cellules souches dérivées de tissus adipeux et des cellules souches dérivées de placenta.

**5.** Utilisation selon la revendication 4, dans laquelle les cellules souches universelles prêtes à l'emploi présentent une ou plusieurs des caractéristiques suivantes :

le gène de l'antigène leucocytaire humain (HLA) a été supprimé ; et
des signaux « Ne me mangez pas » sont en surexpression.

**6.** Procédé *ex vivo* de revêtement de vaisseaux sanguins d'un foie décellularisé, le procédé comprenant une étape de traitement avec une composition comprenant des aptamères anti-CD31, dans lequel les aptamères anti-CD31 comprennent une séquence de base représentée par la SEQ ID NO : 1.

**7.** Procédé selon la revendication 6, dans lequel le procédé comprend en outre les étapes suivantes :

a) la fourniture d'un foie à partir d'une source tissulaire ;
b) la décellularisation du foie fourni ; et
c) le traitement du foie décellularisé avec les aptamères anti-CD31.

**8.** Procédé selon la revendication 7, dans lequel le procédé comprend en outre une ou plusieurs étapes sélectionnées dans le groupe consistant en les étapes suivantes :

d-1) la recellularisation d'un foie décellularisé en cellules endothéliales vasculaires ;
d-2) la recellularisation d'un foie décellularisé en cellules parenchymateuses ; et
d-3) la recellularisation d'un foie décellularisé en cellules non parenchymateuses.

【Fig. 1 a】

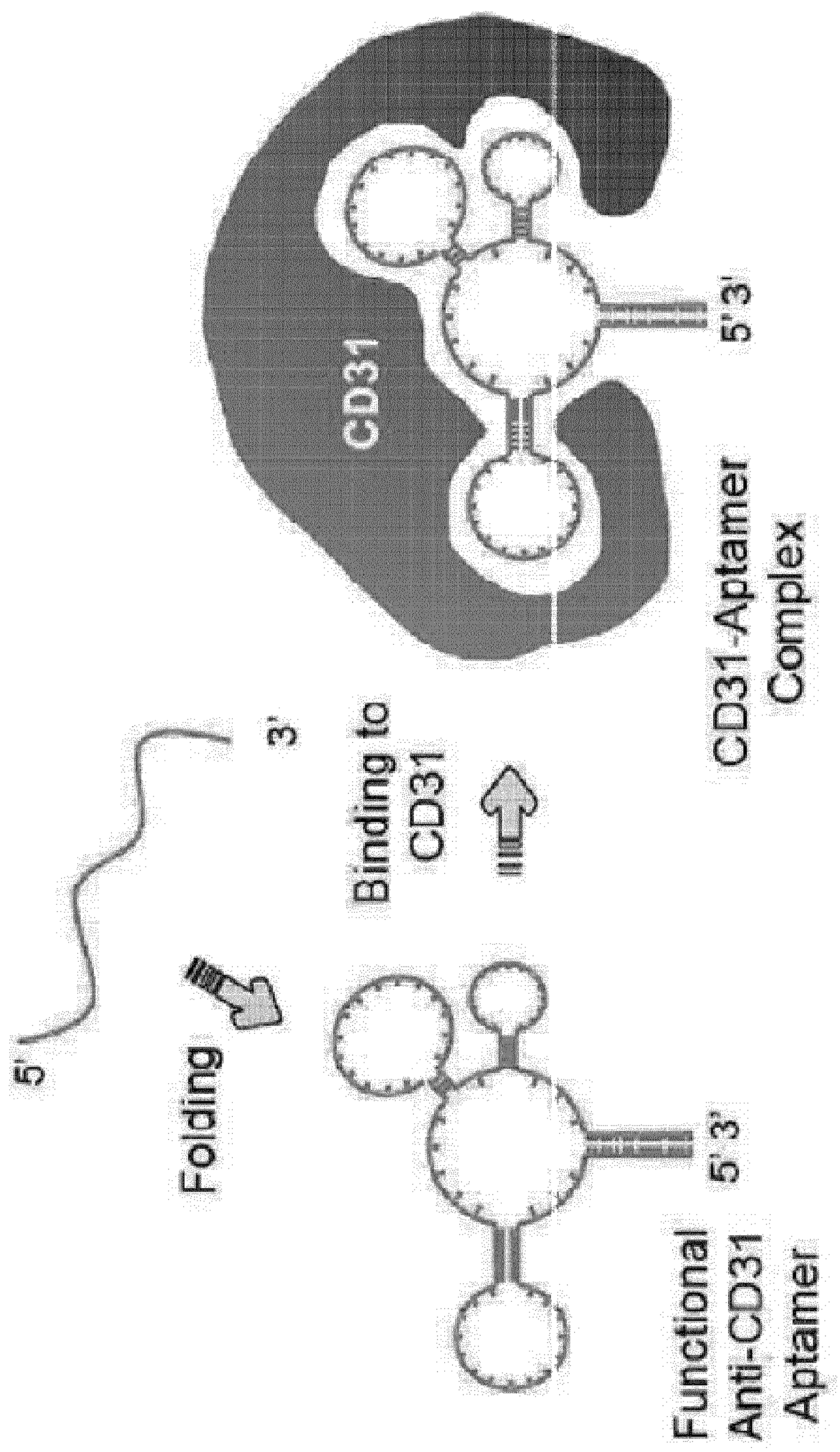
5'
3'
Folding
Binding to
CD31
CD31
Functional
Anti-CD31
Aptamer
5' 3'
5' 3'
CD31-Aptamer
Complex

【Fig. 1 b】

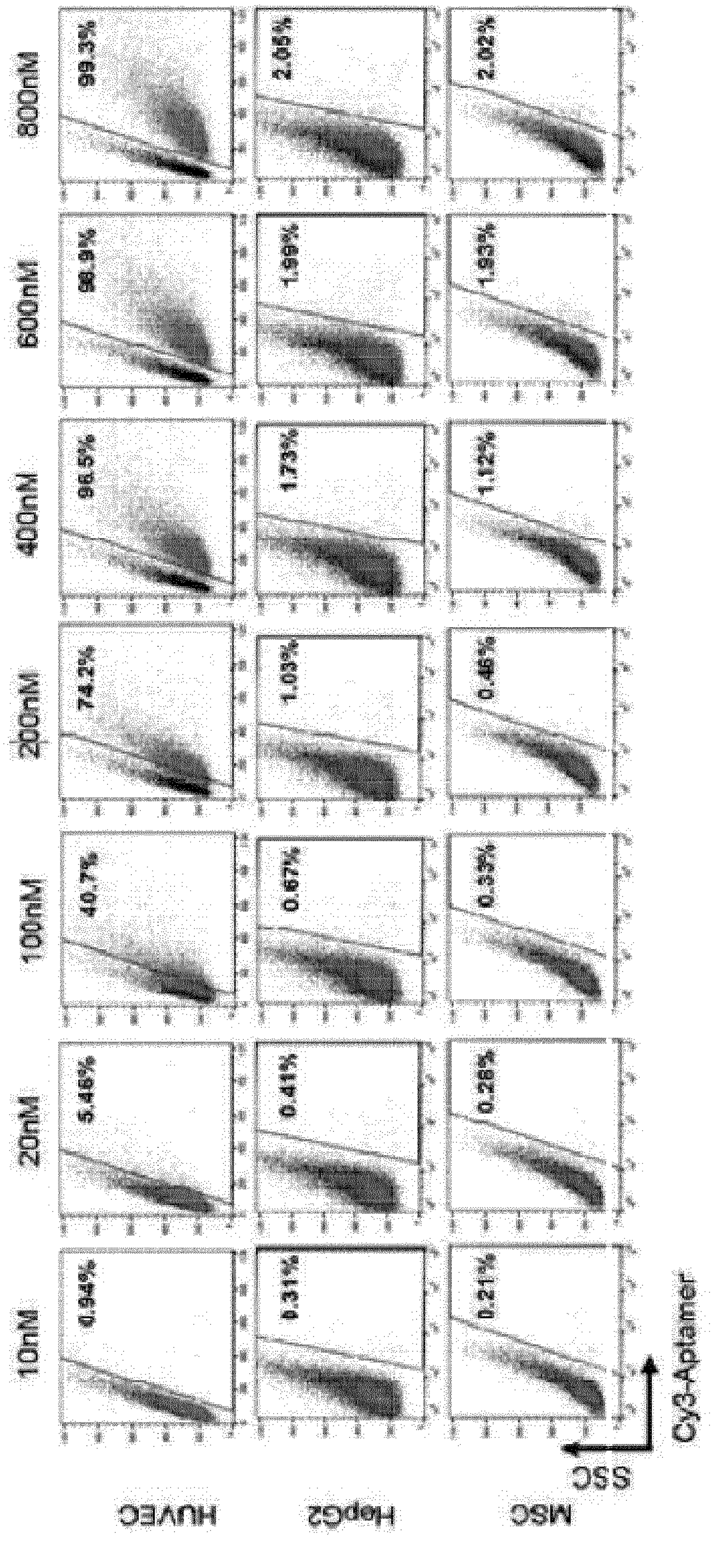
10nM
20nM
100nM
200nM
400nM
600nM
800nM
HUVEC
HepG2
MSC
0.94%
5.46%
40.7%
74.2%
96.5%
98.9%
99.3%
0.31%
0.41%
0.67%
1.03%
1.73%
1.99%
2.05%
0.21%
0.28%
0.33%
0.46%
1.12%
1.93%
2.02%
SSC
Cy3-Aptamer

【Fig. 1 c】

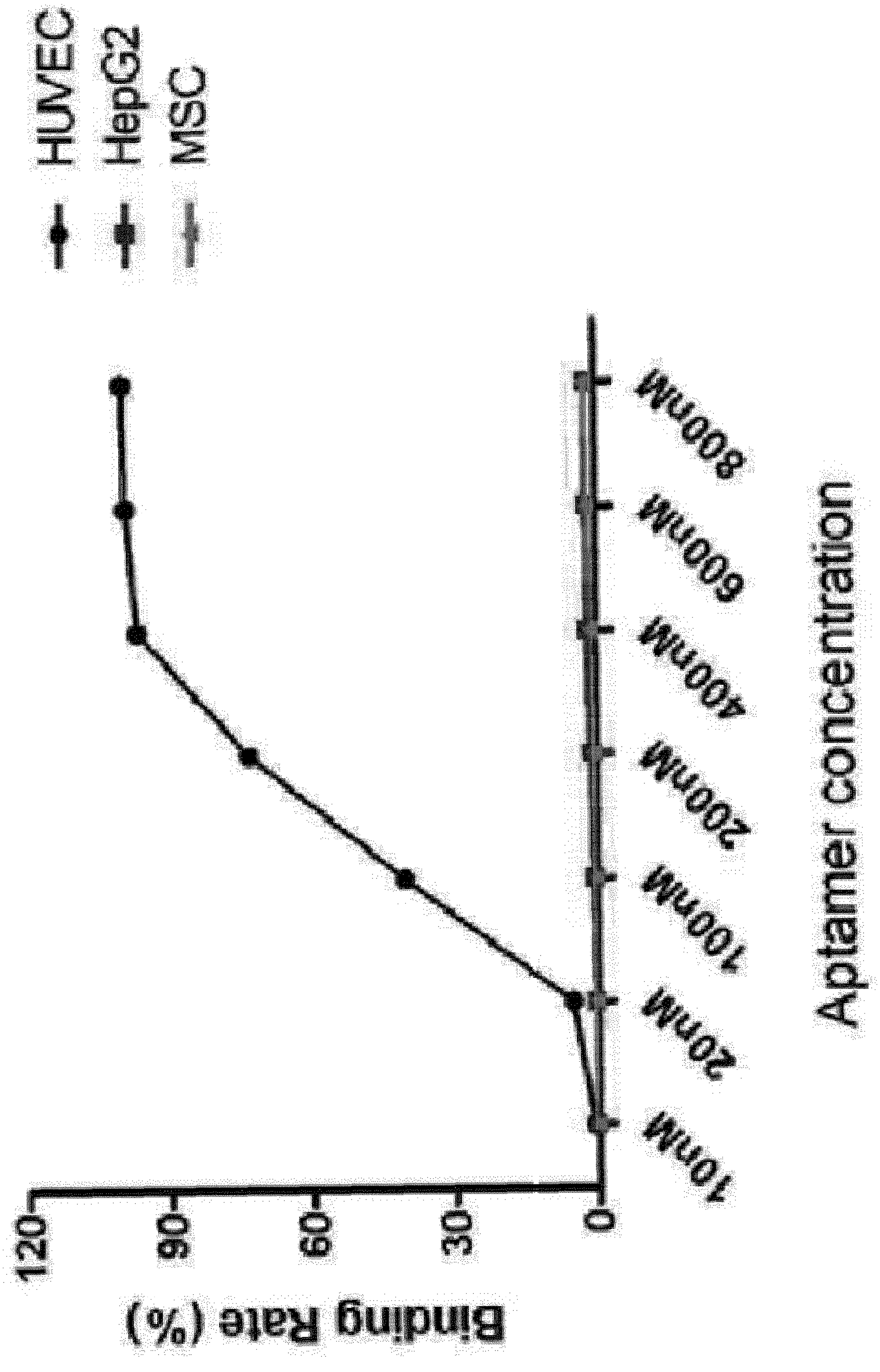
HUVEC
HepG2
MSC
Binding Rate (%)
120
90
60
30
0
10nM
20nM
100nM
200nM
400nM
600nM
800nM
Aptamer concentration

【Fig. 1 d】

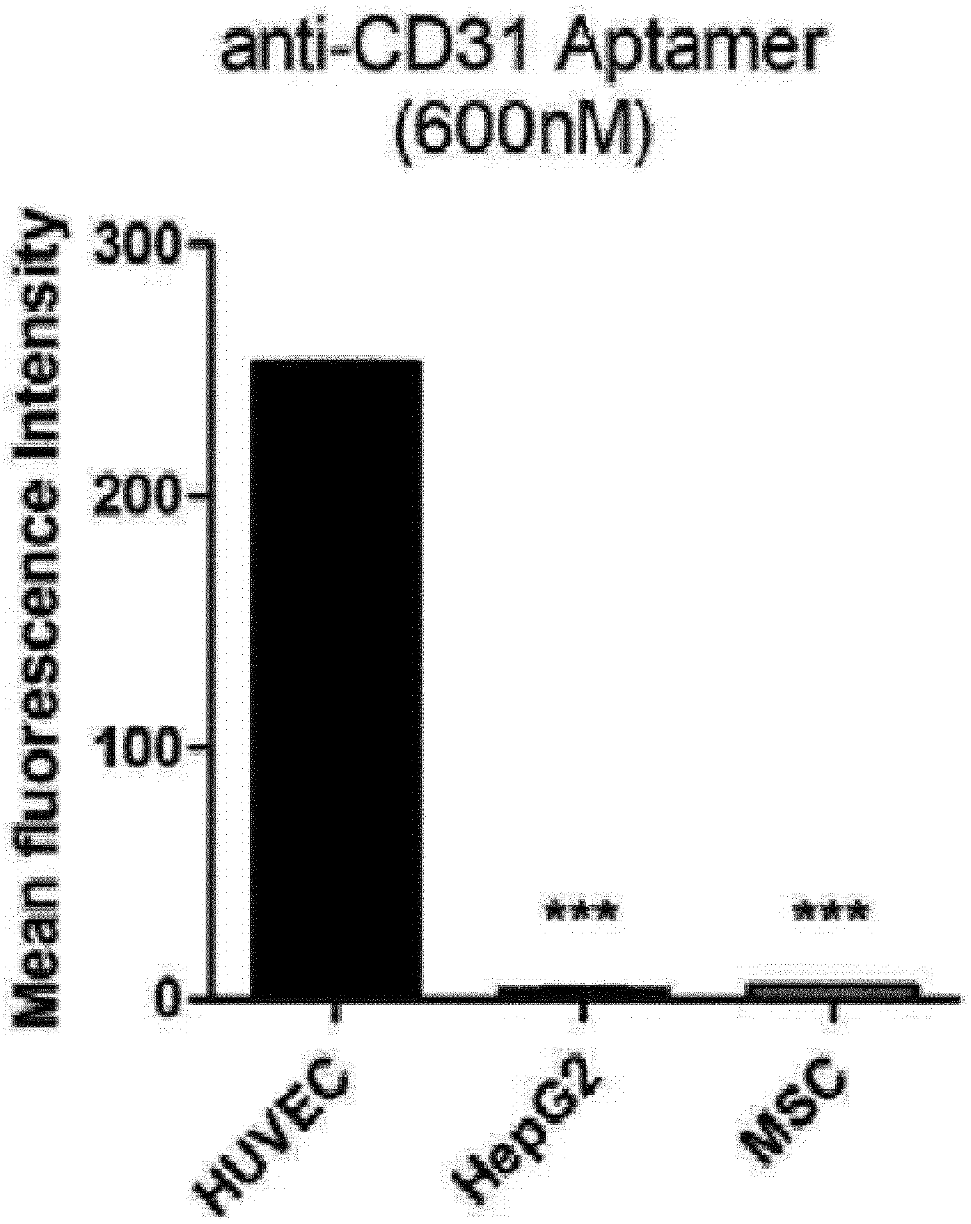
anti-CD31 Aptamer
(600nM)
Mean fluorescence Intensity
300
200
100
0
***
***
HUVEC
HepG2
MSC

【Fig. 1 e】

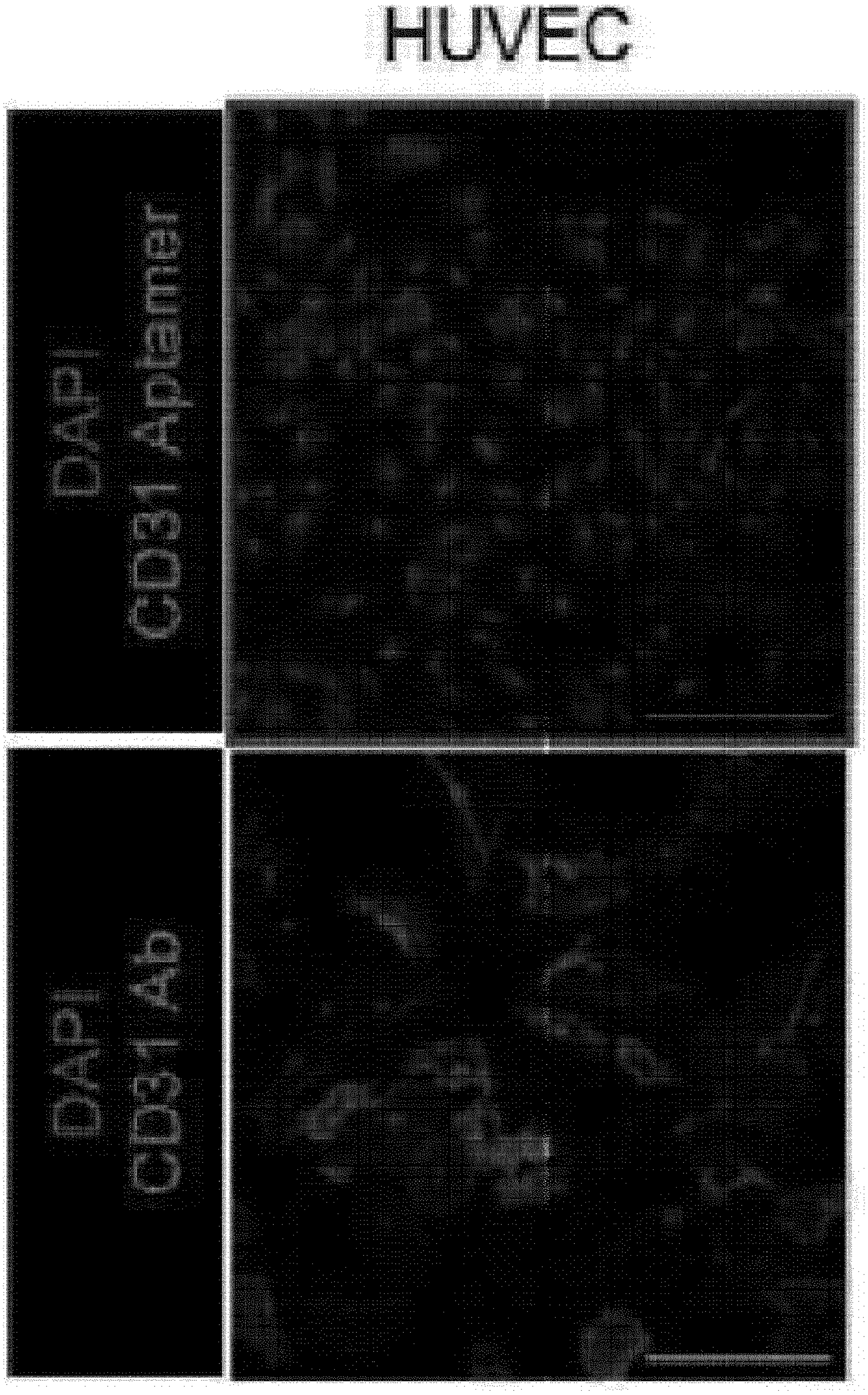

【Fig. 1 f】

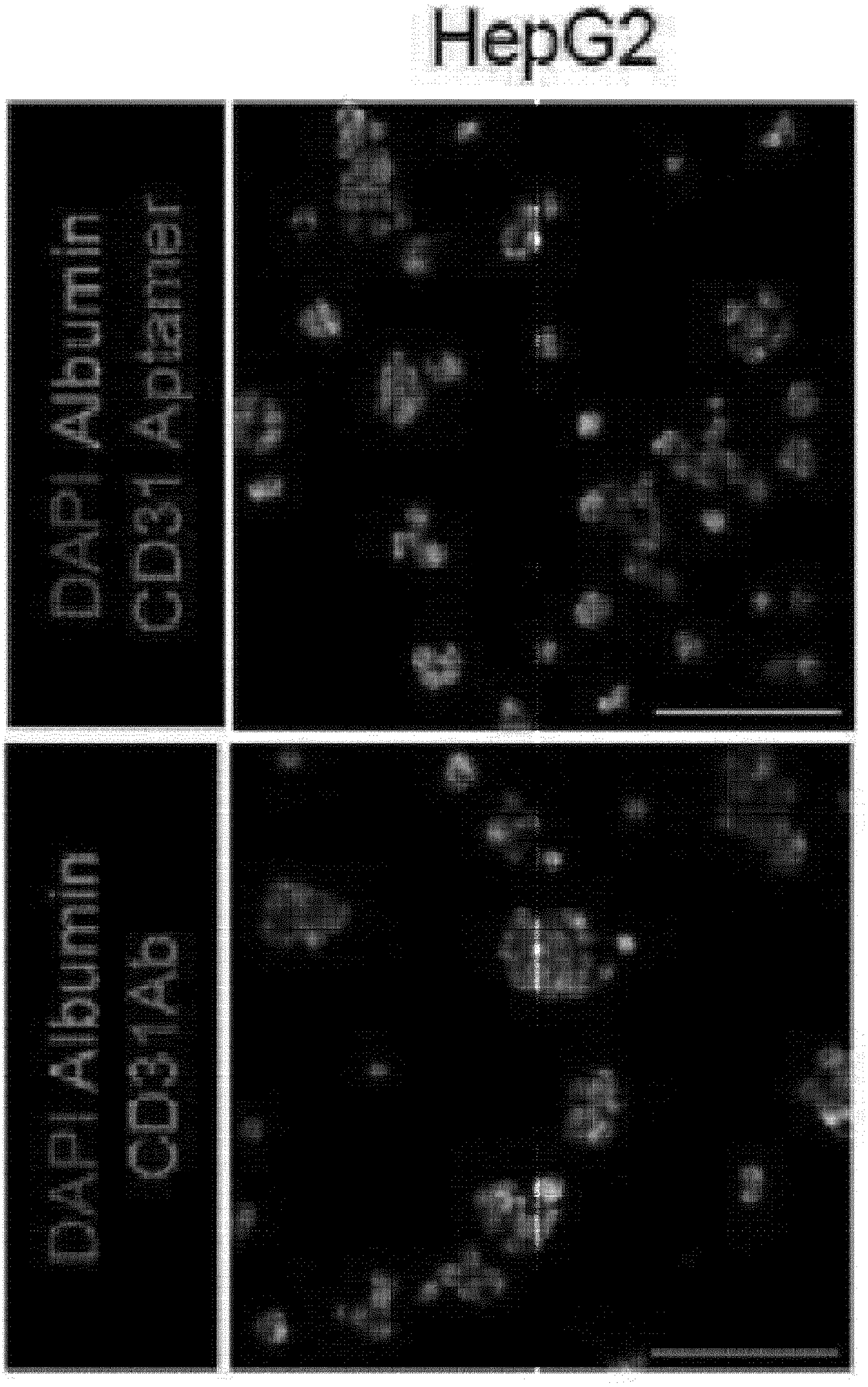

【Fig. 1 g】

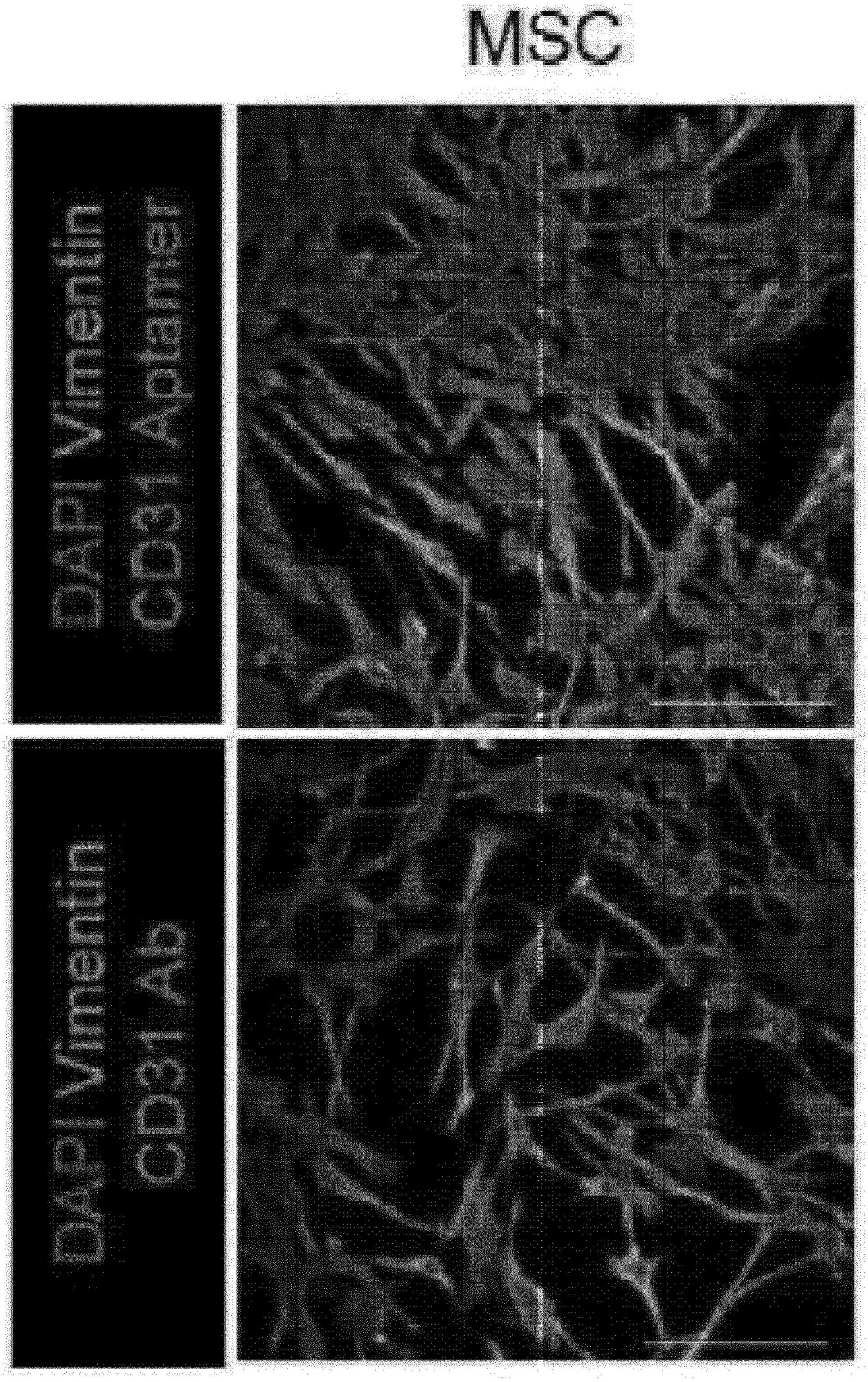

【Fig. 2 a】

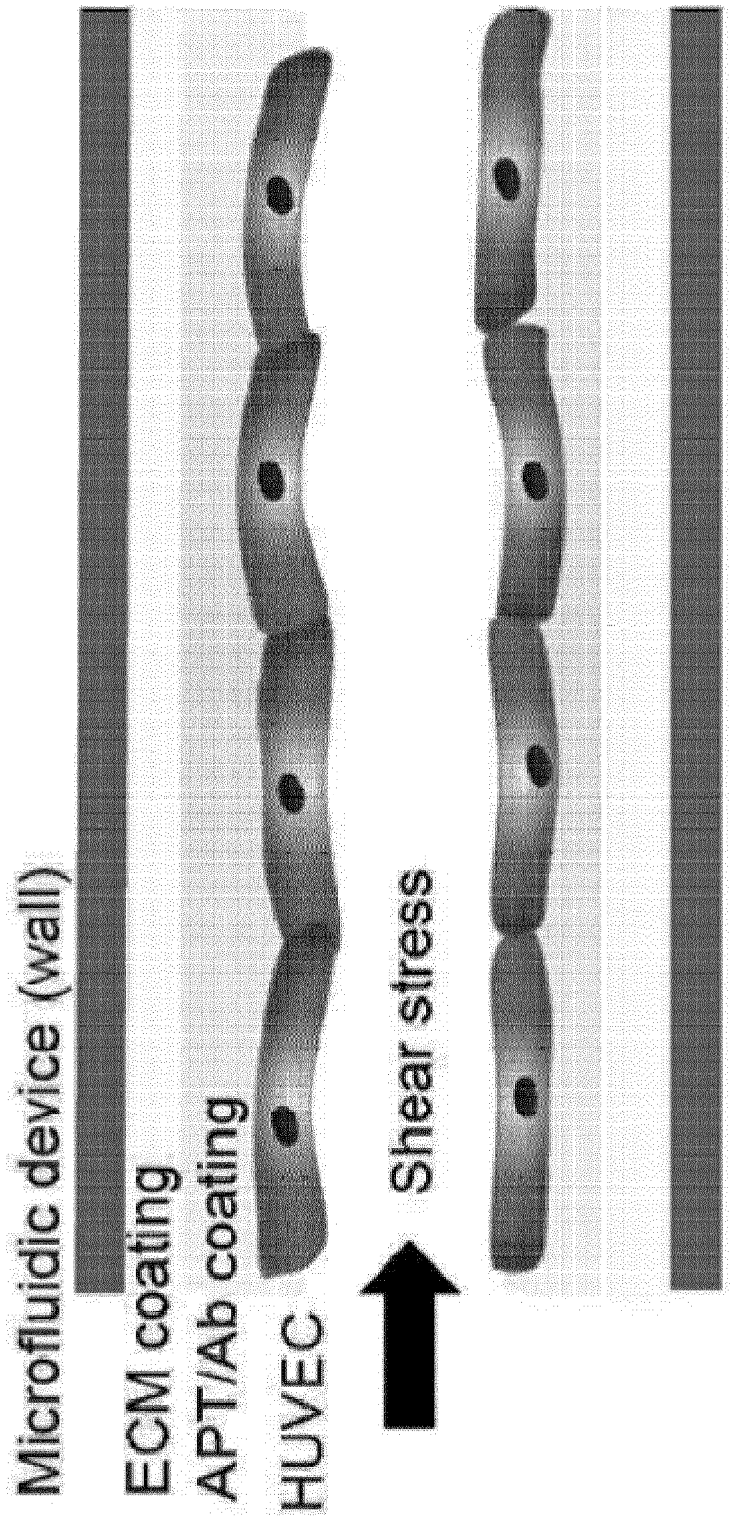
Microfluidic device (wall)
ECM coating
APT/Ab coating
HUVEC
Shear stress

【Fig. 2 b】

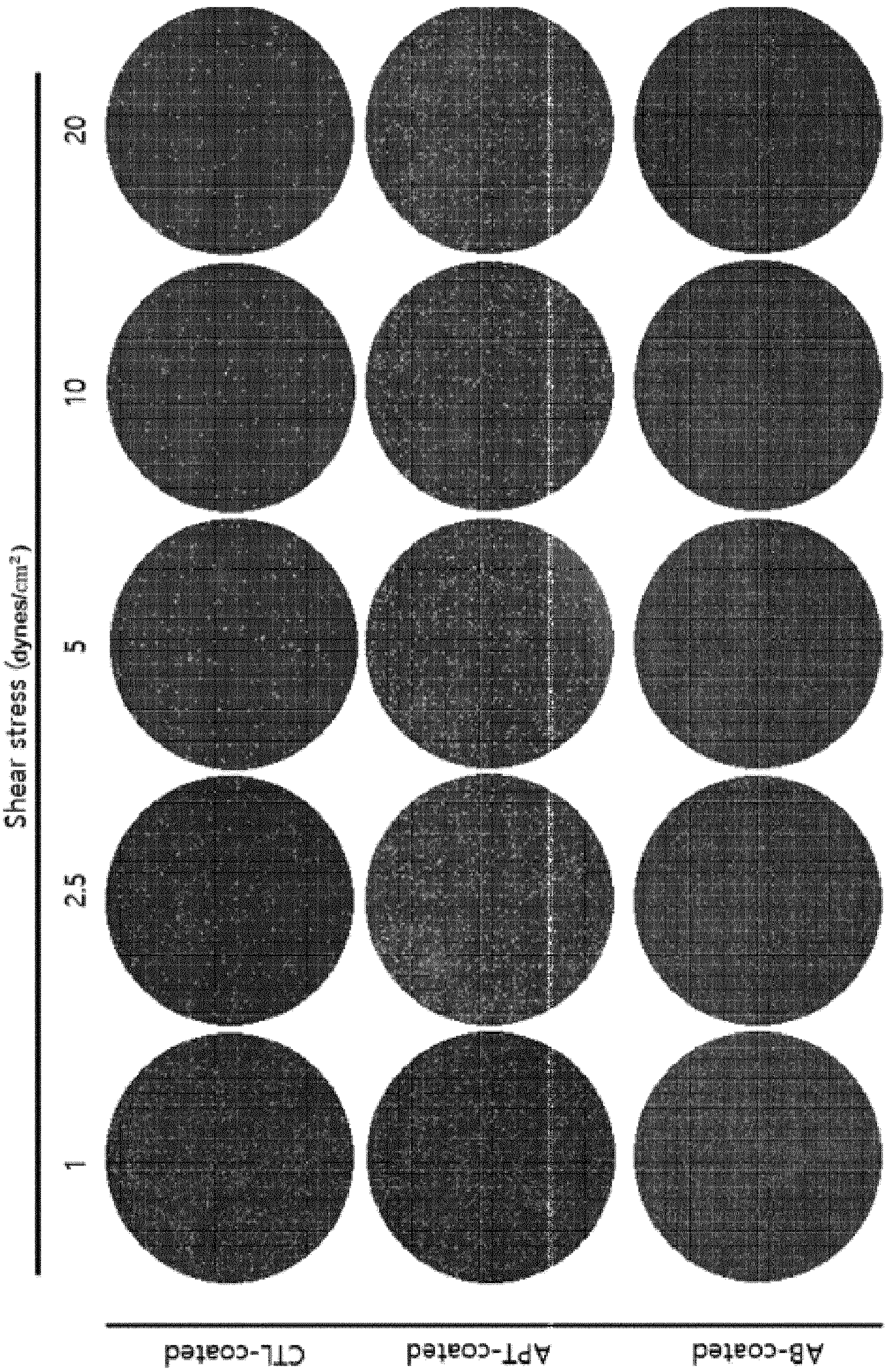
Shear stress (dynes/cm²)
1
2.5
5
10
20
CTL-coated
APT-coated
AB-coated

【Fig. 2 c】

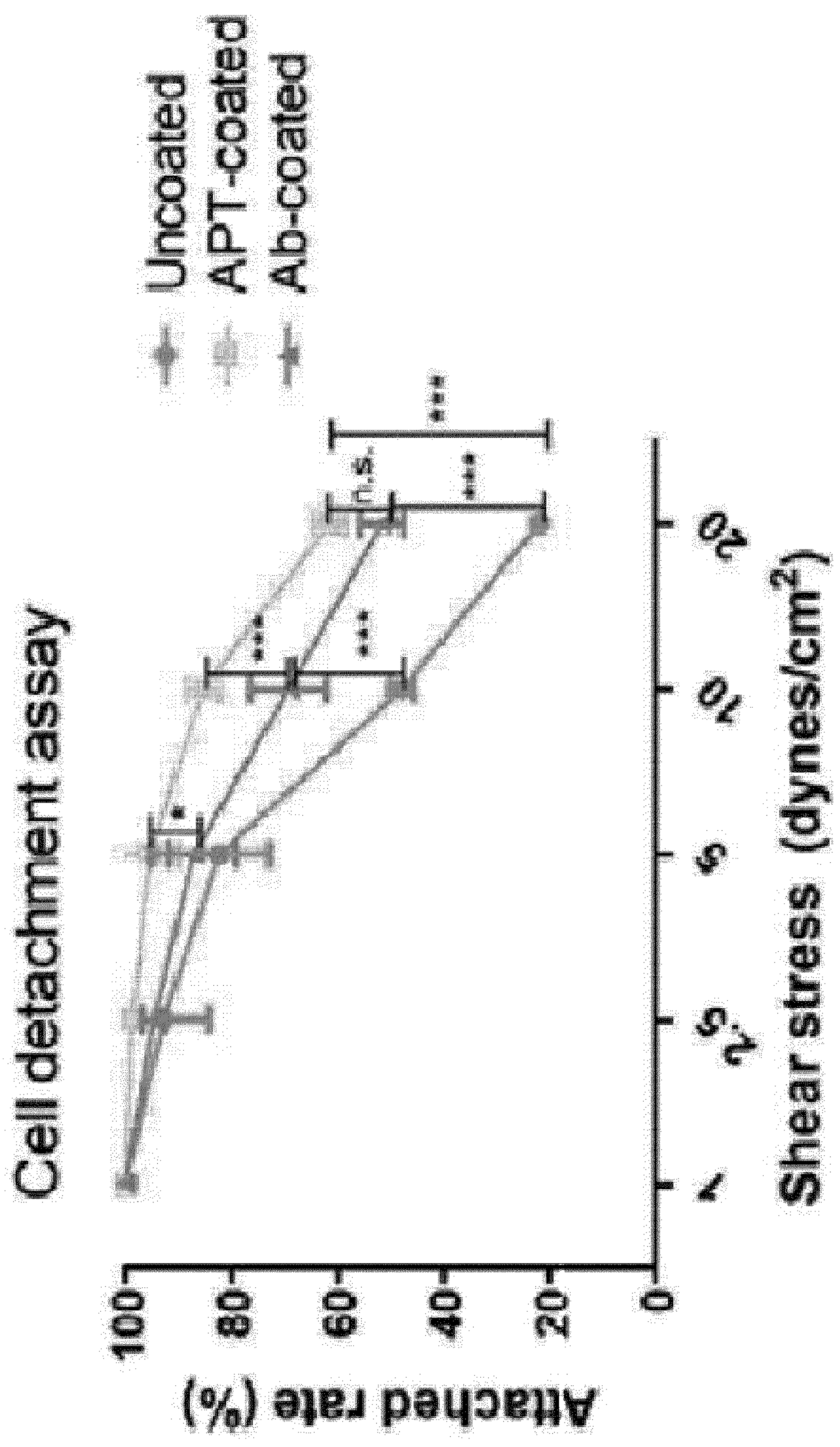
Cell detachment assay
Attached rate (%)
100
80
60
40
20
0
1
2.5
5
10
20
Shear stress (dynes/cm2)
*
***
***
n.s.
***
***
Uncoated
APT-coated
Ab-coated

【Fig. 2 d】

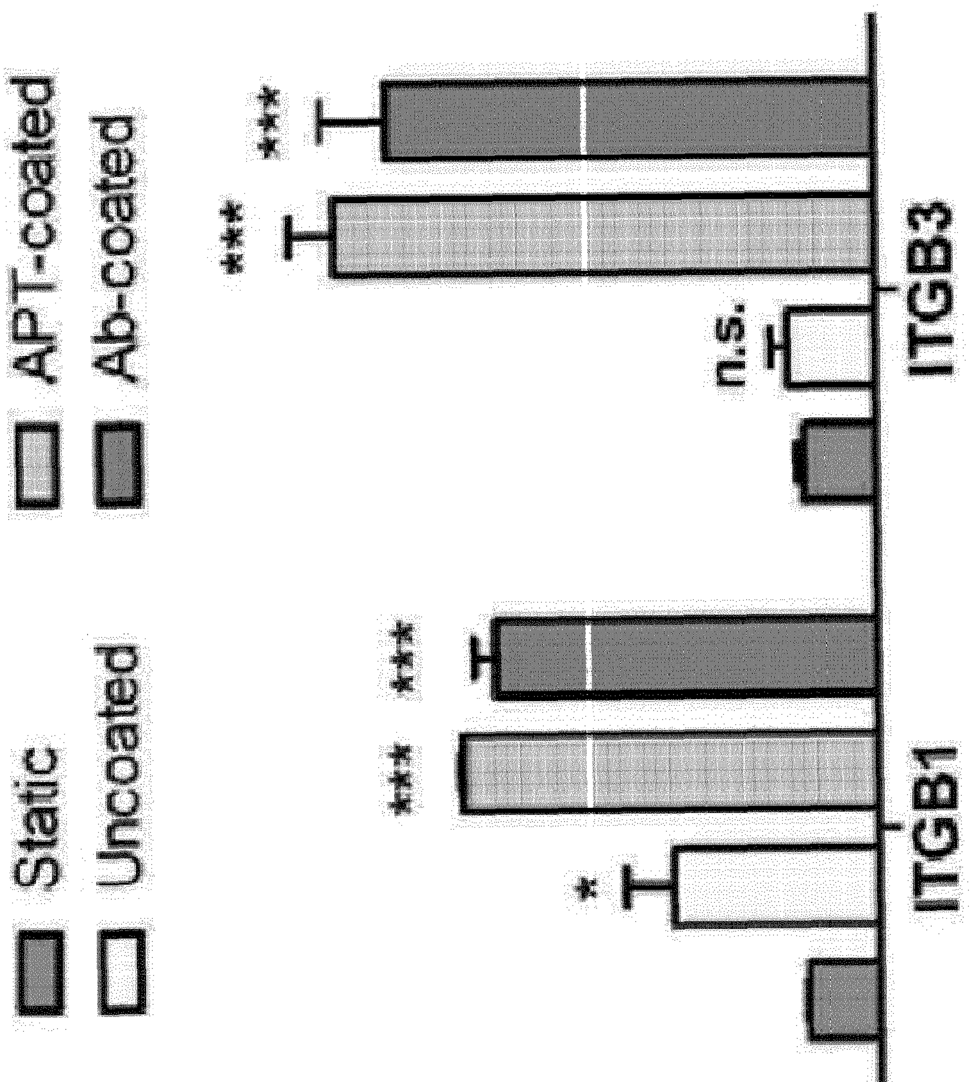
Static
Uncoated
APT-coated
Ab-coated
Relative mRNA expression
10
8
6
4
2
0
*
***
***
n.s.
***
***
ITGB1
ITGB3

【Fig. 2 e】

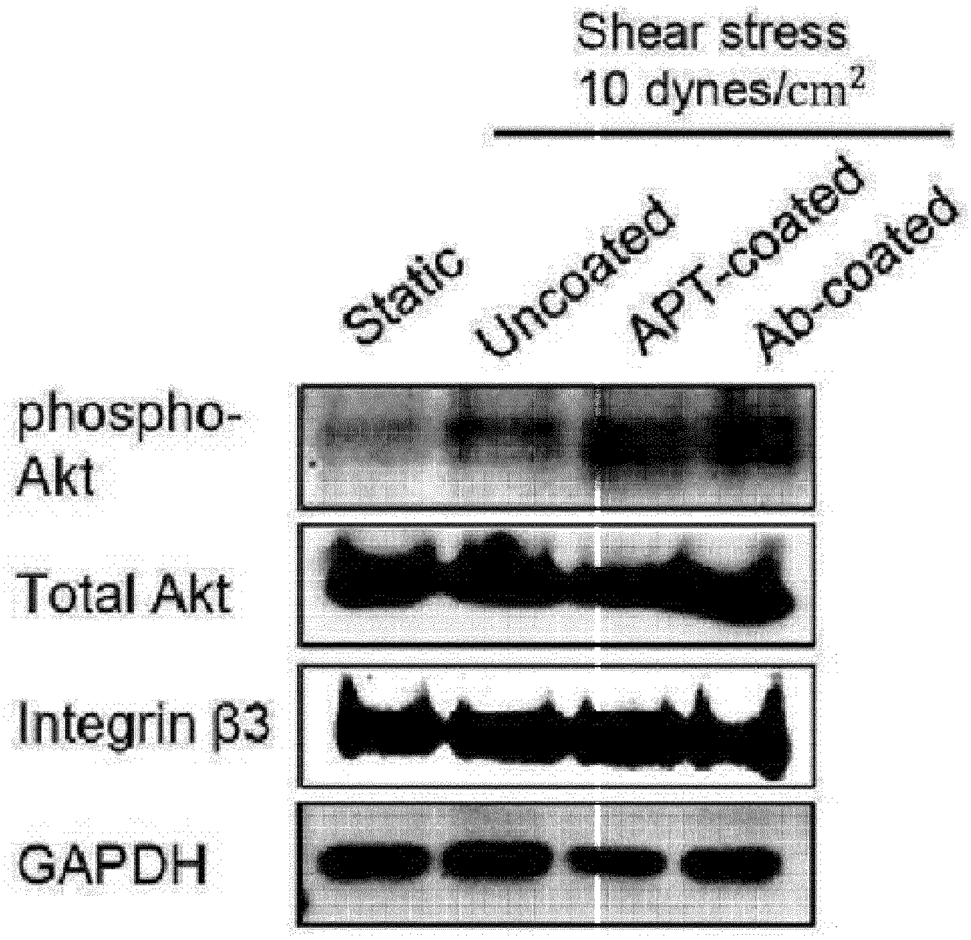
Shear stress
10 dynes/cm²
Static
Uncoated
APT-coated
Ab-coated
phospho-Akt
Total Akt
Integrin β3
GAPDH

【Fig. 2 f】

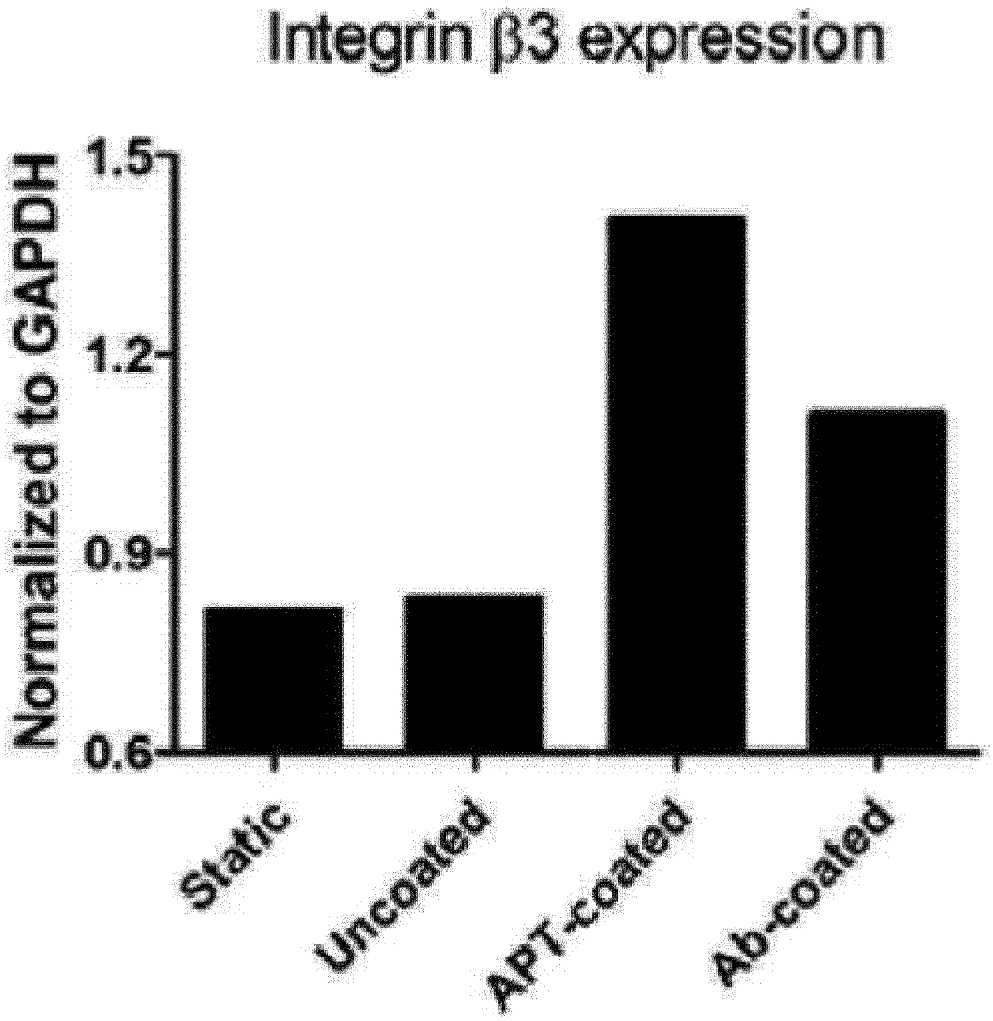
Integrin β3 expression
Normalized to GAPDH
1.5
1.2
0.9
0.6
Static
Uncoated
APT-coated
Ab-coated

【Fig. 2 g】

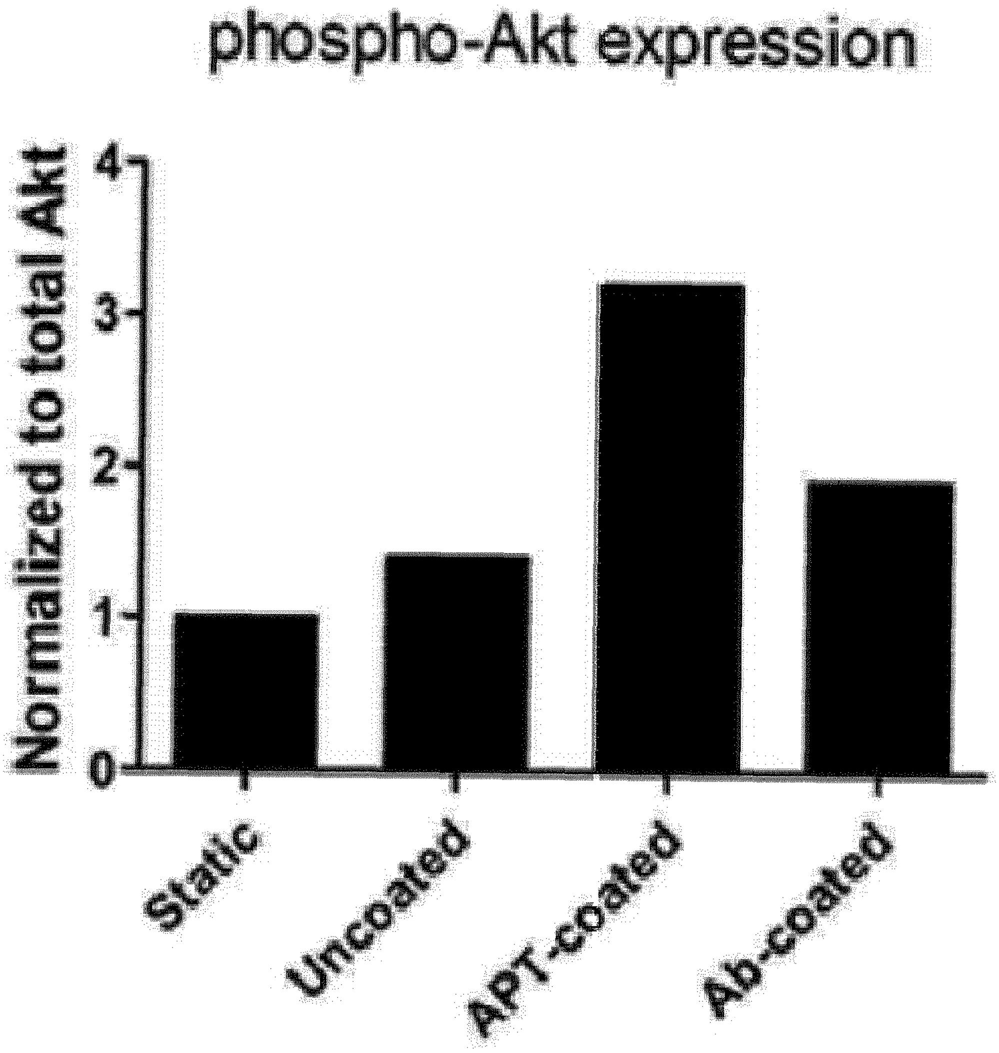
phospho-Akt expression
Normalized to total Akt
4
3
2
1
0
Static
Uncoated
APT-coated
Ab-coated

【Fig. 2 h】

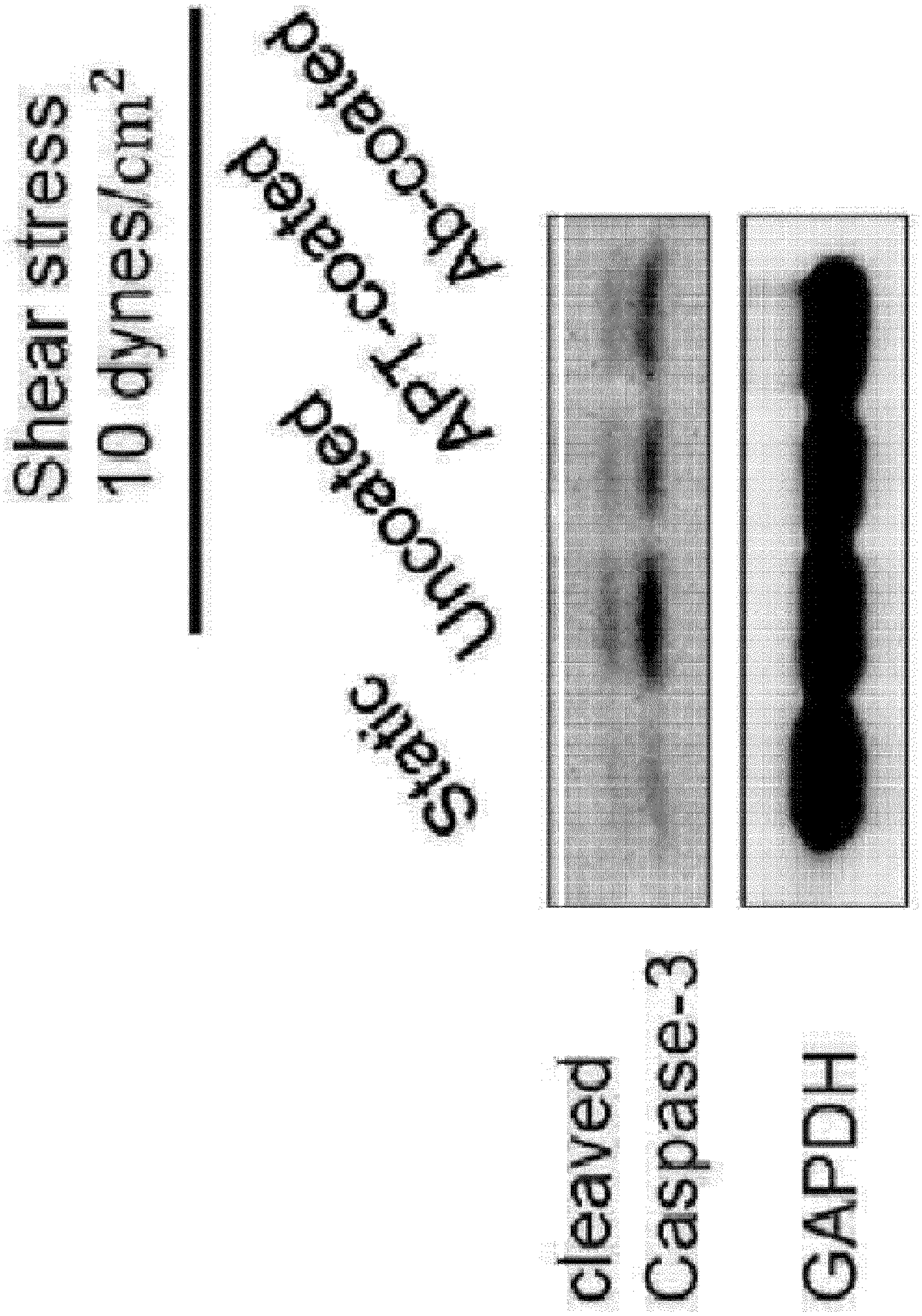
Shear stress
10 dynes/cm2
Ab-coated
APT-coated
Uncoated
Static
cleaved
Caspase-3
GAPDH

【Fig. 2 i】

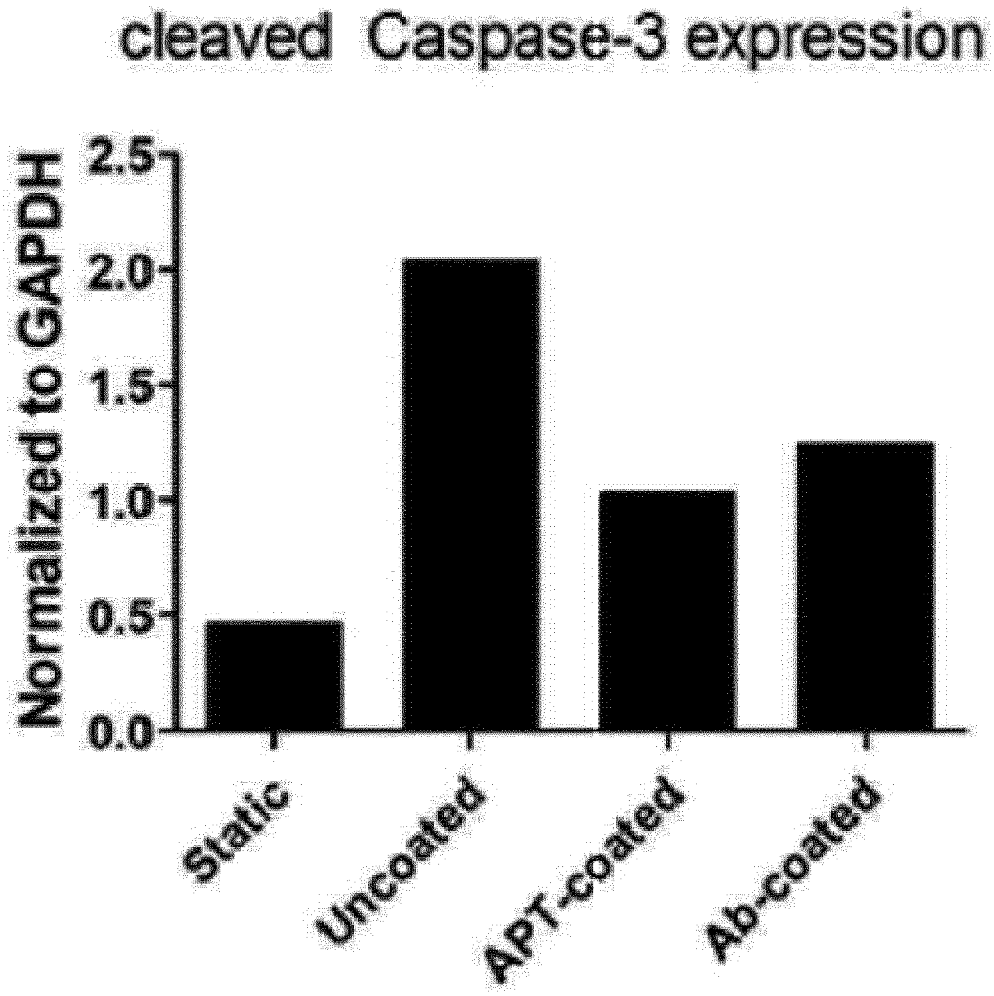
cleaved Caspase-3 expression
Normalized to GAPDH
2.5
2.0
1.5
1.0
0.5
0.0
Static
Uncoated
APT-coated
Ab-coated

【Fig. 2 j】

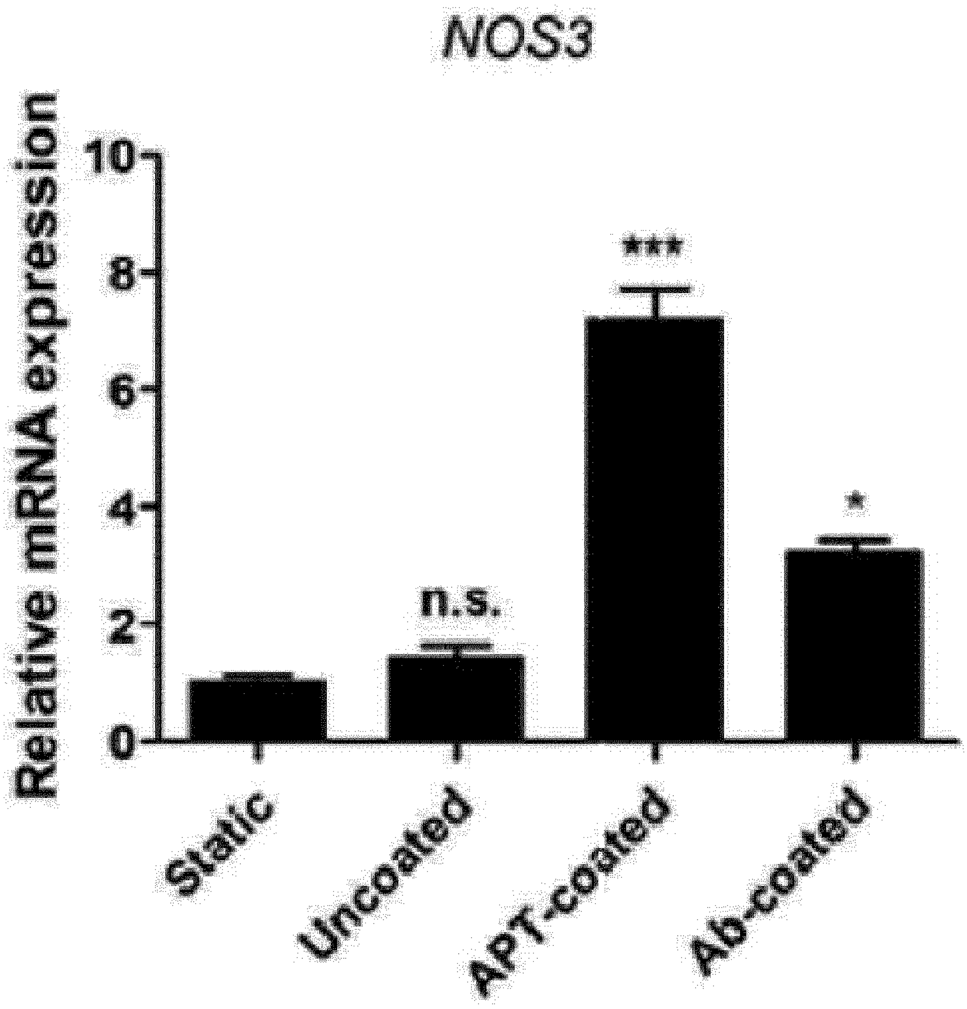
NOS3
Relative mRNA expression
10
8
6
4
2
0
***
*
n.s.
Static
Uncoated
APT-coated
Ab-coated

【Fig. 3 a】

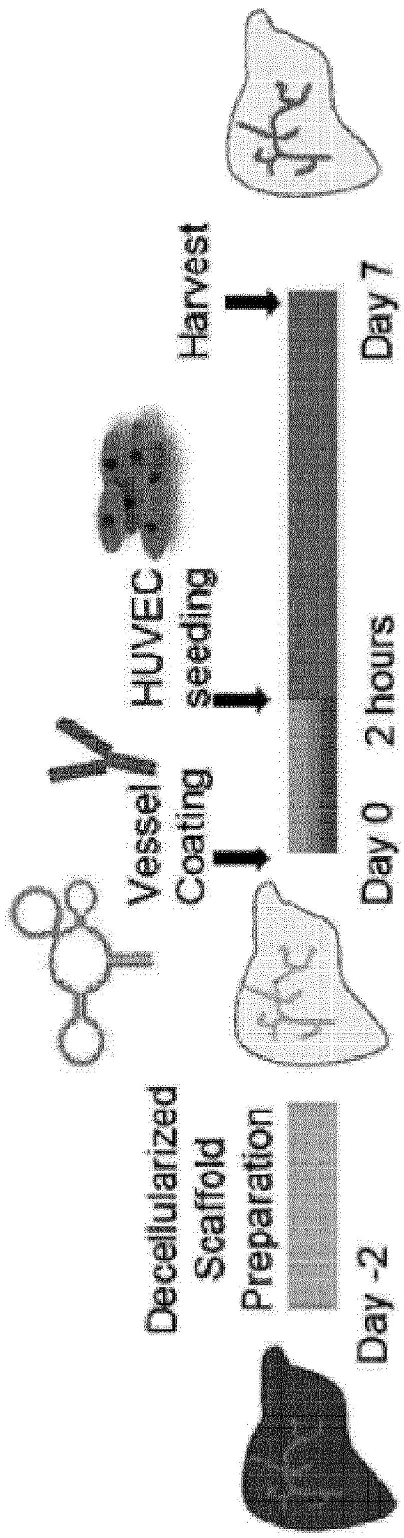
Decellularized
Scaffold
Preparation
Day -2
Vessel
Coating
Day 0
2 hours
HUVEC
seeding
Harvest
Day 7

【Fig. 3 b】

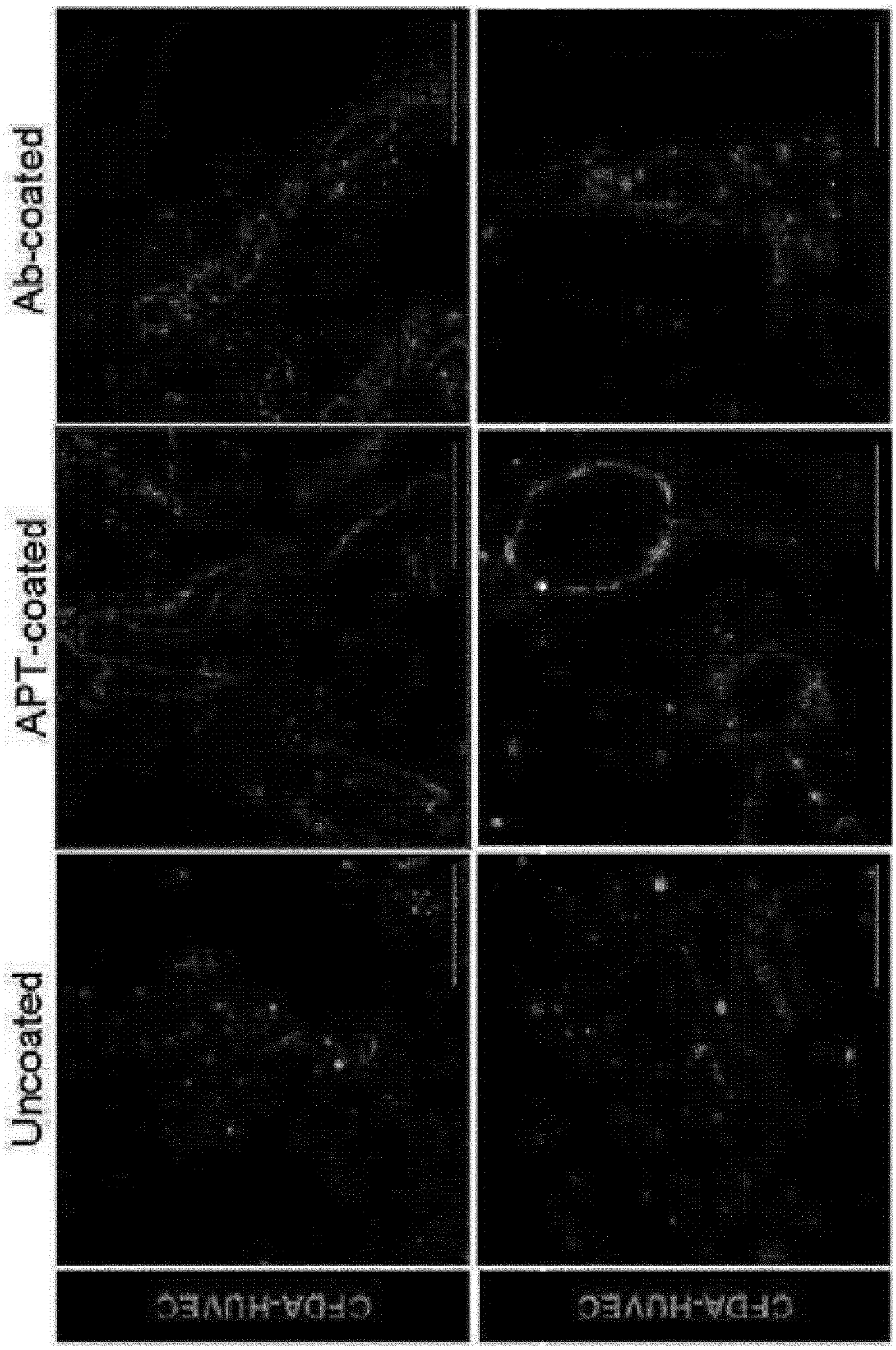

【Fig. 3 c】

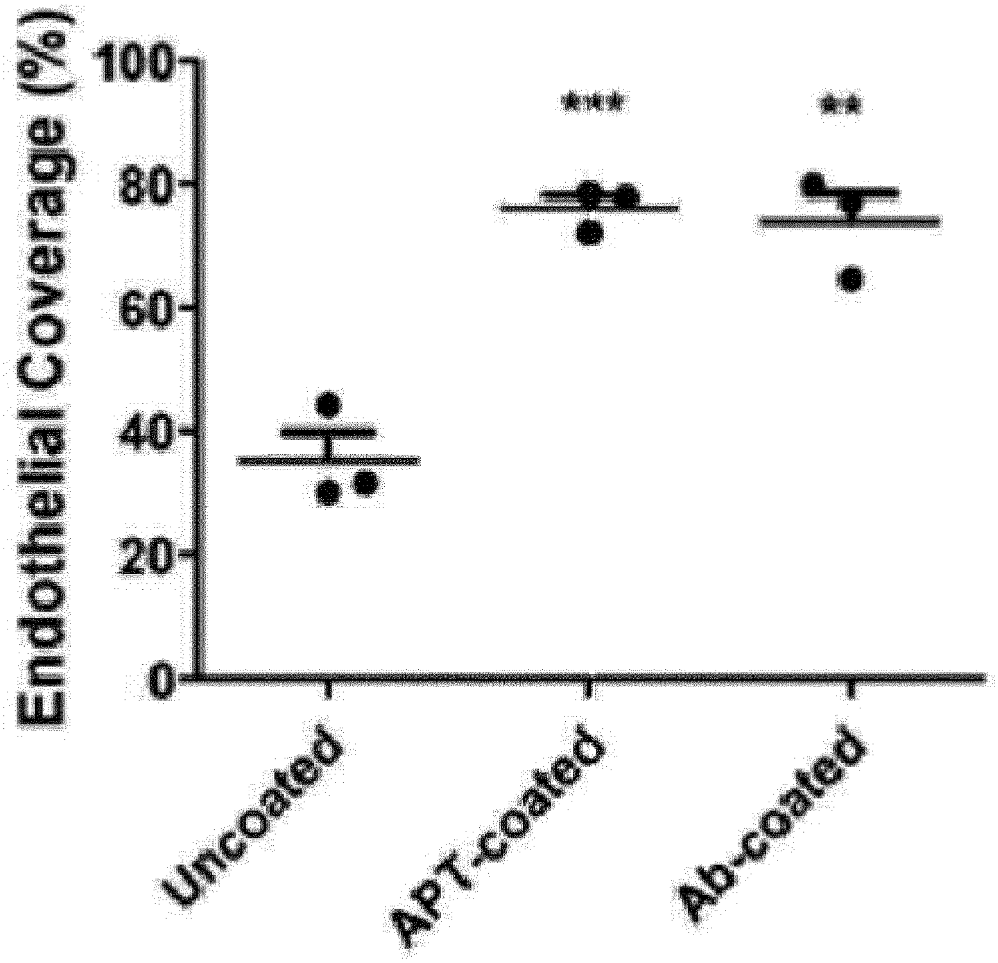
Endothelial Coverage (%)
100
80
60
40
20
0
***
**
Uncoated
APT-coated
Ab-coated

【Fig. 3 d】

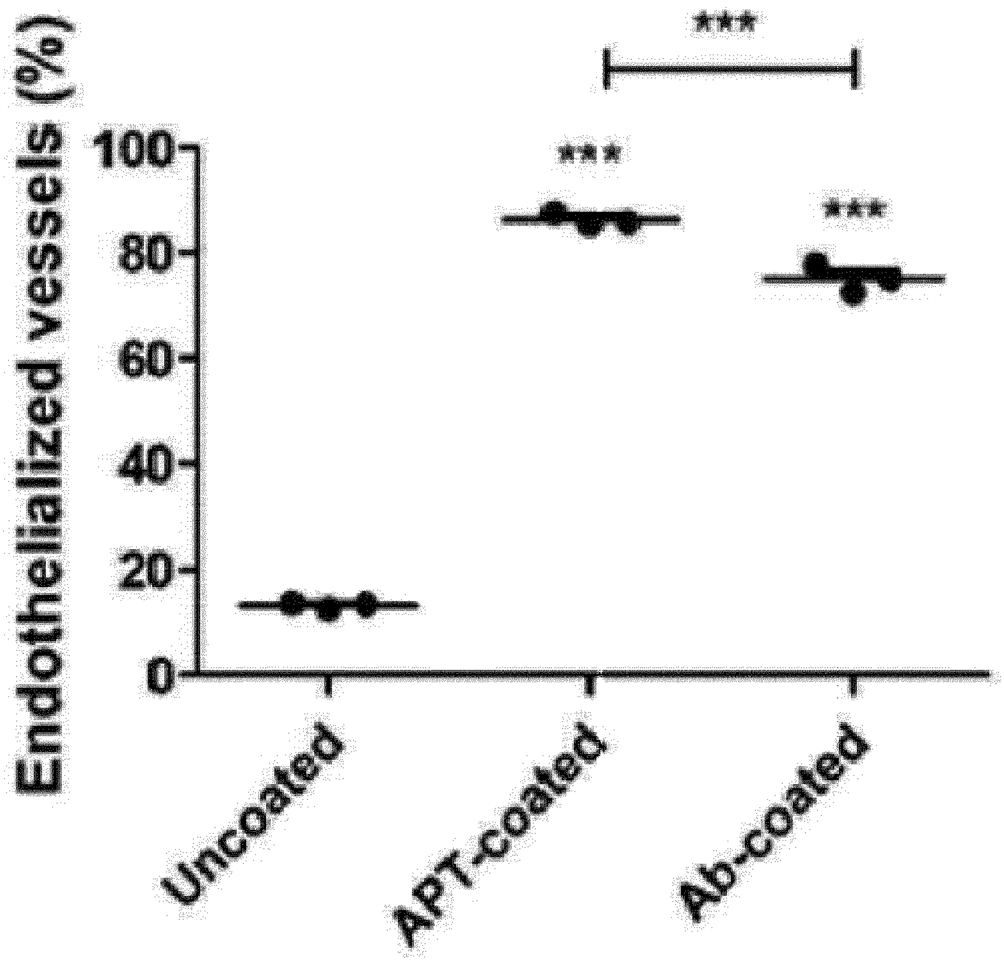
***
***
***
Endothelialized vessels (%)
100
80
60
40
20
0
Uncoated
APT-coated
Ab-coated

【Fig. 3 e】

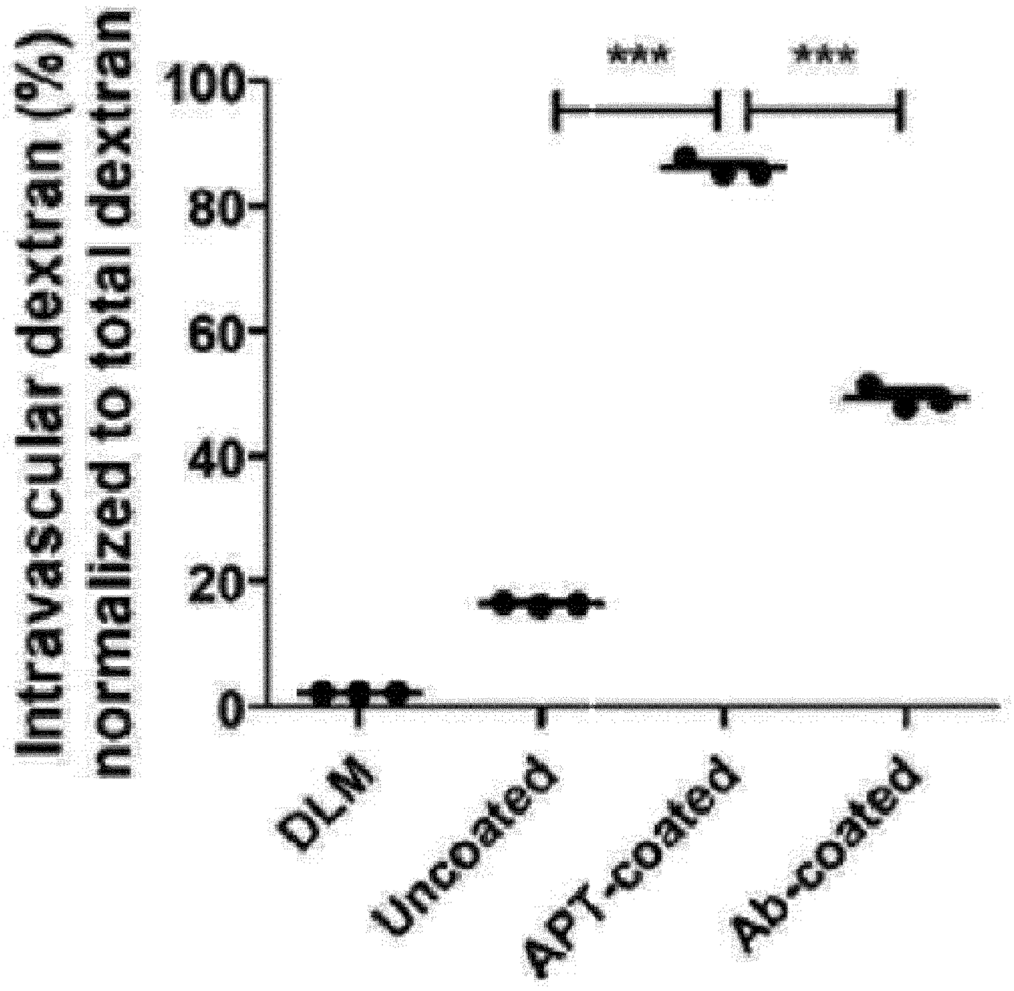
Intravascular dextran (%) normalized to total dextran
100
80
60
40
20
0
***
***
DLM
Uncoated
APT-coated
Ab-coated

【Fig. 3 f】

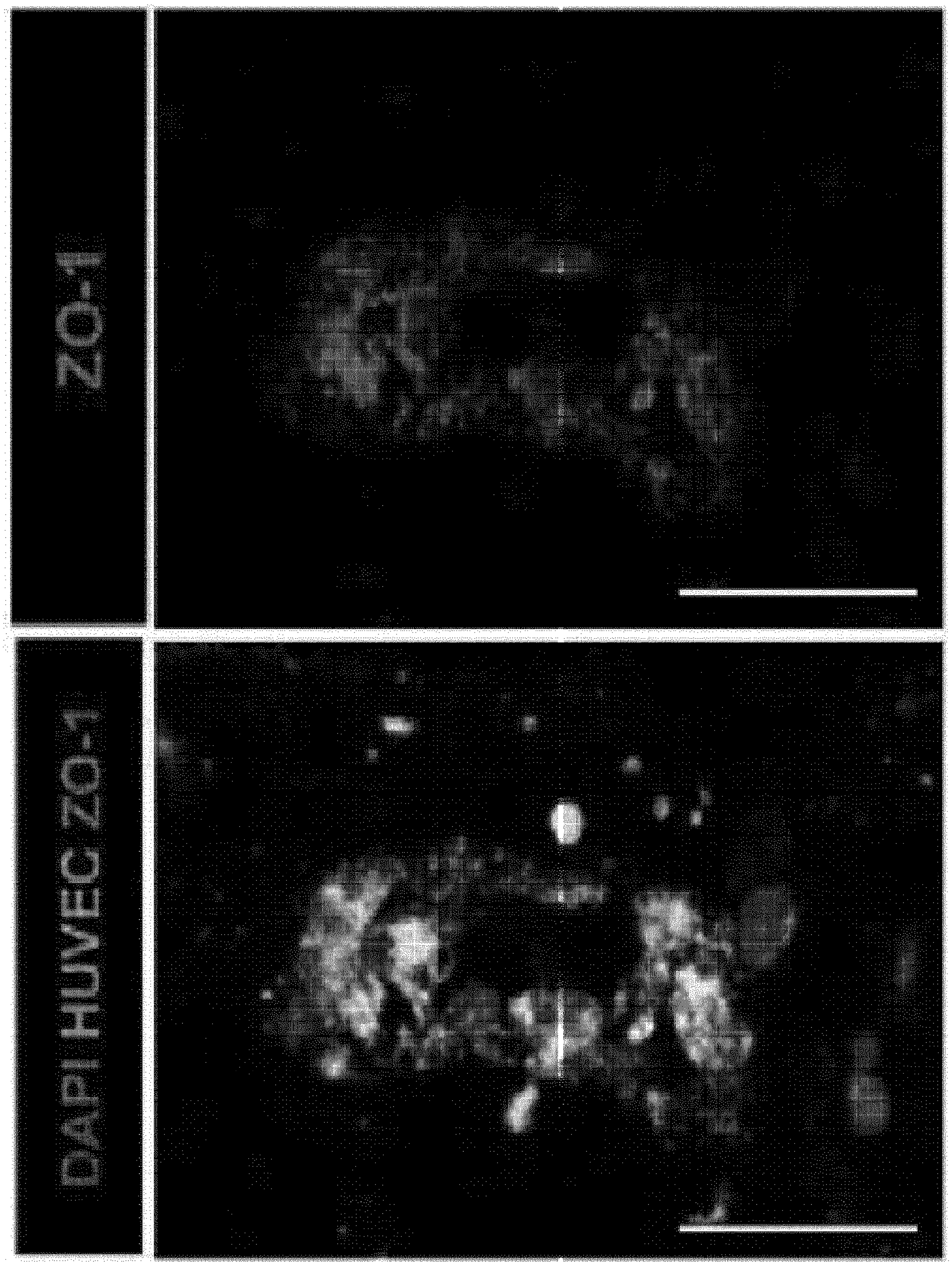

【Fig. 3 g】

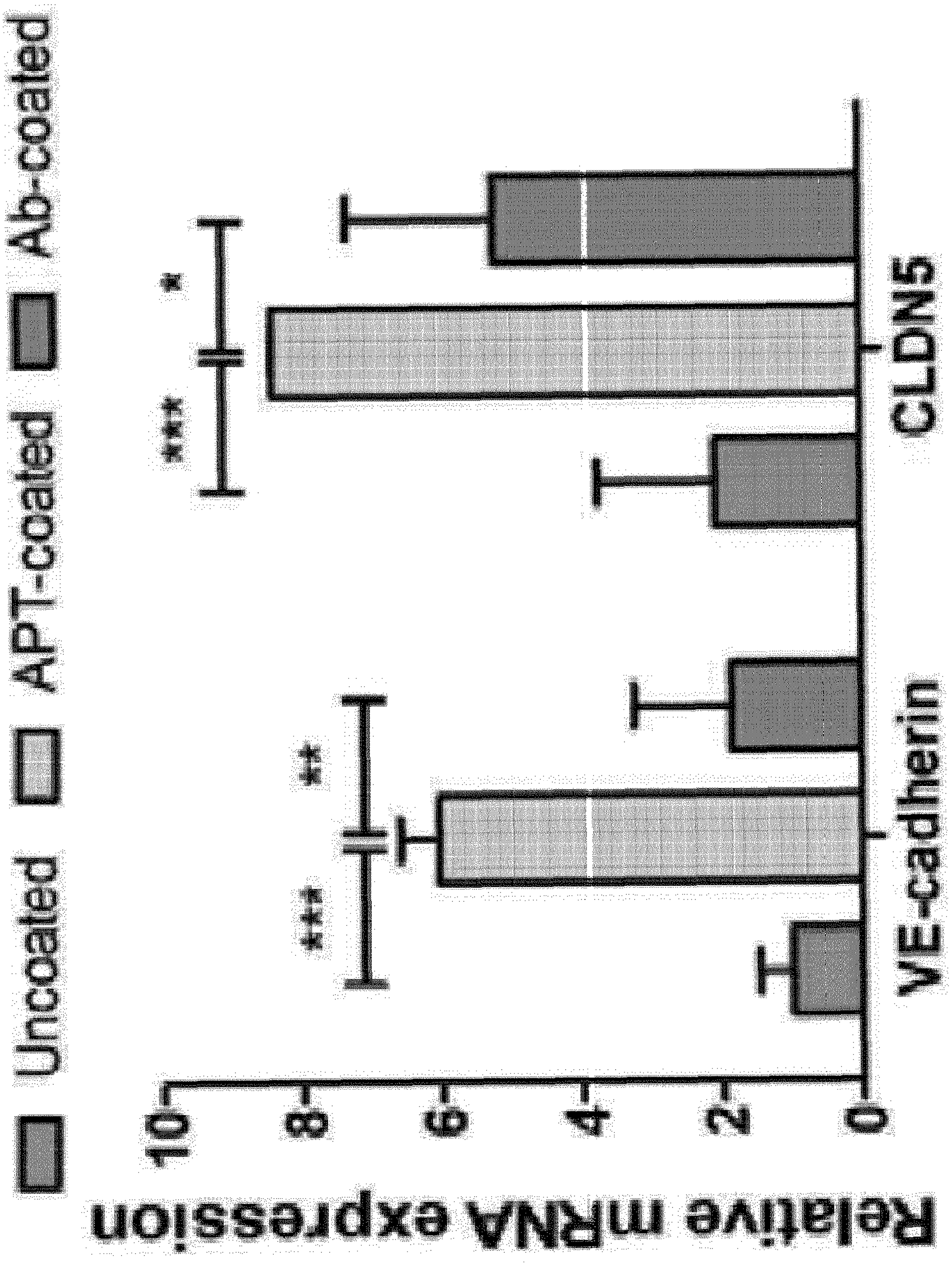
Uncoated
APT-coated
Ab-coated
Relative mRNA expression
10
8
6
4
2
0
***
**
*
VE-cadherin
CLDN5

【Fig. 3 h】

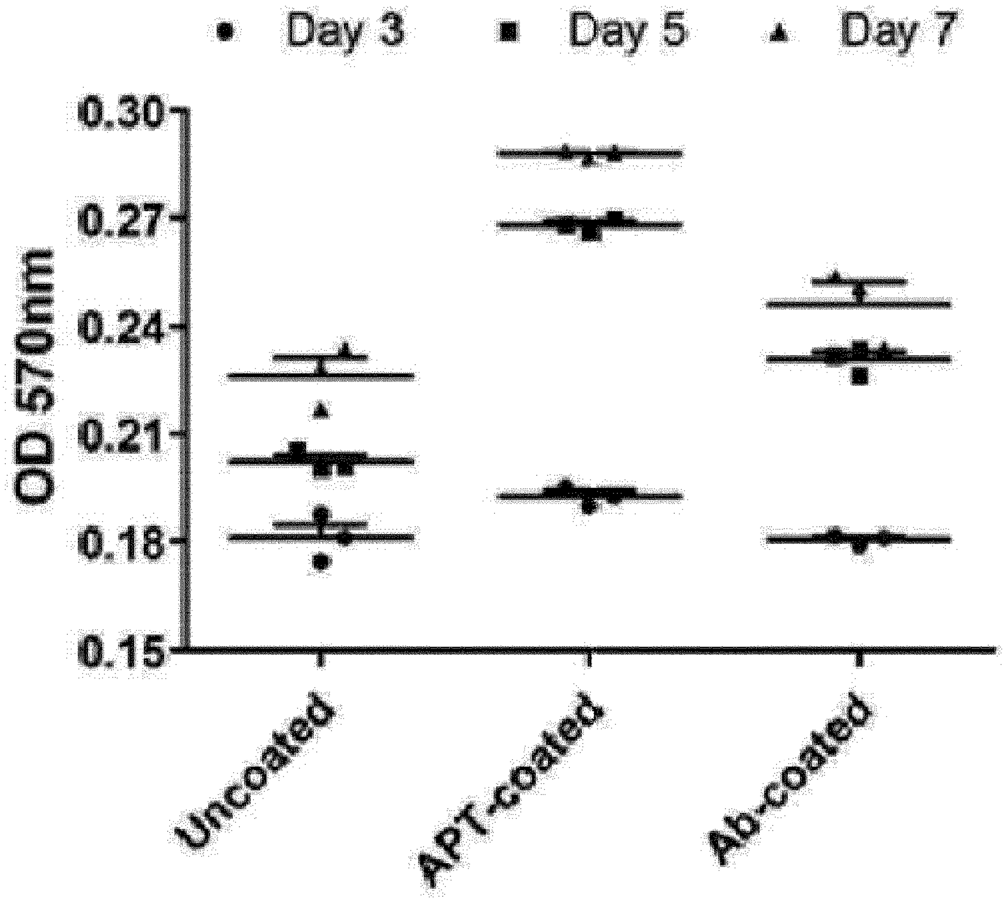
Day 3
Day 5
Day 7
0.30
0.27
0.24
0.21
0.18
0.15
OD 570nm
Uncoated
APT-coated
Ab-coated

【Fig. 3 i】

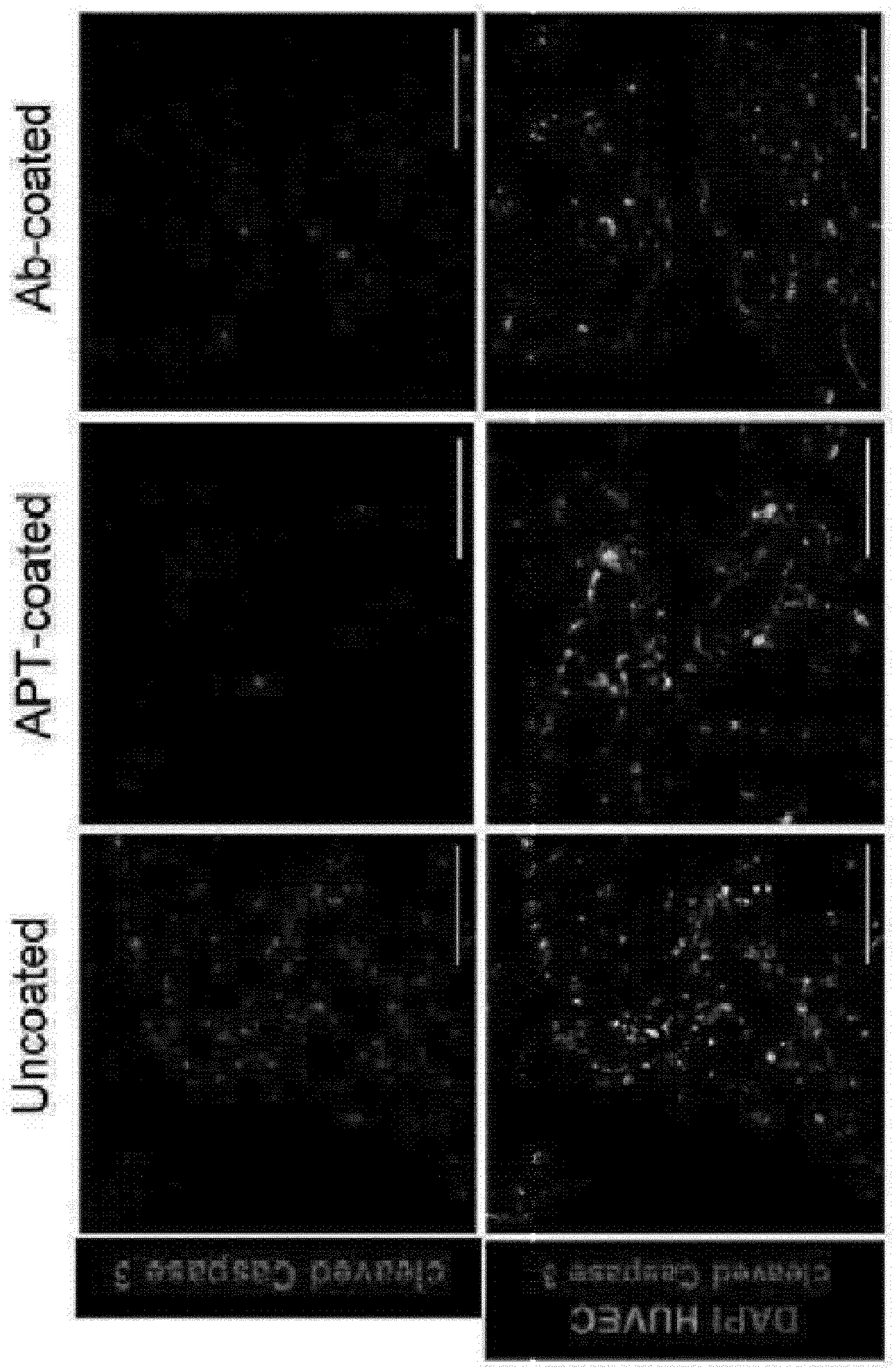
Ab-coated
APT-coated
Uncoated
cleaved Caspase 3
DAPI HUVEC
cleaved Caspase 3

【Fig. 3 j】

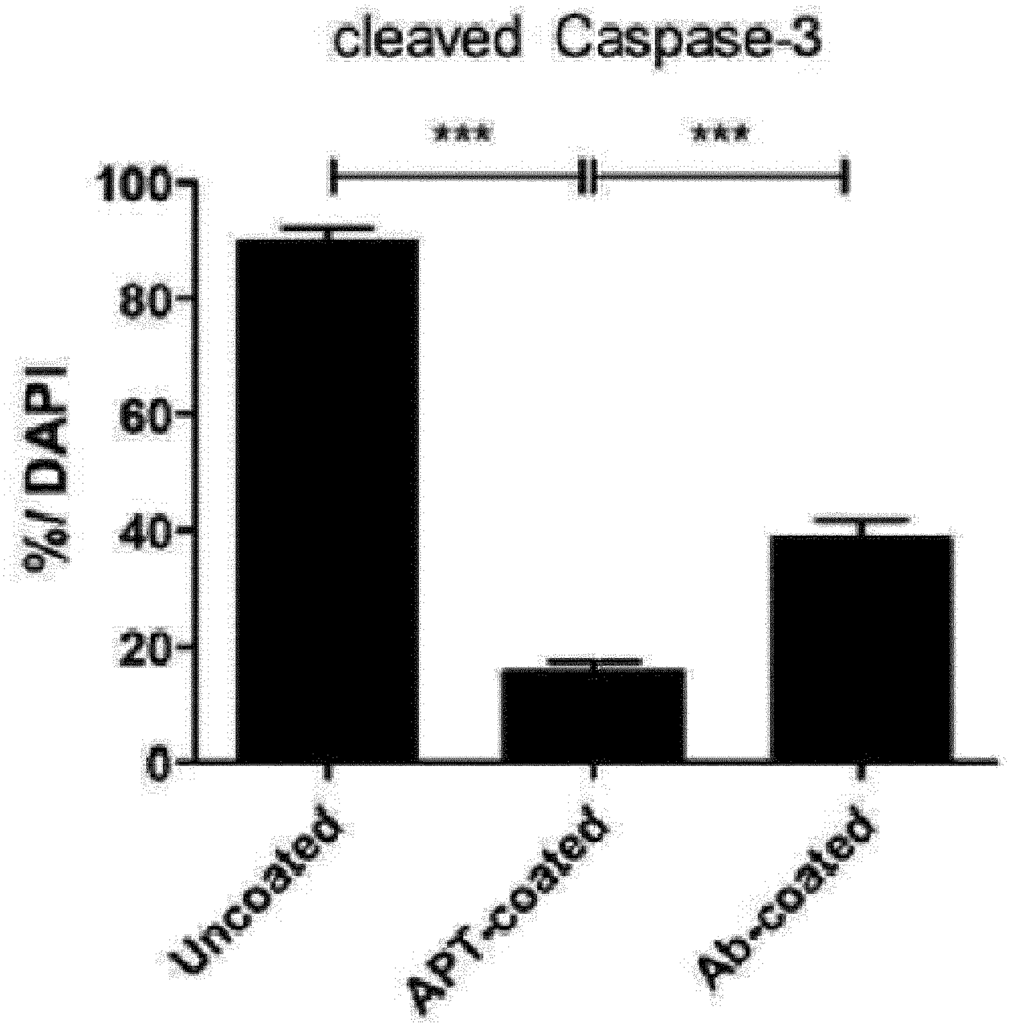
cleaved Caspase-3
***
***
100
80
60
40
20
0
%/ DAPI
Uncoated
APT-coated
Ab-coated

【Fig. 3 k】

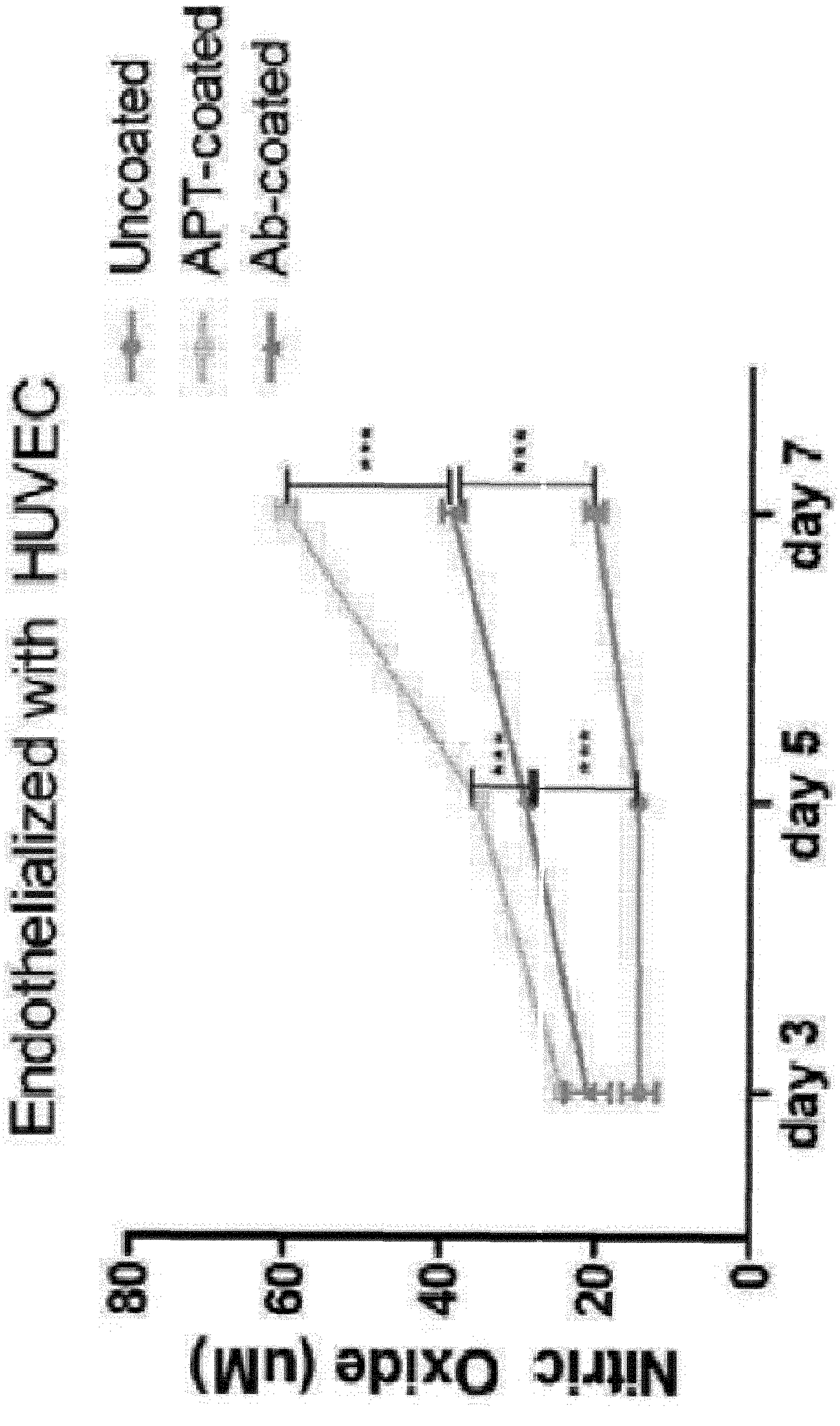
Endothelialized with HUVEC
Uncoated
APT-coated
Ab-coated
Nitric Oxide (uM)
80
60
40
20
0
day 3
day 5
day 7
***
***
***
***

【Fig. 3 l】

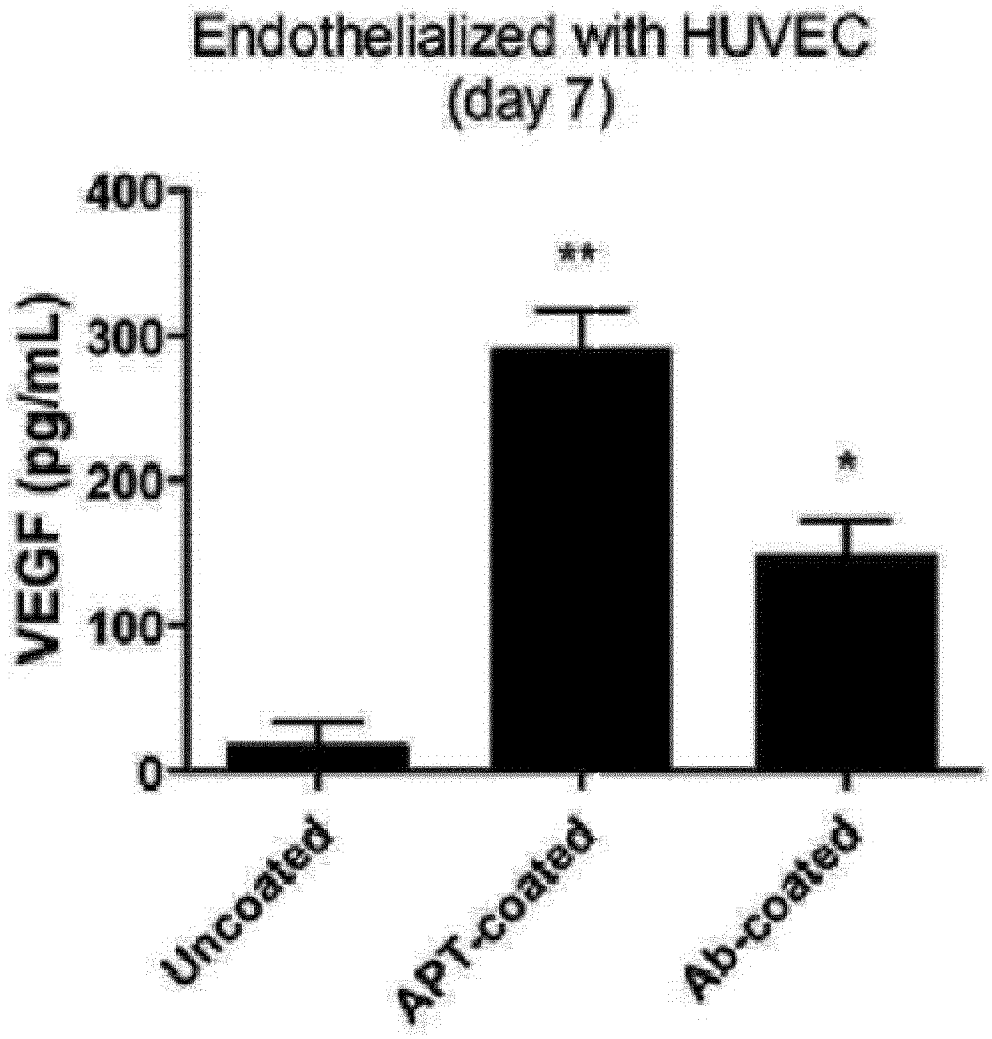
Endothelialized with HUVEC
(day 7)
VEGF (pg/mL)
400
300
200
100
0
**
*
Uncoated
APT-coated
Ab-coated

【Fig. 4 a】

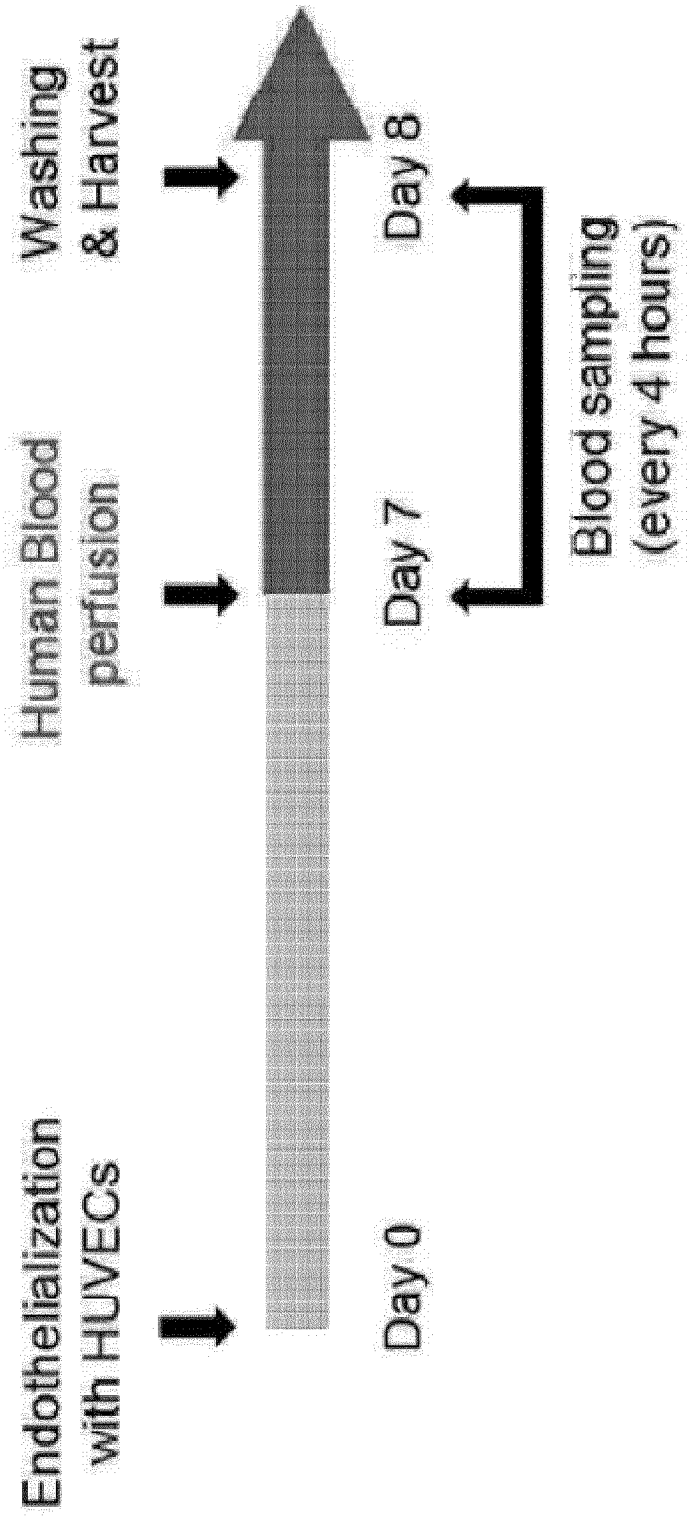
Endothelialization
with HUVECs
Human Blood
perfusion
Washing
& Harvest
Day 0
Day 7
Day 8
Blood sampling
(every 4 hours)

【Fig. 4 b】

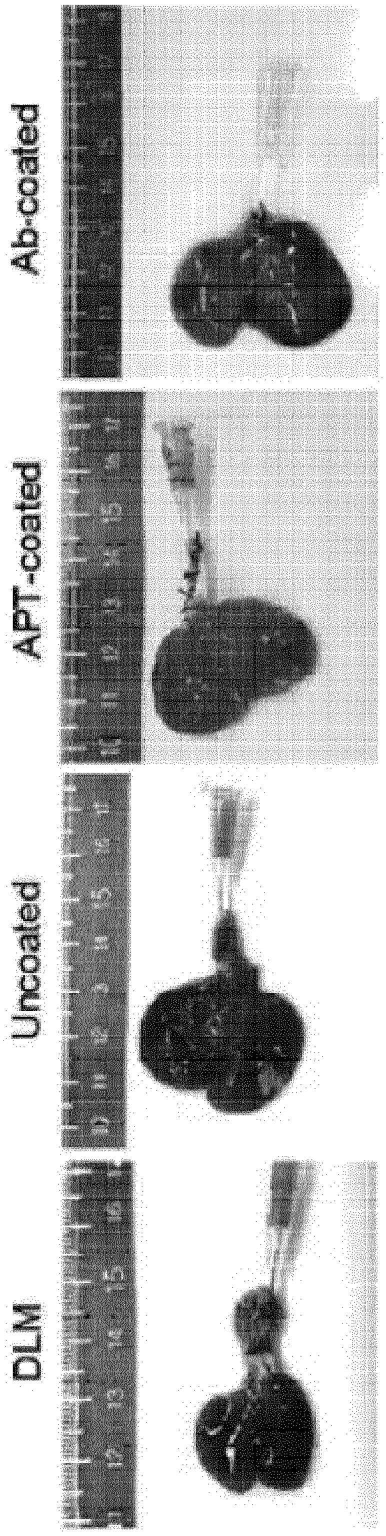

【Fig. 4 c】

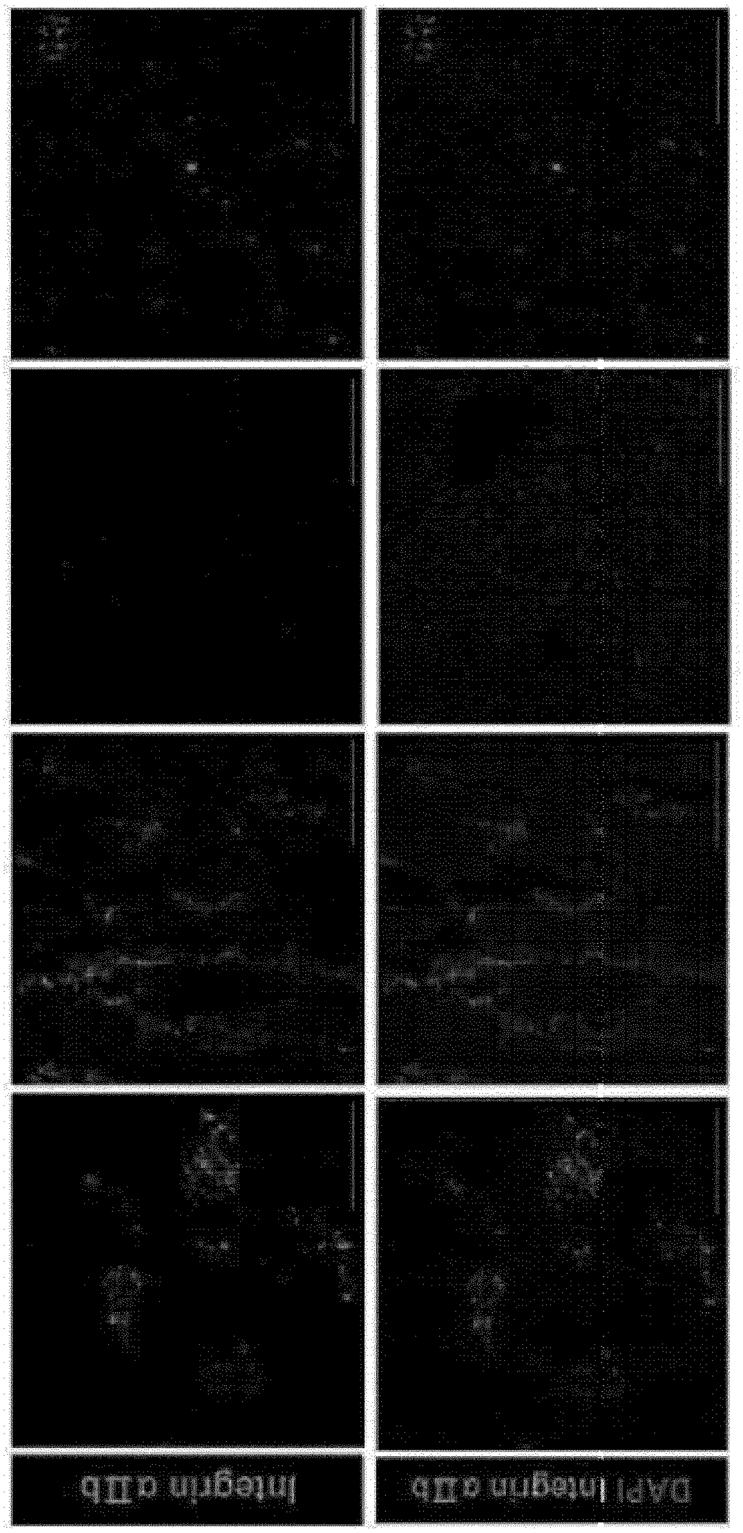

【Fig. 4 d】

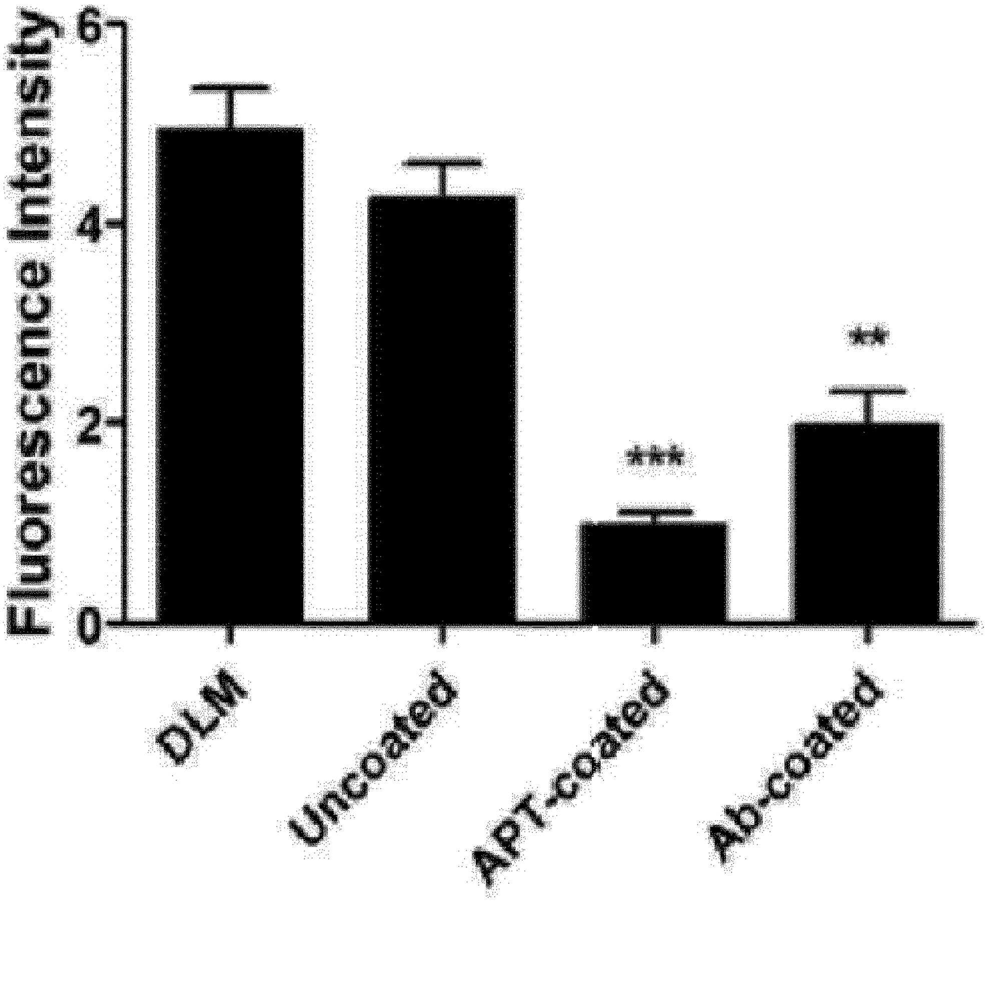
Fluorescence Intensity
6
4
2
0
***
**
DLM
Uncoated
APT-coated
Ab-coated

【Fig. 4 e】

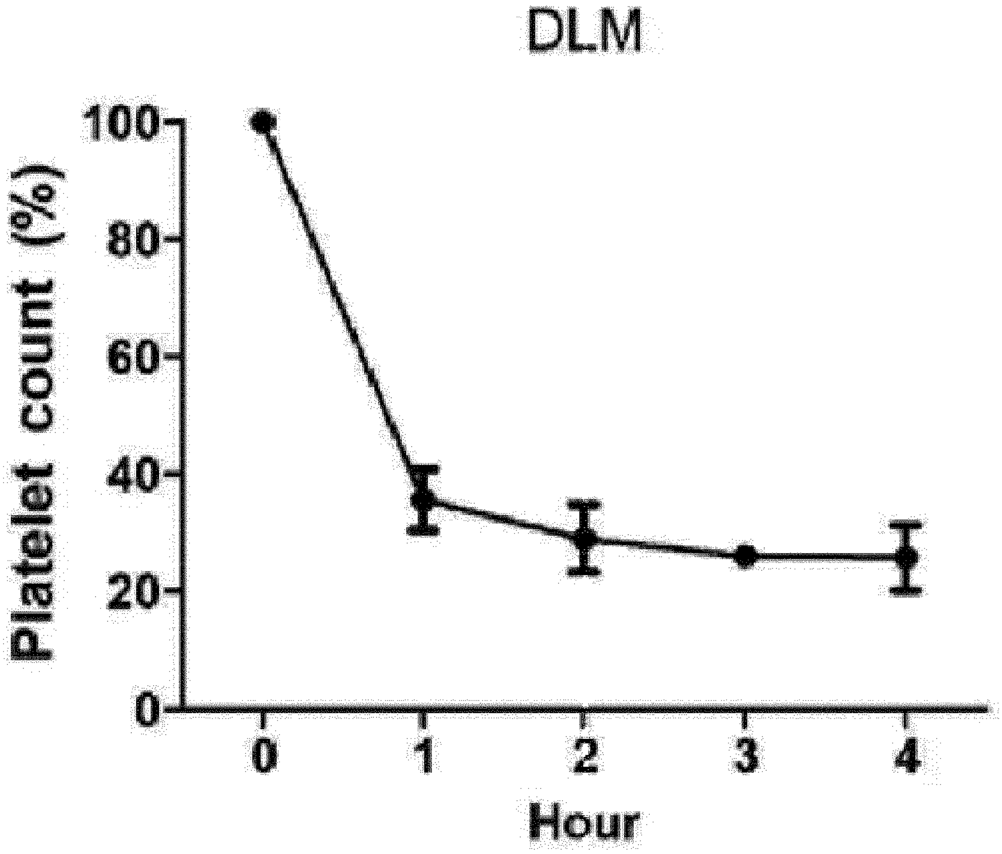

【Fig. 4 f】

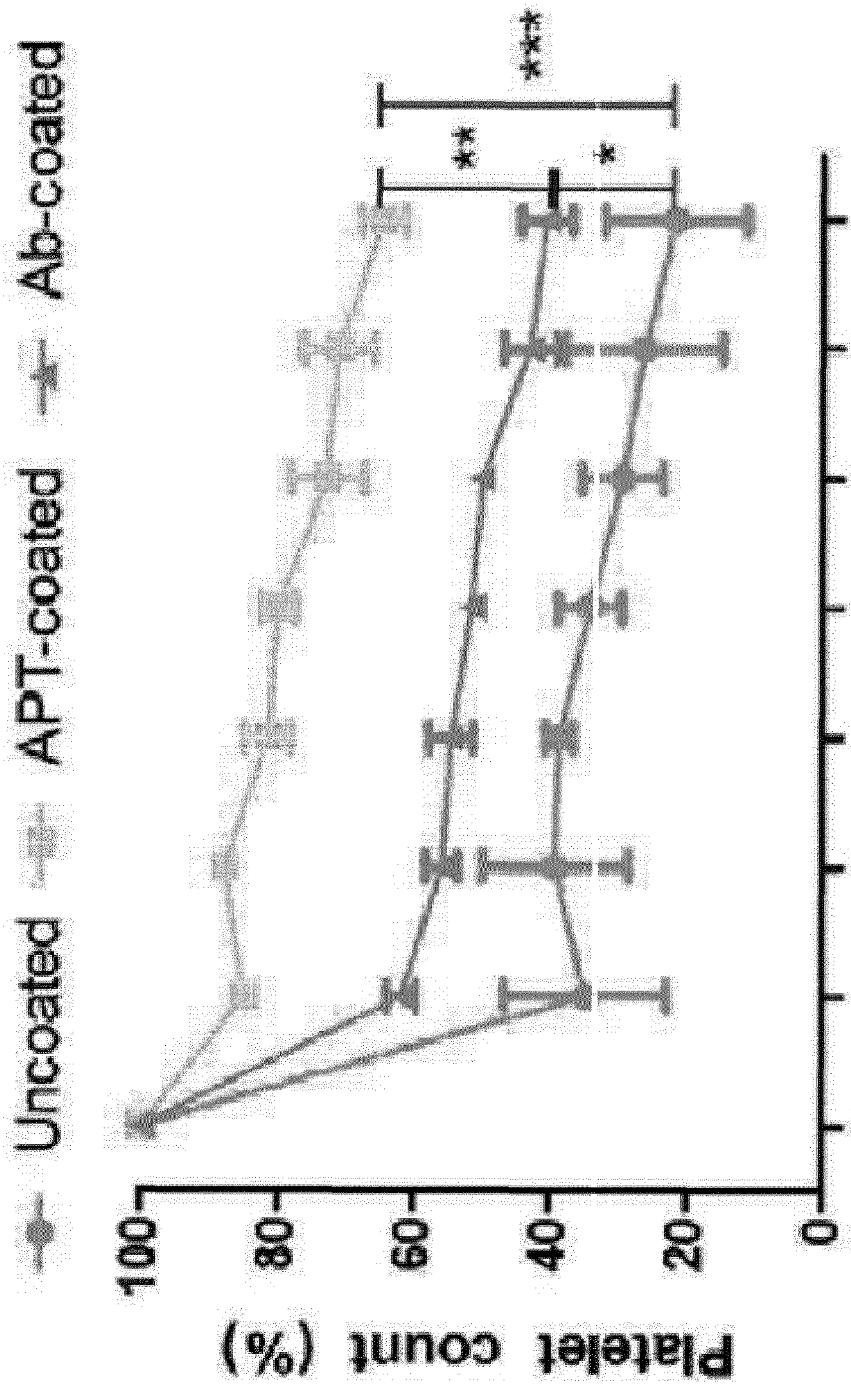

Uncoated
APT-coated
Ab-coated
Platelet count (%)
100
80
60
40
20
0
0
2
4
8
12
16
20
24
Hour
**
***
*

【Fig. 4 g】

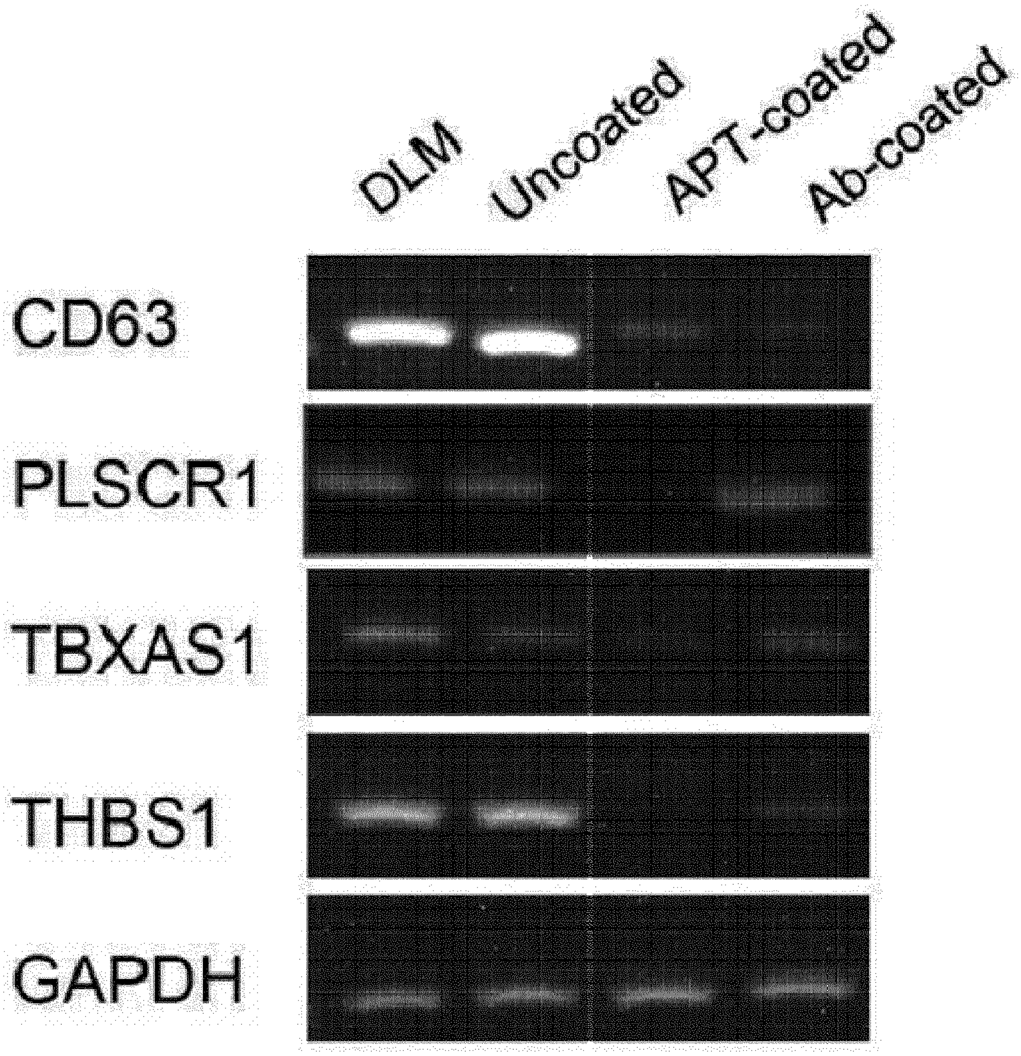
DLM
Uncoated
APT-coated
Ab-coated
CD63
PLSCR1
TBXAS1
THBS1
GAPDH

【Fig. 5 a】

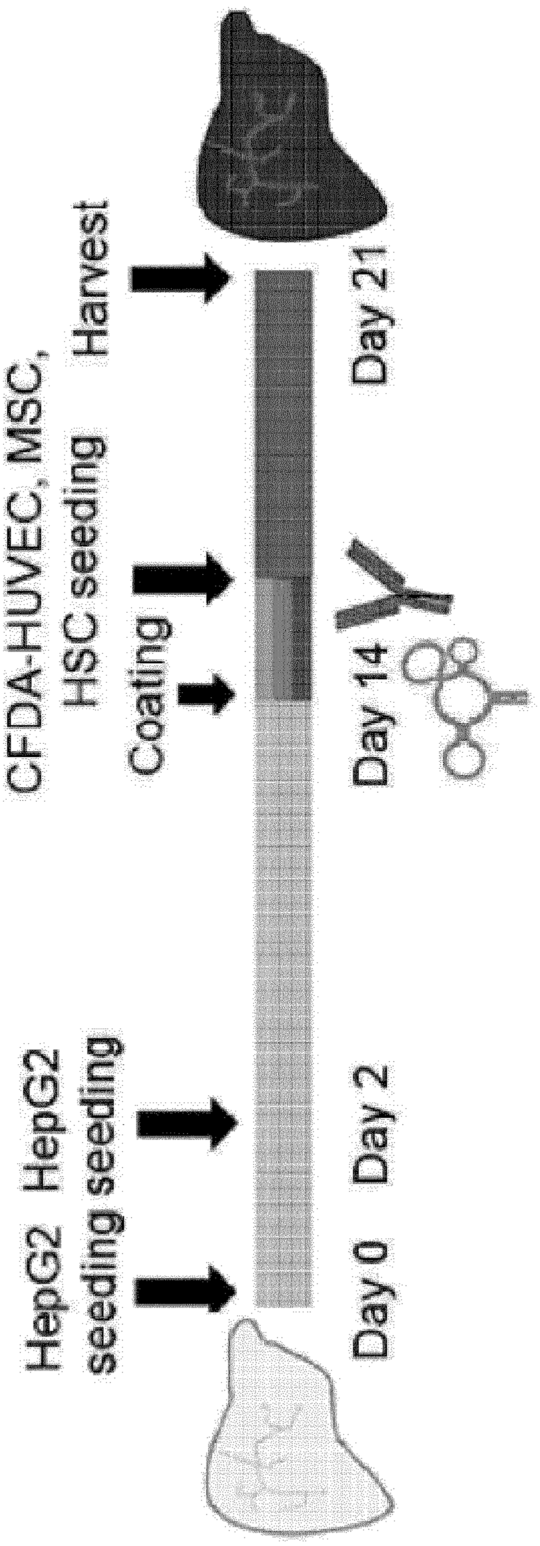
HepG2 seeding
HepG2 seeding
Coating
CFDA-HUVEC, MSC, HSC seeding
Harvest
Day 0
Day 2
Day 14
Day 21

【Fig. 5 b】

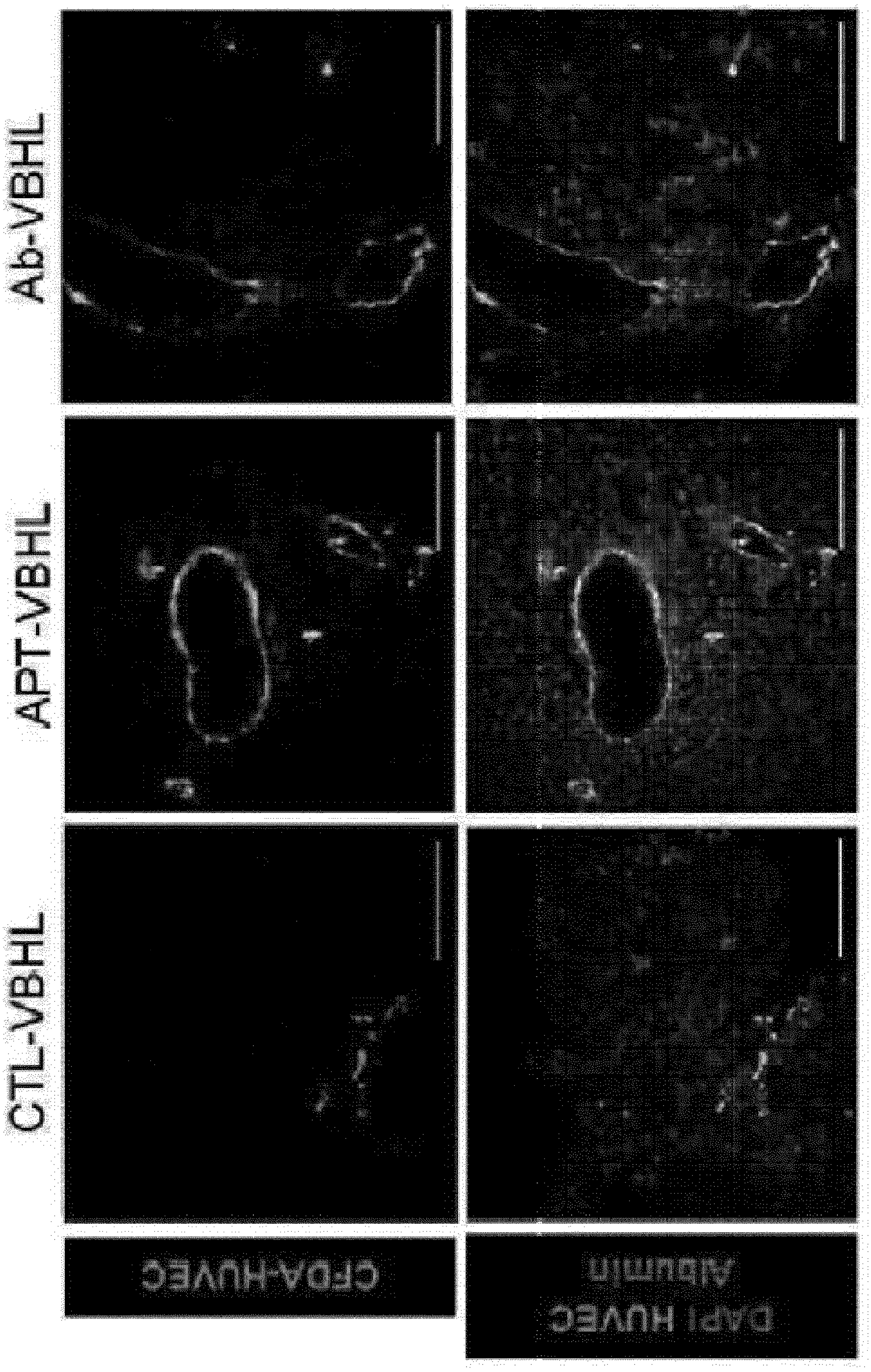

【Fig. 5 c】

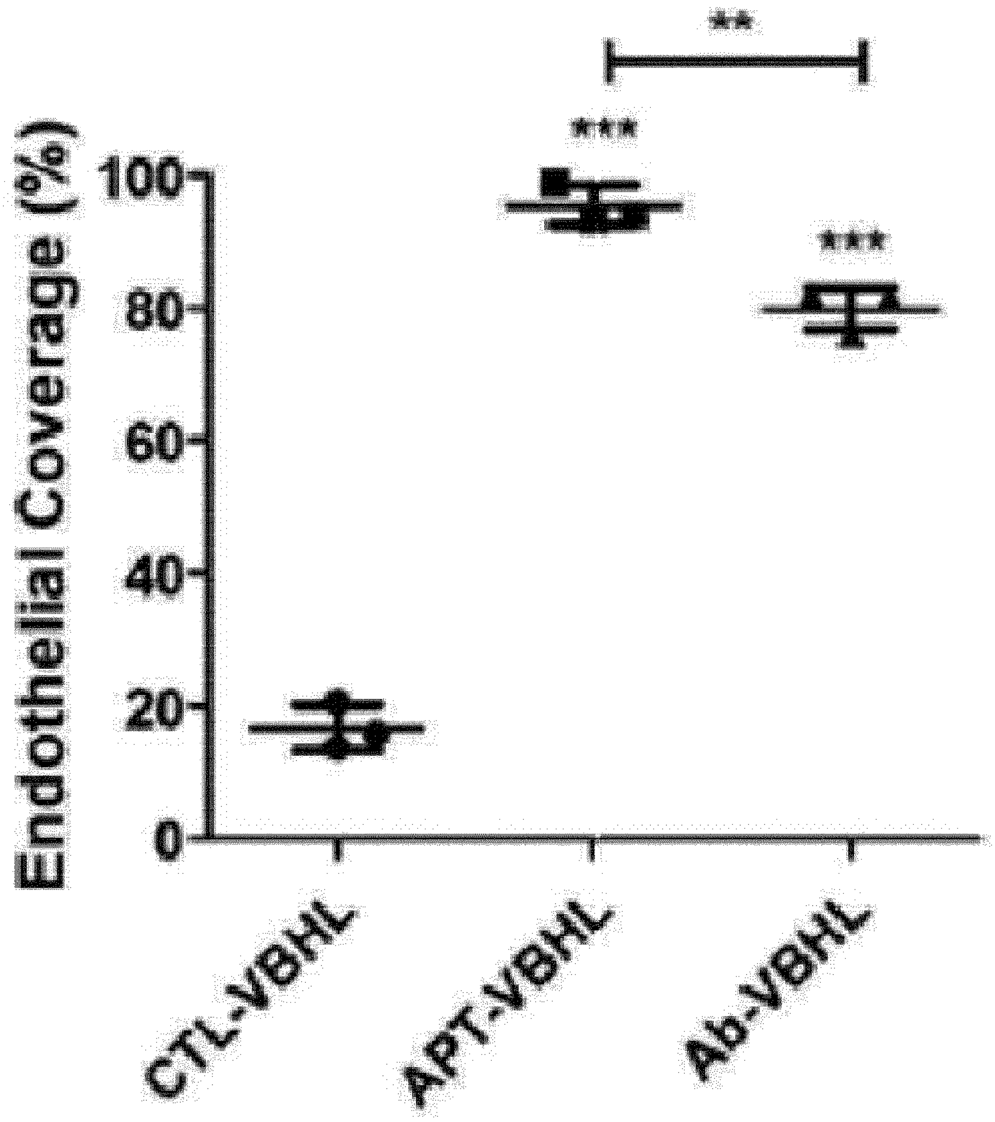
**
***
***
Endothelial Coverage (%)
100
80
60
40
20
0
CTL-VBHL
APT-VBHL
Ab-VBHL

【Fig. 5 d】

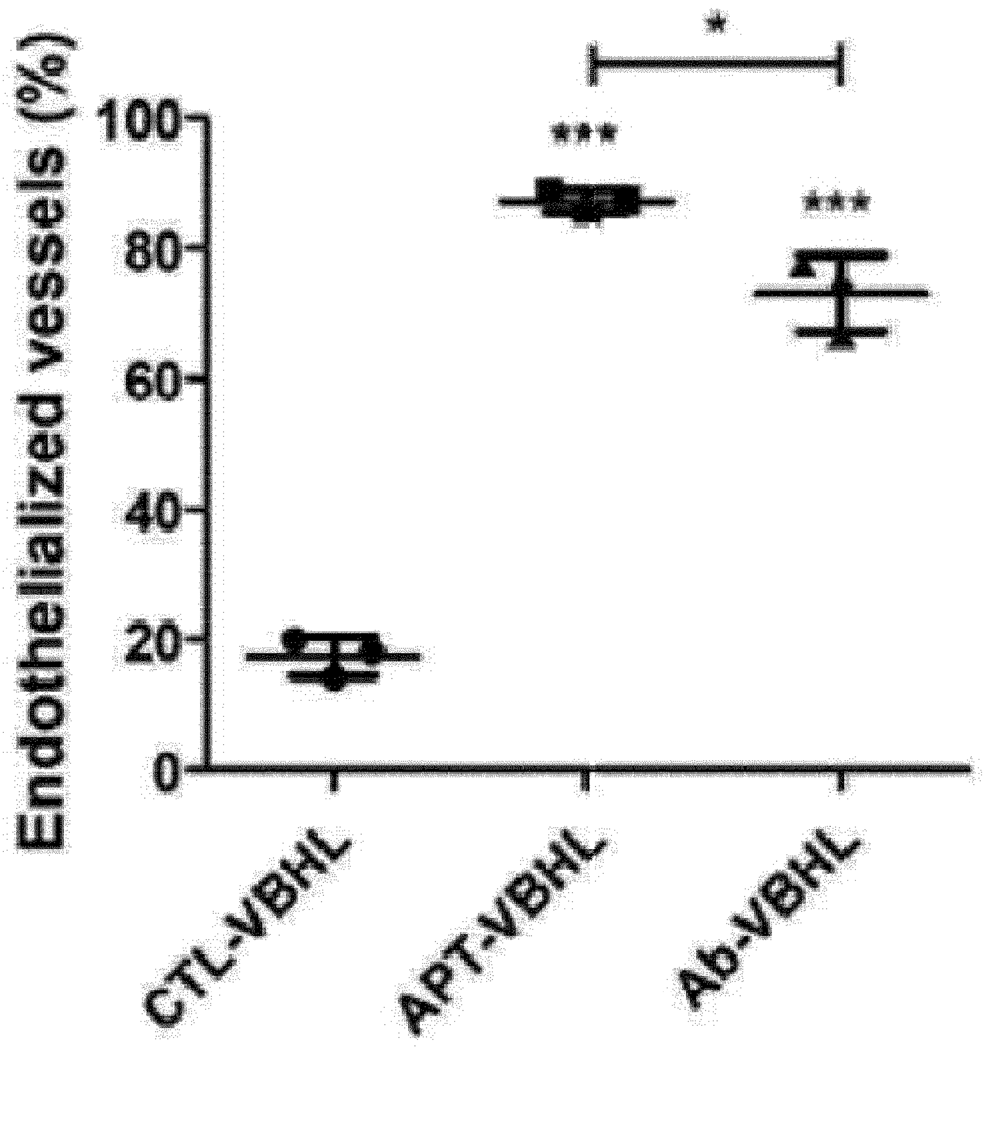
Endothelialized vessels (%)
100
80
60
40
20
0
***
***
*
CTL-VBHL
APT-VBHL
Ab-VBHL

【Fig. 5 e】

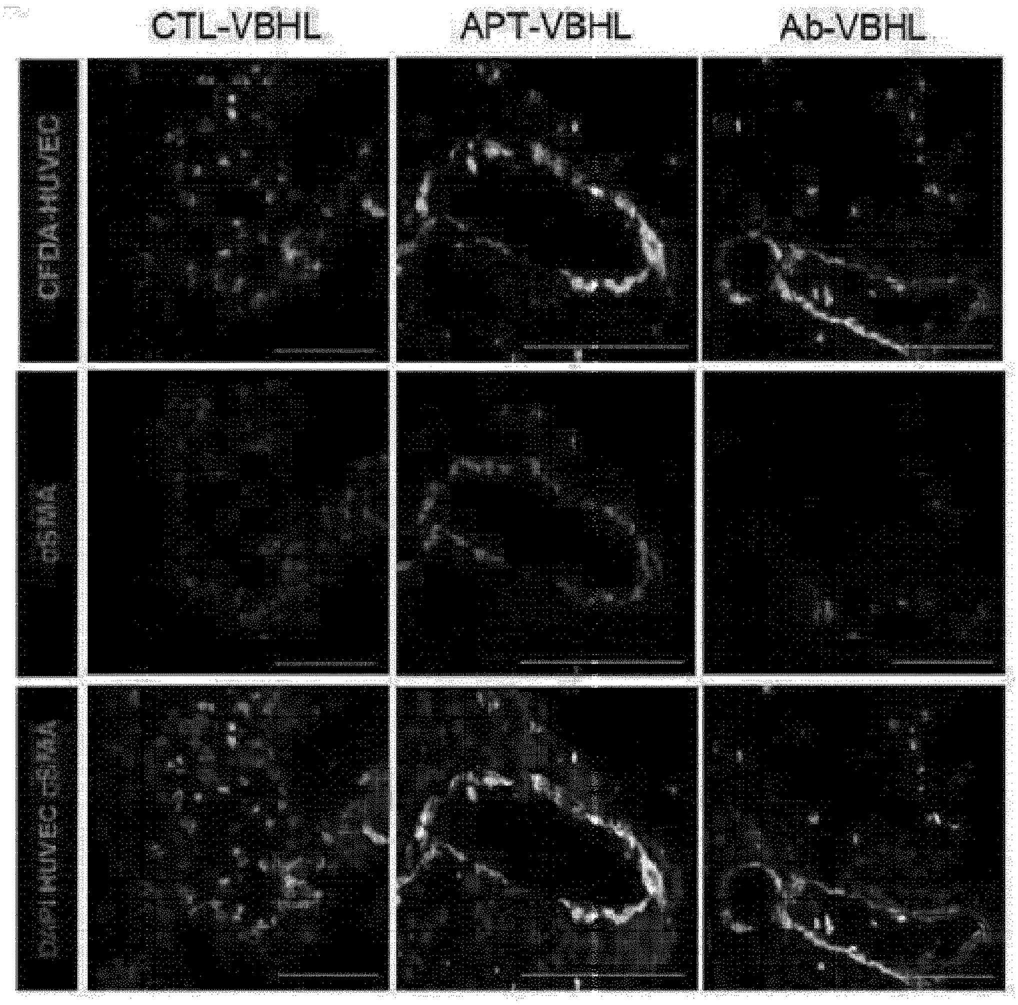

【Fig. 5 f】

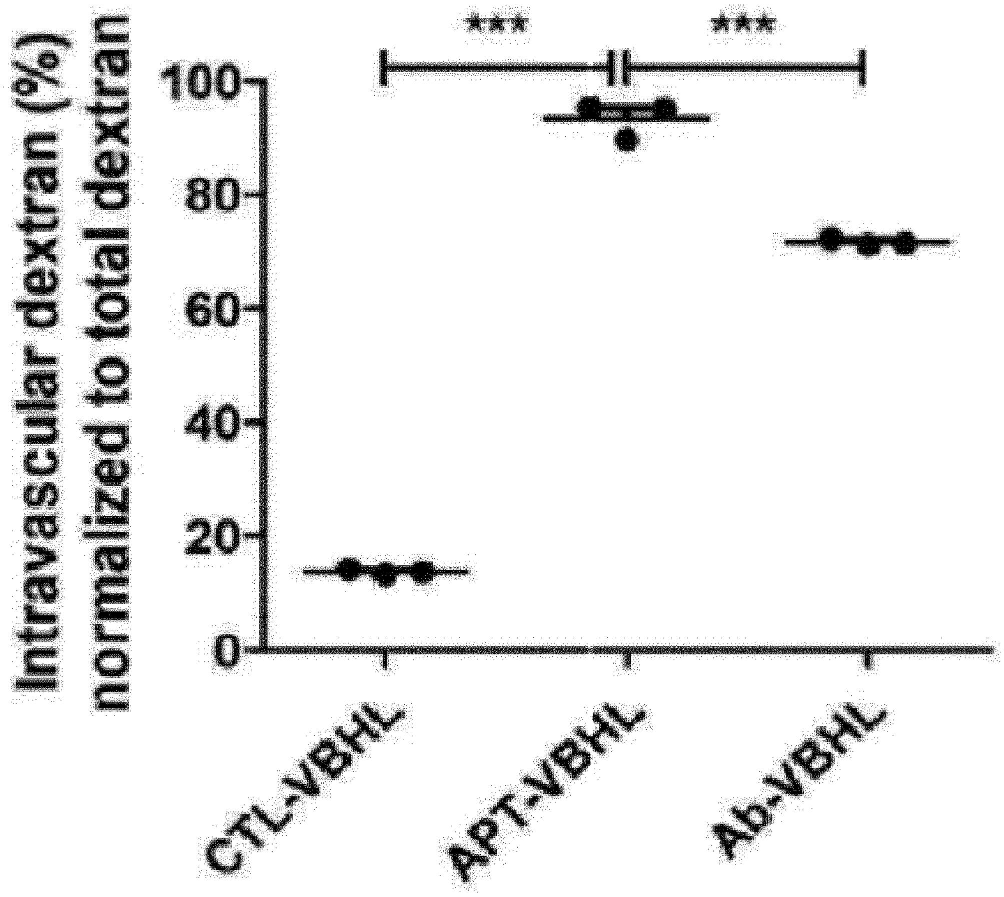
Intravascular dextran (%) normalized to total dextran
***
***
100
80
60
40
20
0
CTL-VBHL
APT-VBHL
Ab-VBHL

【Fig. 5 g】

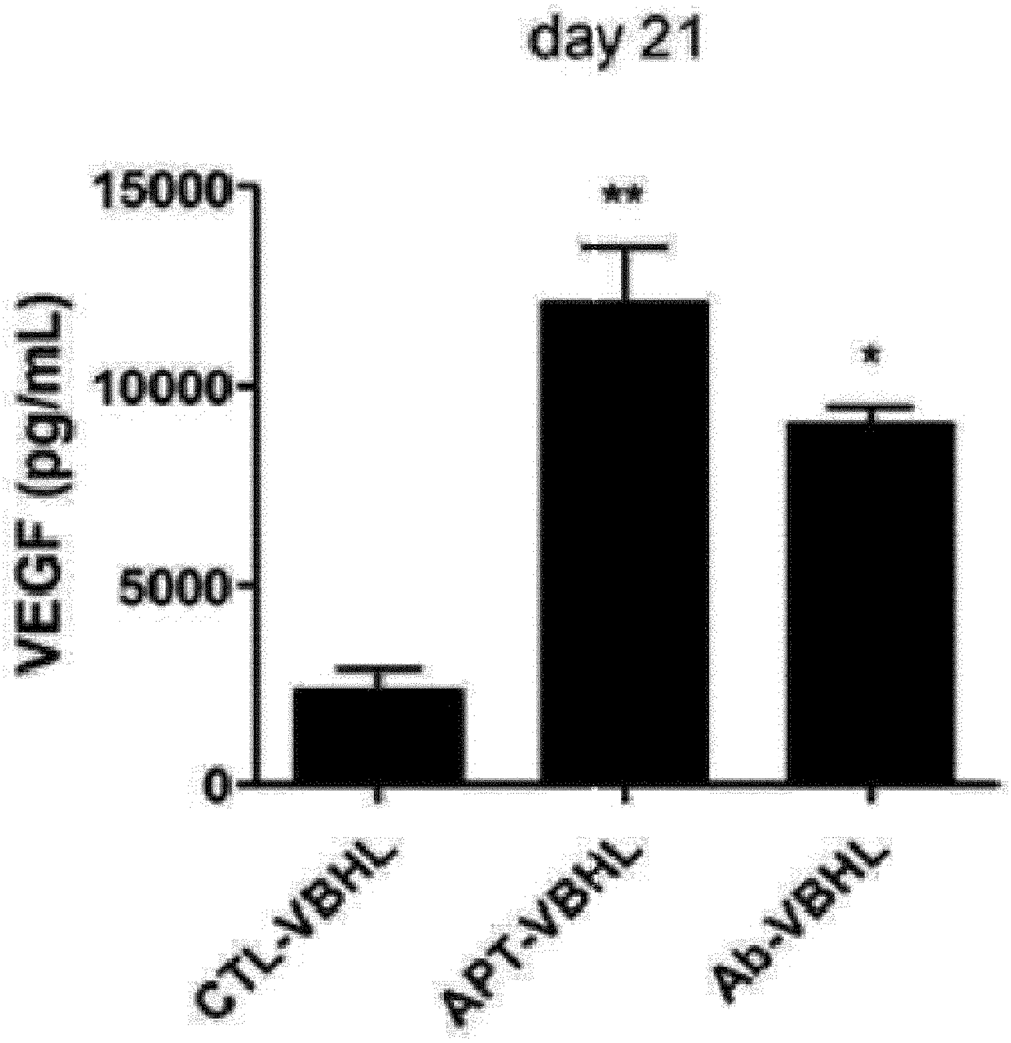

【Fig. 5 h】

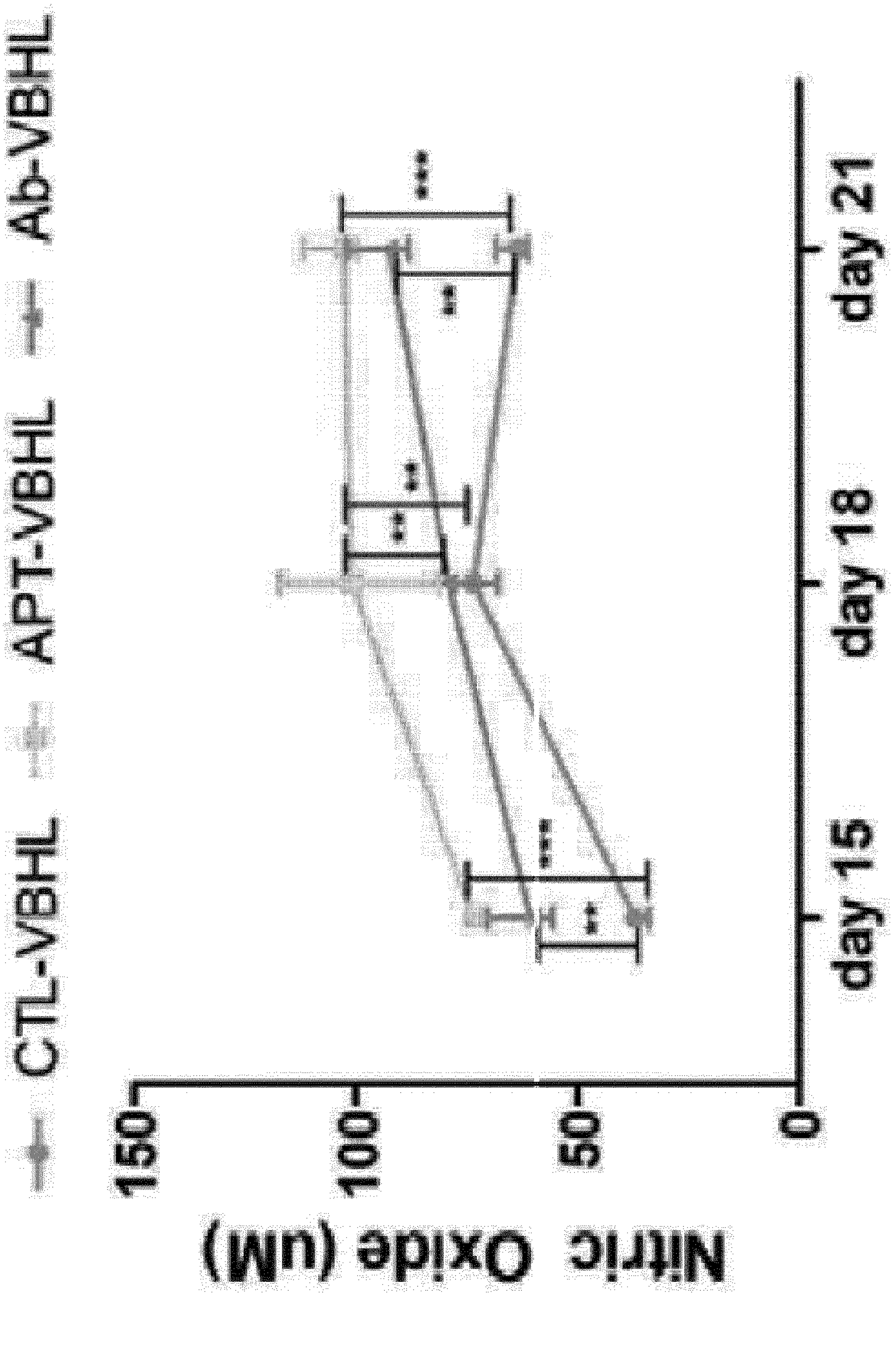
CTL-VBHL
APT-VBHL
Ab-VBHL
Nitric Oxide (uM)
150
100
50
0
day 15
day 18
day 21
**
***
**
**
**
***

【Fig. 5 i】

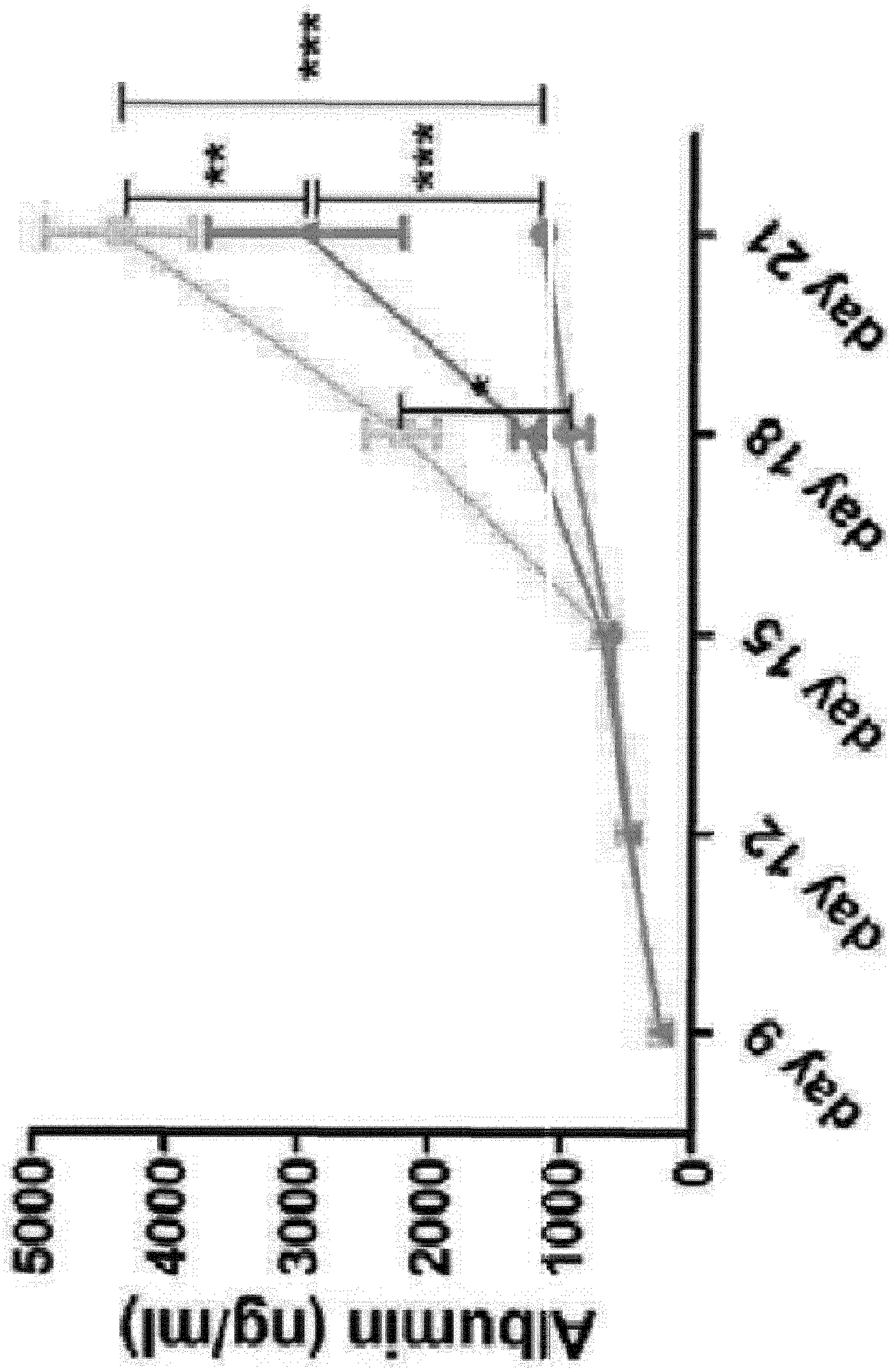
Albumin (ng/ml)
5000
4000
3000
2000
1000
0
day 9
day 12
day 15
day 18
day 21
*
**
***
***

【Fig. 5 j】

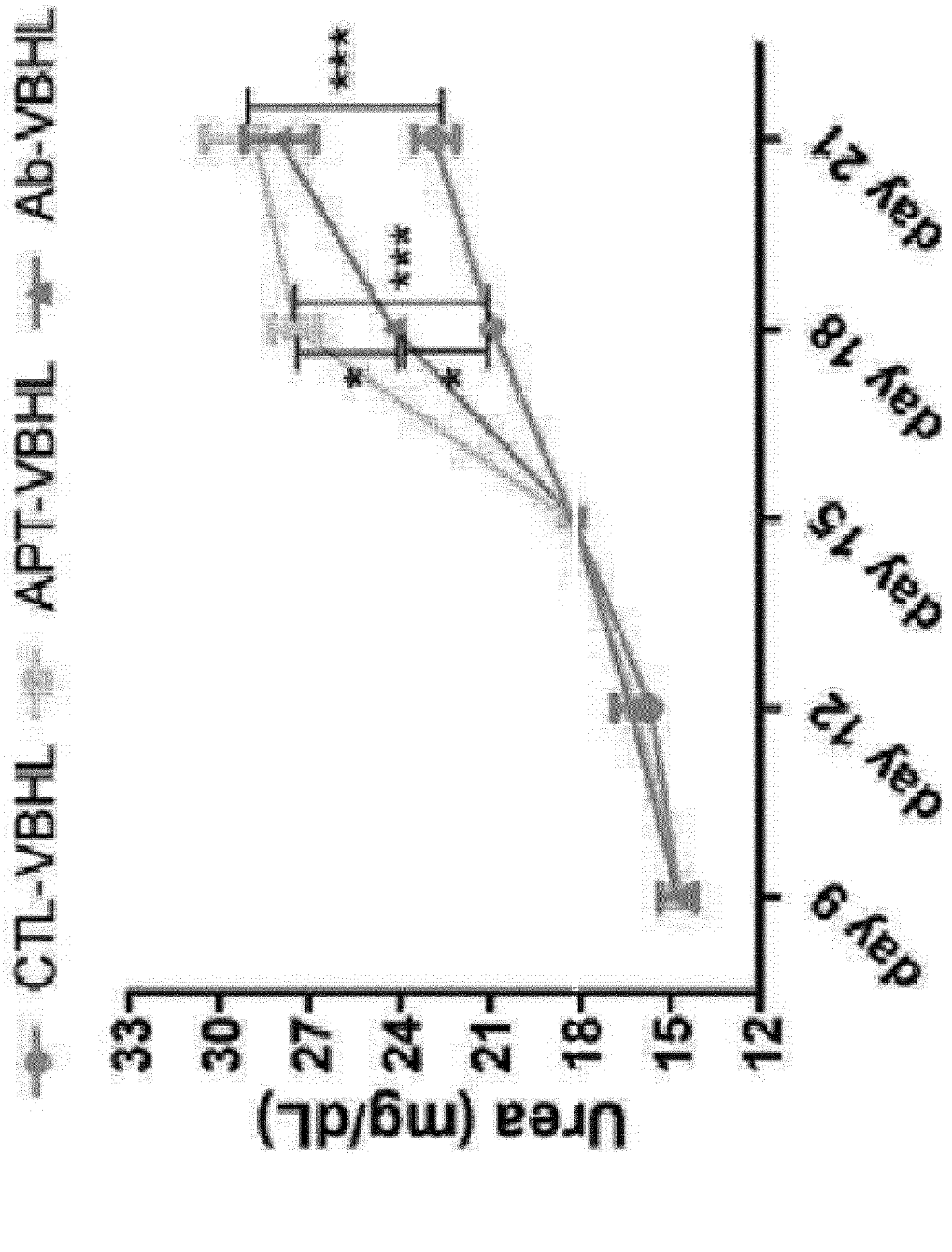
CTL-VBHL
APT-VBHL
Ab-VBHL
Urea (mg/dL)
33
30
27
24
21
18
15
12
day 9
day 12
day 15
day 18
day 21
*
*
***
***

【Fig. 5 k】

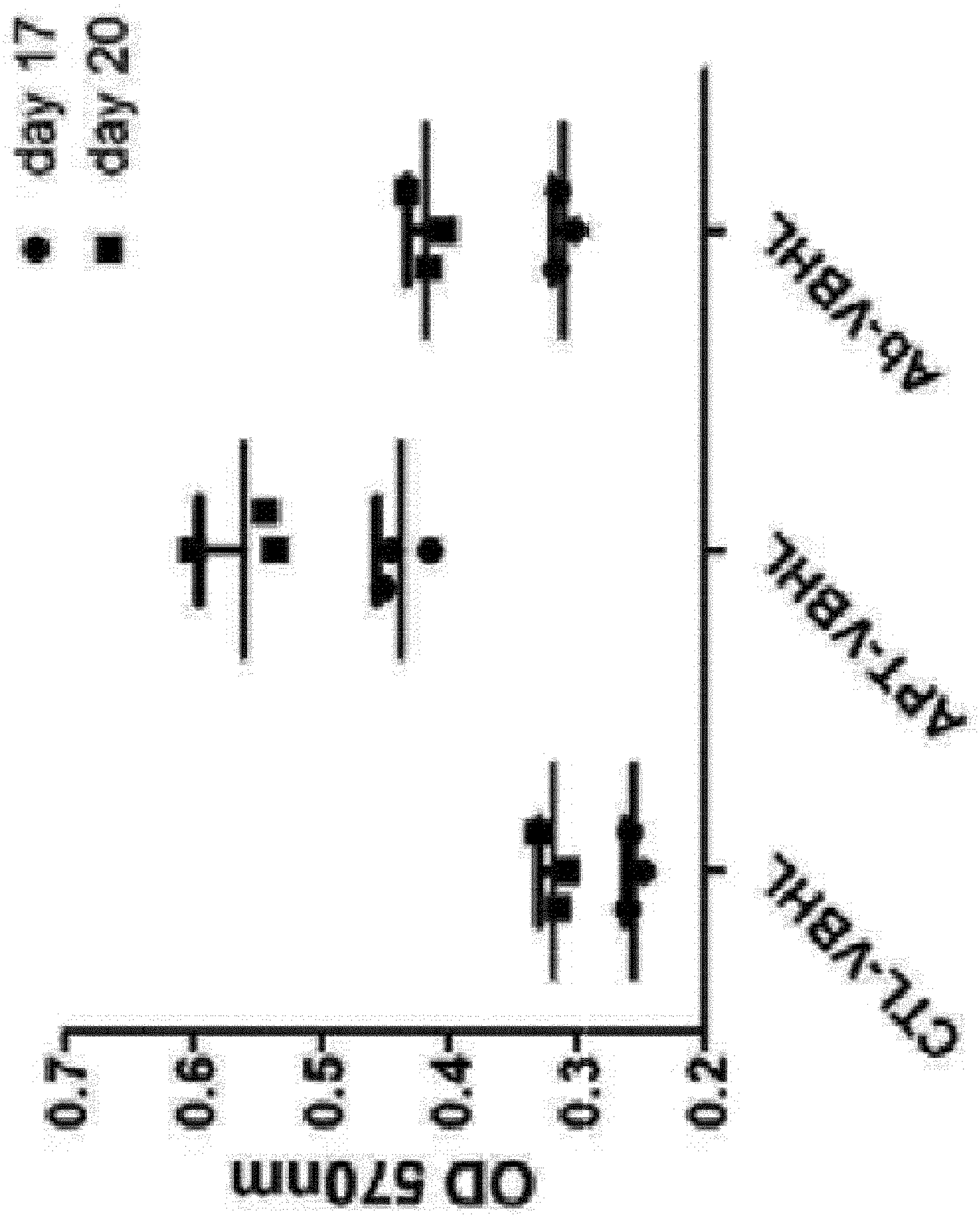

【Fig. 5 Ｉ】

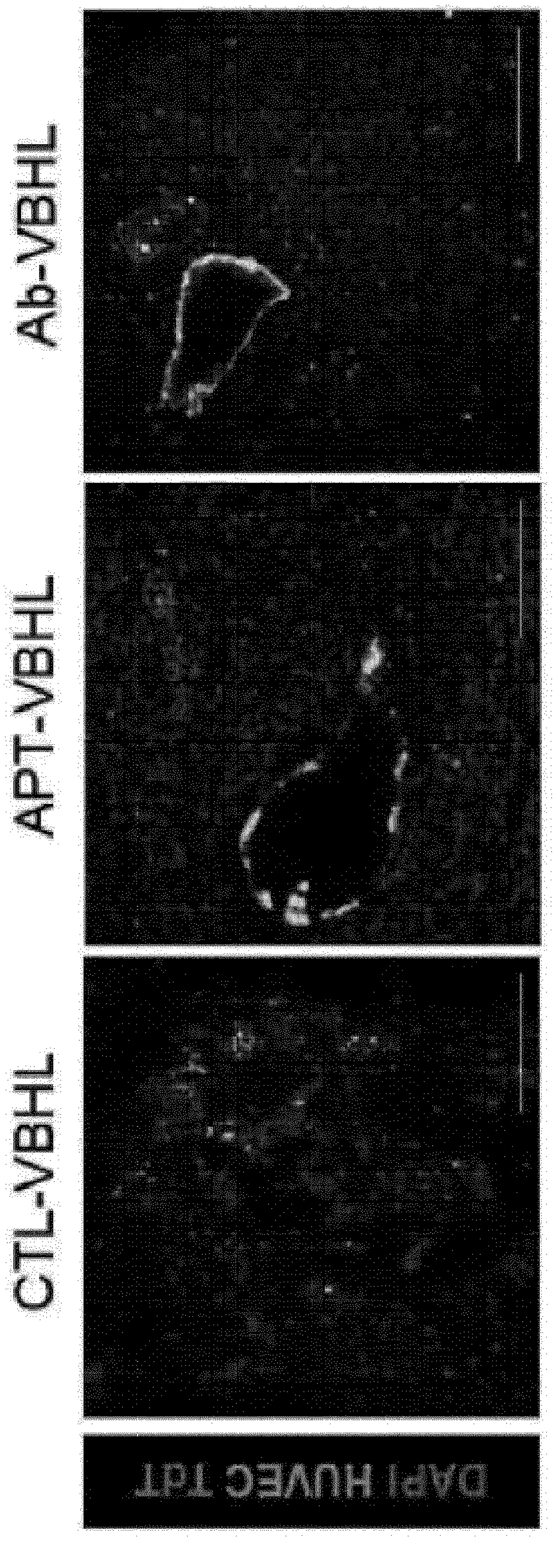

【Fig. 5 m】

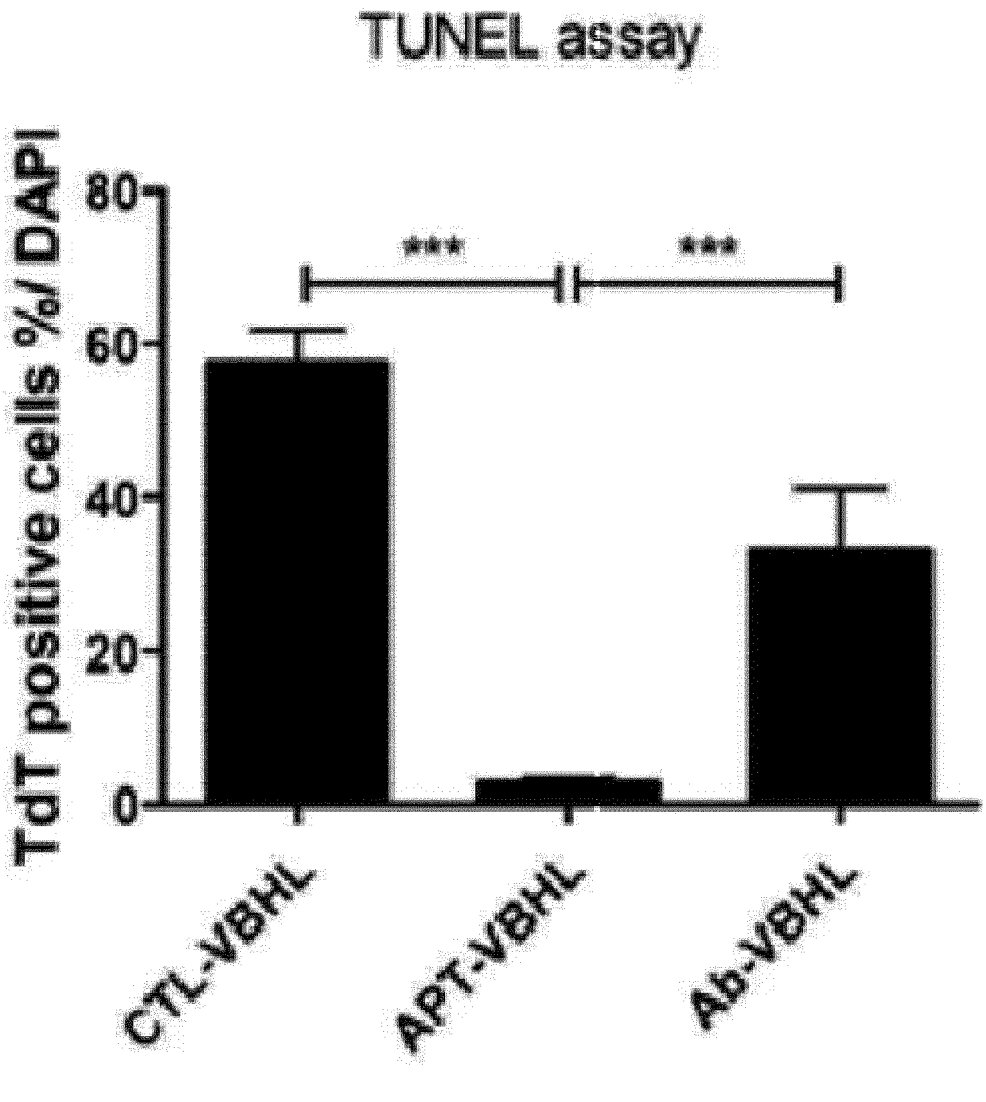
TUNEL assay
TdT positive cells %/ DAPI
80
60
40
20
0
***
***
CTL-VBHL
APT-VBHL
Ab-VBHL

【Fig. 6 a】

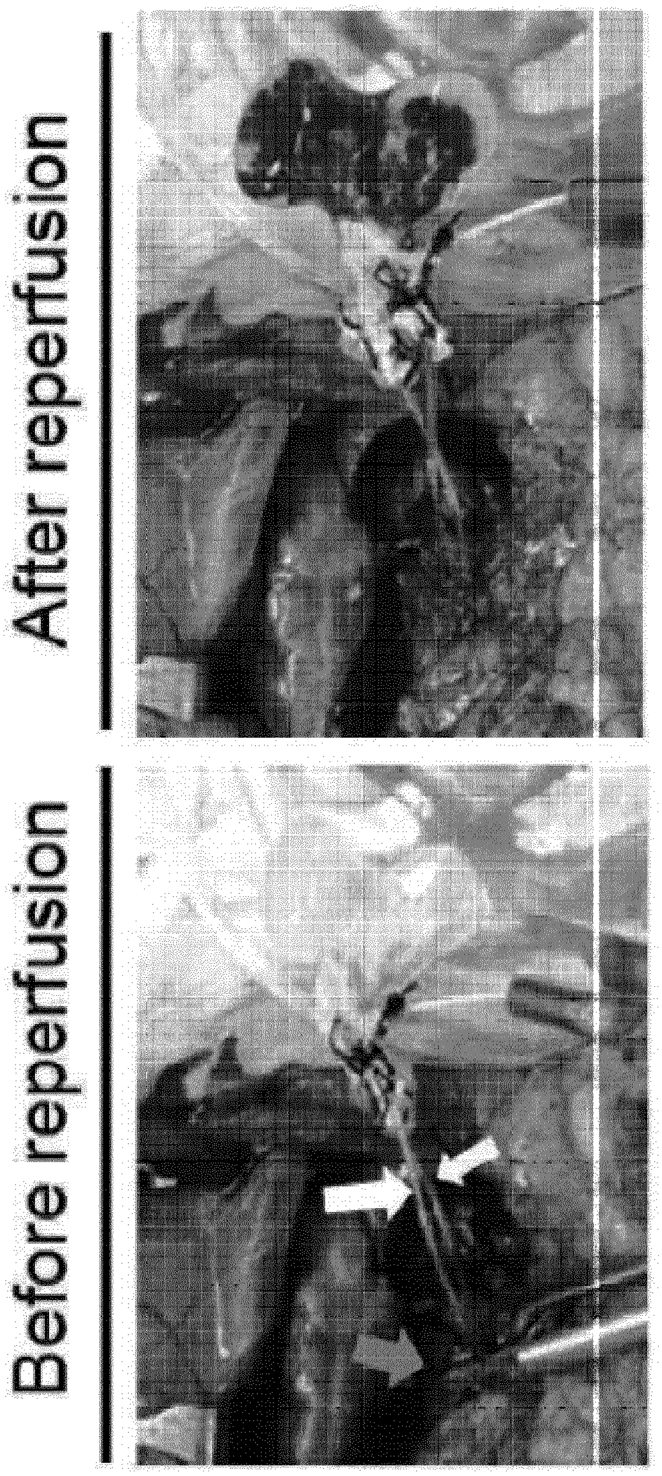

【Fig. 6 b】

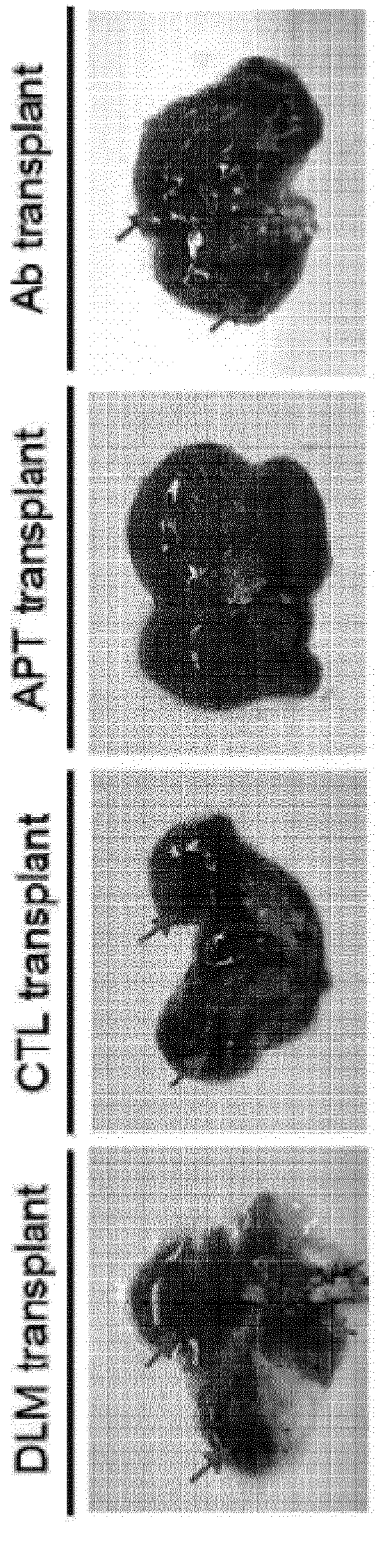
Ab transplant
APT transplant
CTL transplant
DLM transplant

【Fig. 6 c】

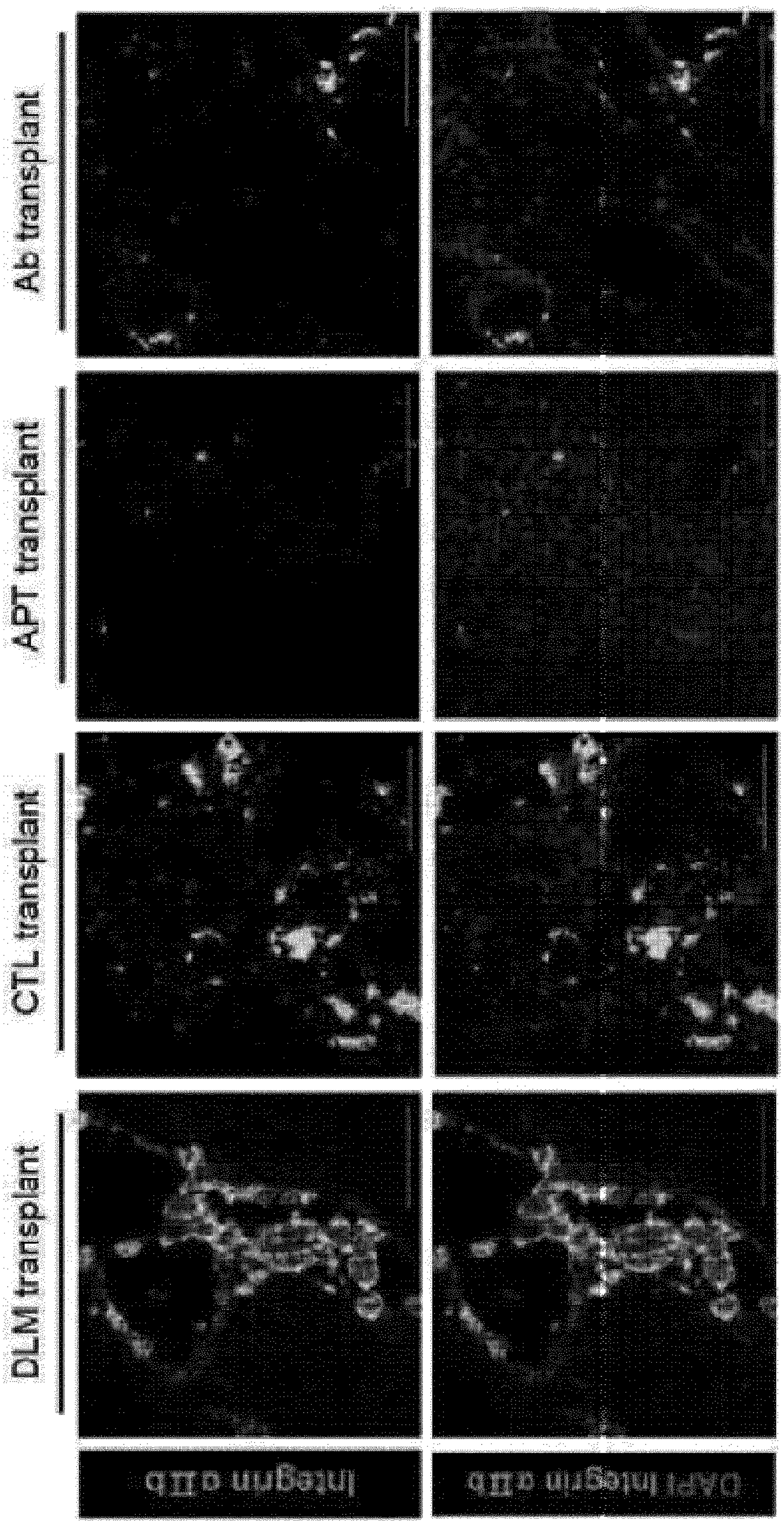
Ab transplant
APT transplant
CTL transplant
DLM transplant
Integrin αⅡb
DAPI Integrin αⅡb

【Fig. 6 d】

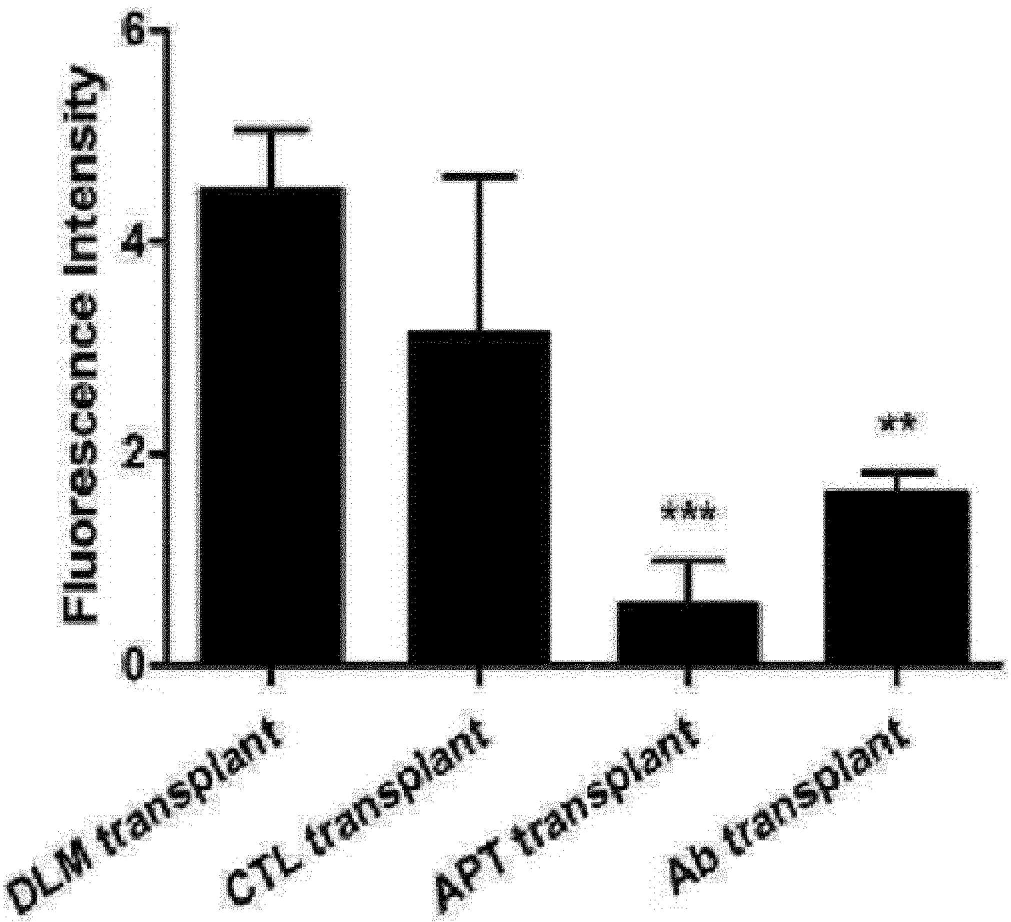
Fluorescence Intensity
6
4
2
0
***
**
DLM transplant
CTL transplant
APT transplant
Ab transplant

【Fig. 6 e】

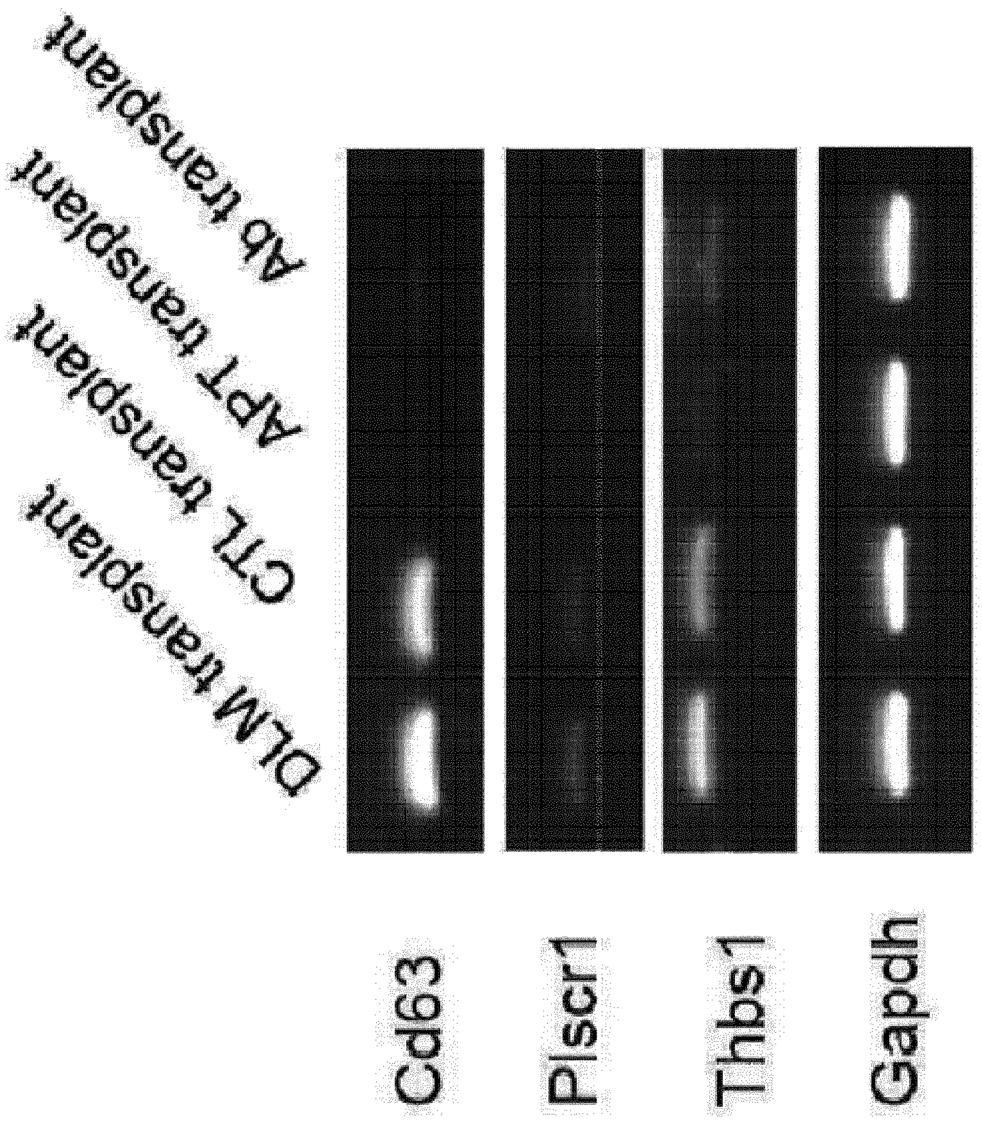
Ab transplant
APT transplant
CTL transplant
DLM transplant
Cd63
Plscr1
Thbs1
Gapdh

【Fig. 7 a】

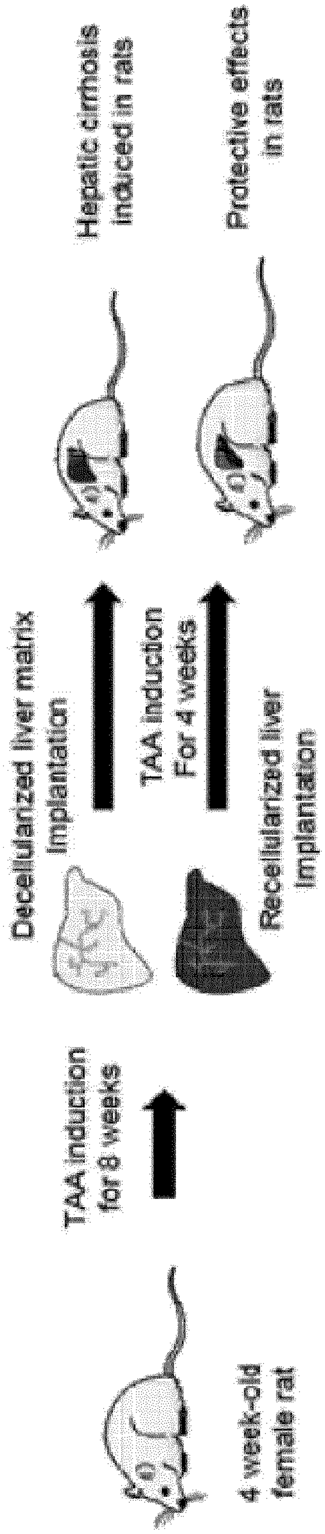
4 week-old
female rat
TAA induction
for 8 weeks
Decellularized liver matrix
Implantation
Recellularized liver
Implantation
TAA induction
For 4 weeks
Hepatic cirrhosis
induced in rats
Protective effects
in rats

【Fig. 7 b】

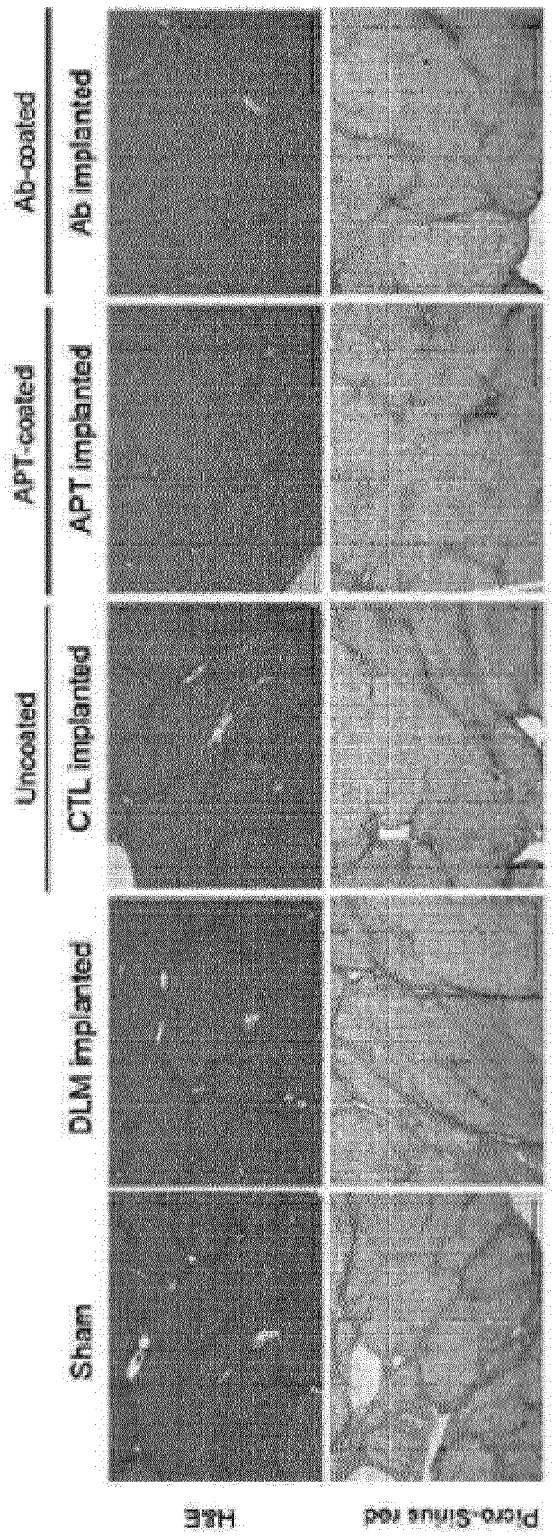
Ab-coated
Ab implanted
APT-coated
APT implanted
Uncoated
CTL implanted
DLM implanted
Sham
H&E
Picro-Sirius red

【Fig. 7 c】

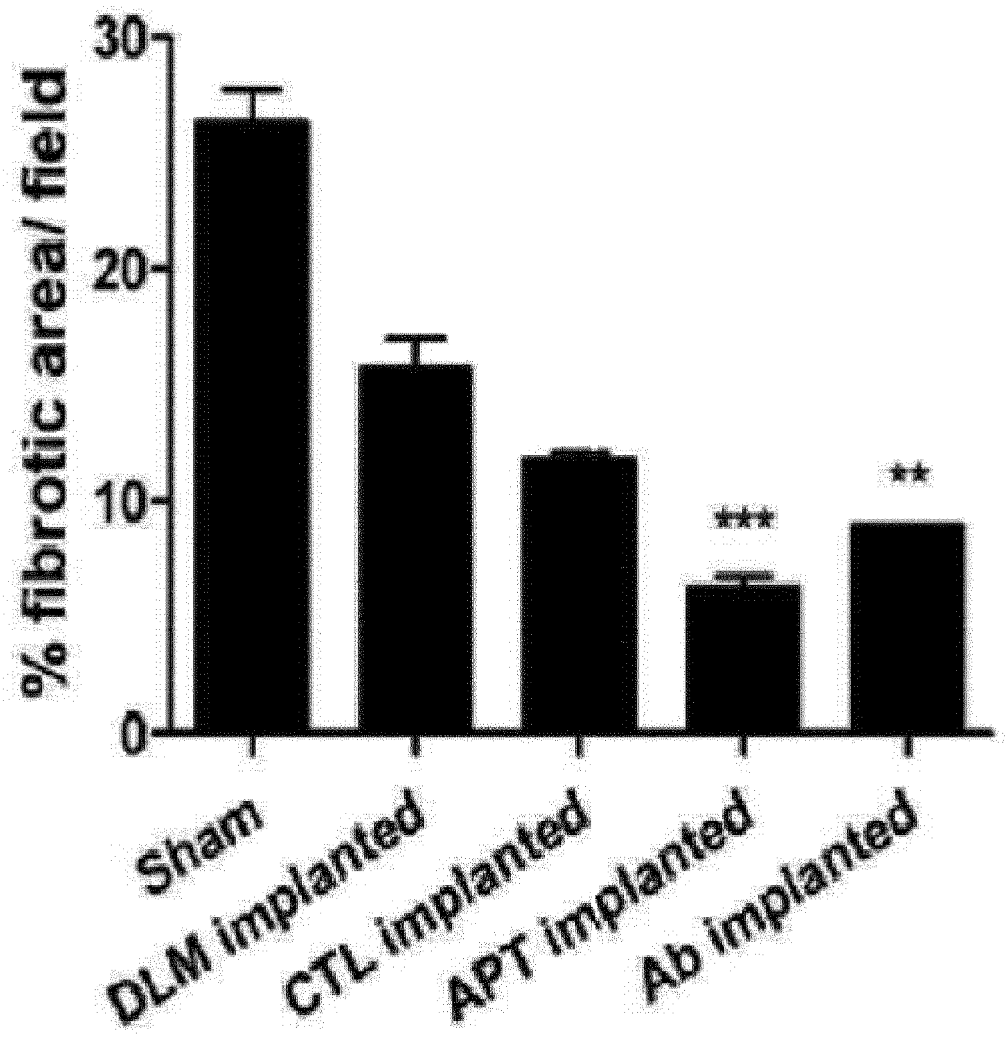
30
20
10
0
% fibrotic area/ field
***
**
Sham
DLM implanted
CTL implanted
APT implanted
Ab implanted

【Fig. 7 d】

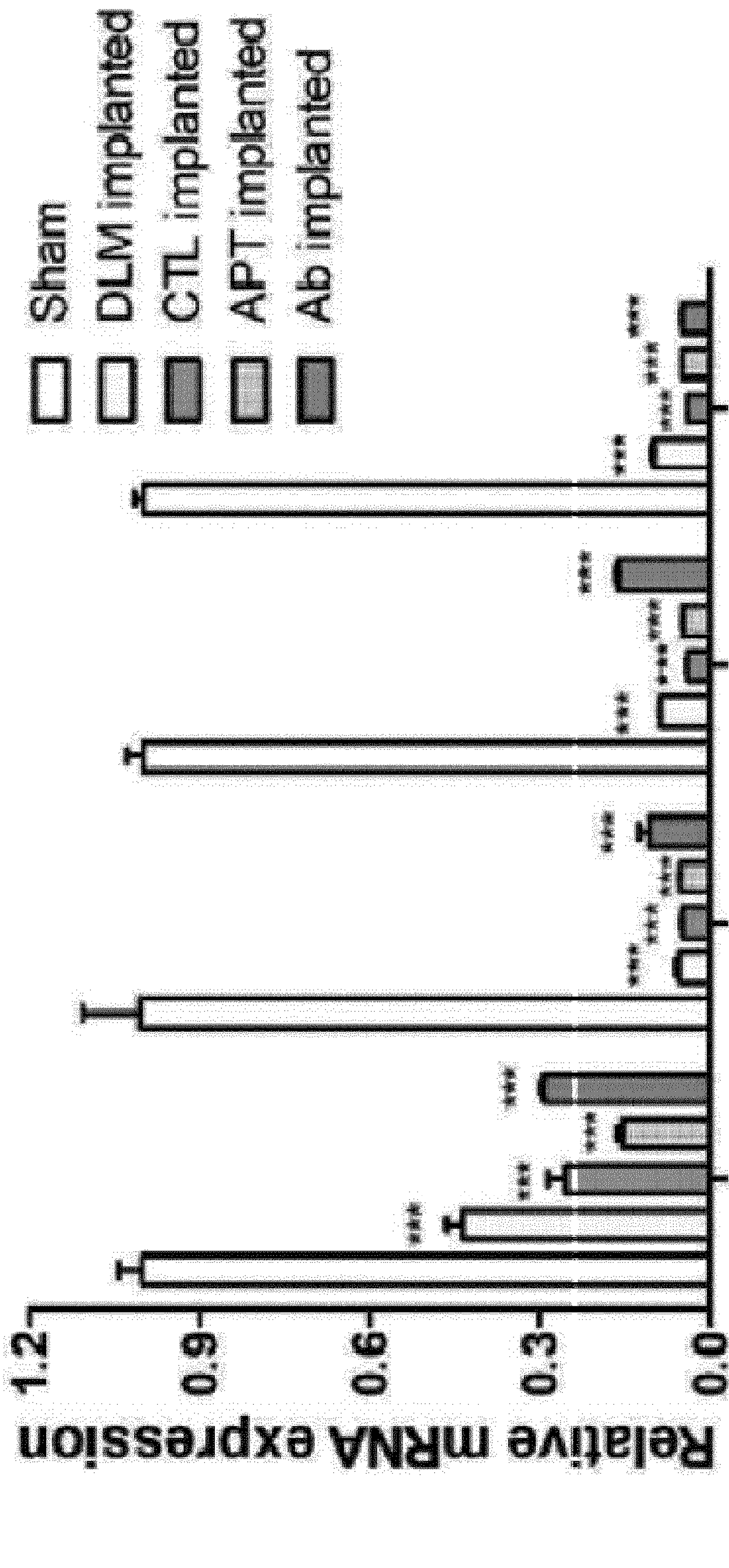
Relative mRNA expression
1.2
0.9
0.6
0.3
0.0
αSma
Vimentin
Tgf-β1
Timp1
Sham
DLM implanted
CTL implanted
APT implanted
Ab implanted

【Fig. 7 e】

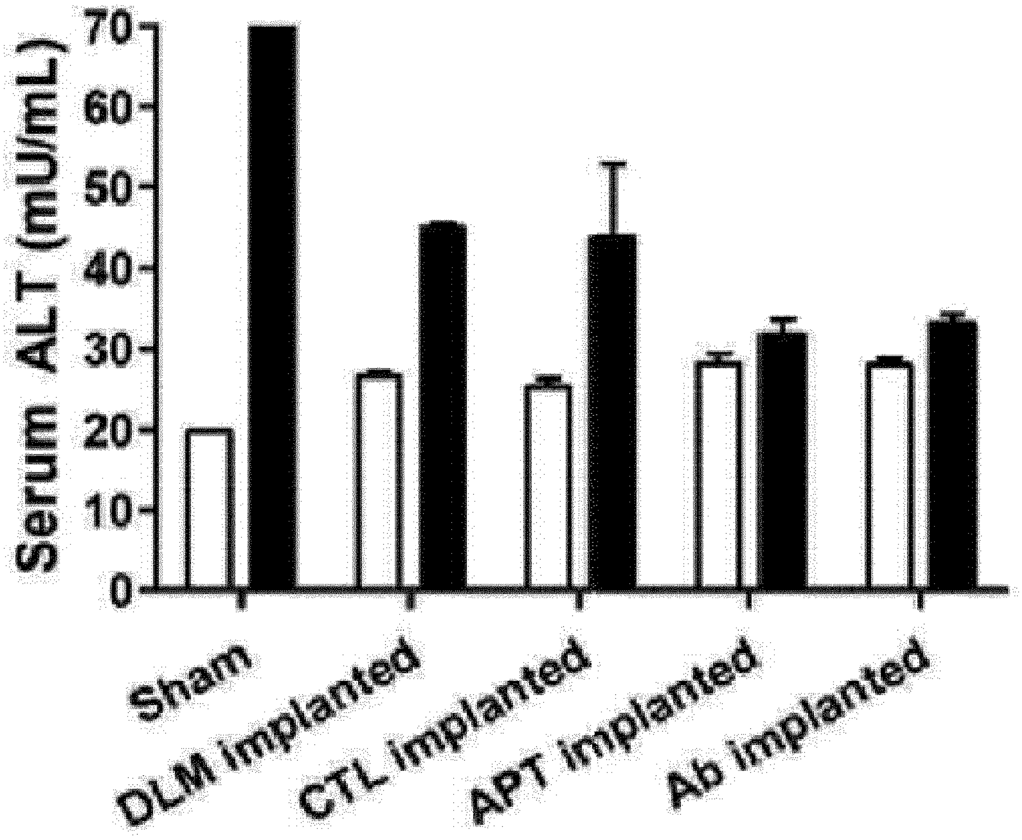
Serum ALT (mU/mL)
70
60
50
40
30
20
10
0
Sham
DLM implanted
CTL implanted
APT implanted
Ab implanted

【Fig. 7 f】

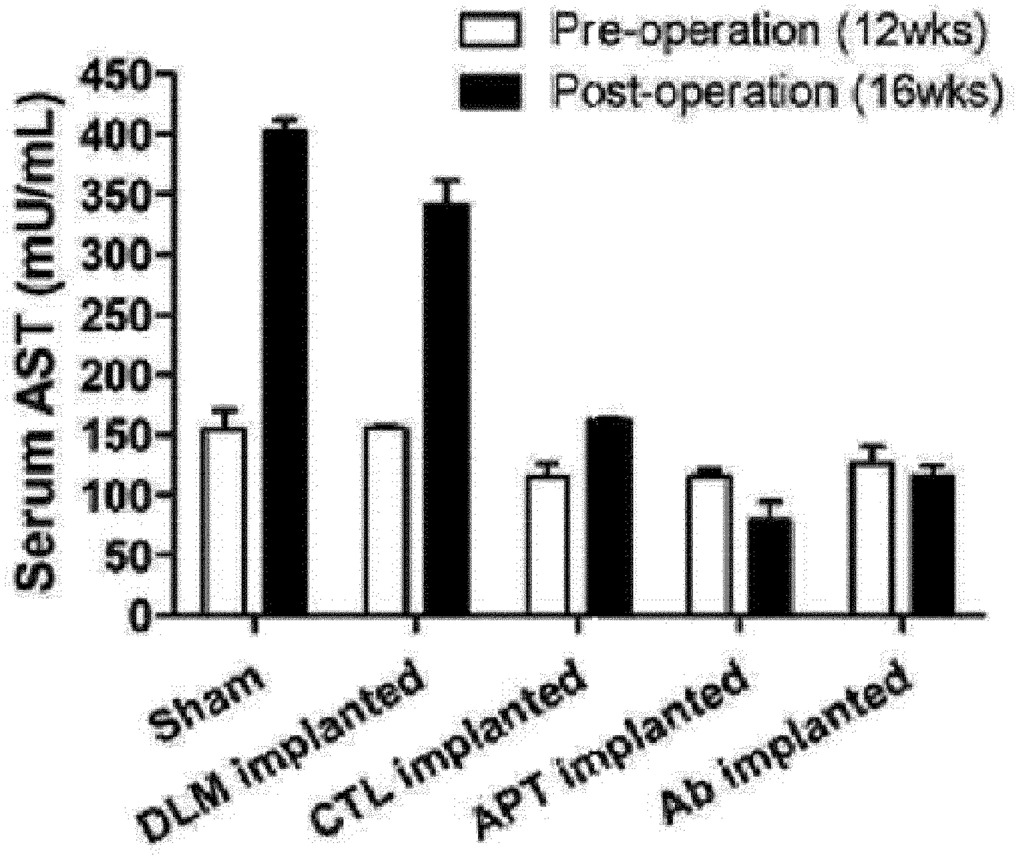
Pre-operation (12wks)
Post-operation (16wks)
Serum AST (mU/mL)
450
400
350
300
250
200
150
100
50
0
Sham
DLM implanted
CTL implanted
APT implanted
Ab implanted

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200098314
- US 1020200150913 A
- KR 101829407
- US 6376244 B
- US 6753181 B


### Non-patent literature cited in the description


- *Acta Biomater*, 2016, vol. 38, 82-93
- **VAN PUTTE et al.** *Ann. Thorac. Surg.*, 2002, vol. 74 (3), 893-8
- **DEN BUTTER et al.** *Transpl. Int.*, 1995, vol. 8, 466-71
- **FIRTH et al.** *Clin. Sci. (Lond.)*, 1989, vol. 77 (6), 657-61
- **MAZZETTI et al.** *Brain Res.*, 2004, vol. 999 (1), 81-90
- **WAGNER et al.** *J. Artif. Organs*, 2003, vol. 6 (3), 183-91